(19) Europäisches Patentamt / European Patent Office / Office européen des brevets

(11) **EP 3 629 719 B1**

(12) **EUROPEAN PATENT SPECIFICATION**

(45) Date of publication and mention
of the grant of the patent:
**23.08.2023   Bulletin 2023/34**

(21) Application number: **18733952.8**

(22) Date of filing: **24.05.2018**

(51) International Patent Classification (IPC):
*A01K 67/027* (2006.01)    *C12N 9/02* (2006.01)
*C12N 15/10* (2006.01)    *C12N 15/90* (2006.01)

(52) Cooperative Patent Classification (CPC):
**C12N 15/902; A01K 67/0275;** A01K 2217/072;
A01K 2227/30; A01K 2267/01; A01K 2267/0393

(86) International application number:
**PCT/IL2018/050573**

(87) International publication number:
**WO 2018/216022 (29.11.2018 Gazette 2018/48)**

(54) **METHODS FOR GENDER DETERMINATION OF AVIAN EMBRYOS IN UNHATCHED EGGS AND MEANS THEREOF**

VERFAHREN ZUR GESCHLECHTSBESTIMMUNG VON VOGELEMBRYONEN IN NICHT AUSGEBRÜTETEN EIERN UND MITTEL DAFÜR

PROCÉDÉS DE DÉTERMINATION DU SEXE D'EMBRYONS AVIAIRES DANS LES UFS NON ÉCLOS ET MOYENS CORRESPONDANTS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.05.2017   US 201762510921 P**

(43) Date of publication of application:
**08.04.2020   Bulletin 2020/15**

(73) Proprietor: **Eggxyt Ltd**
**9100760 Jerusalem (IL)**

(72) Inventor: **OFFEN, Daniel**
**3895500 Kfar HaRoe (IL)**

(74) Representative: **Boult Wade Tennant LLP**
**Salisbury Square House**
**8 Salisbury Square**
**London EC4Y 8AP (GB)**

(56) References cited:
**WO-A1-2017/094015**

- **TIZARD ET AL: "Precision genome engineering in the chicken: The gap between science and market place.", IWRAB&HYPHEN;II, 1 August 2014 (2014-08-01), pages 1-21, XP055387323, Brasilia cited in the application**
- **T. M. SHAFEY ET AL: "Effects of Eggshell Pigmentation and Egg Size on the Spectral Properties and Characteristics of Eggshell of Meat and Layer Breeder Eggs", ASIAN-AUSTRALASIAN JOURNAL OF ANIMAL SCIENCES., vol. 15, no. 2, 1 January 2002 (2002-01-01), pages 297-302, XP055501008, KR ISSN: 1011-2367, DOI: 10.5713/ajas.2002.297**
- **T. J. DORAN ET AL: "Sex selection in layer chickens", ANIMAL PRODUCTION SCIENCE, vol. 58, no. 3, 9 June 2017 (2017-06-09), page 476, XP055500801, AU ISSN: 1836-0939, DOI: 10.1071/AN16785**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

**FIELD OF THE INVENTION**

The present invention relates to methods of gender determination and identification in avian subjects. More specifically, the invention provides non-invasive methods and transgenic avian animals for gender determination and selection of embryos in unhatched avian eggs.

**BACKGROUND ART**

References considered to be relevant as background to the presently disclosed subject matter are listed below:

- WO 2010/103111

- WO 2014/0296707

- US 6244214

- 06124456A2

- US2014069336A

- WO16005539

- WO 96/39505

- WO 97/49806

- Quansah, E., Long, J.A., Donovan, D.M., Becker, S.C., Telugu, B., Foster Frey, J.A., Urwin, N. (2014). Sperm-mediated transgenesis in chicken using a PiggyBac transposon system. Poultry Science Association Meeting Abstract. BARC Poster Day.

- Jinek, M., Chylinski, K., Fonfara, I., Hauer, M., Doudna, J. A., & Charpentier, E. (2012). A programmable dual-RNA-guided DNA endonuclease in adaptive bacterial immunity. Science, 337(6096), 816-821.

- Cong, L., & Zhang, F. (2015). Genome engineering using CRISPR-Cas9 system. Chromosomal Mutagenesis, 197-217.

- US Patent 6, 244,214.

- WO 2014/0296707.

- WO 06124456A2;

- WO16005539.

- Véron N., Qu Z., Kipen PA., Hirst CE., Marcelle C. (2015). CRISPR mediated somatic cell genome engineering in the chicken. Dev. Biol. 407(1):68-74. doi: 10.1016/j.ydbio.2015.08.007. Epub 2015 Aug 13.

- CA2264450.

- Niu, Y., B. Shen, Y. Cui, Y. Chen, J. Wang et al., (2014). Generation of genemodified cynomolgus monkey via cas9/rna-mediated gene targeting in one-cell embryos. Cell, 156(4): 836-843.

- Hwang, W. Y., Y. Fu, D. Reyon, M. L. Maeder, S. Q. Tsai et al., (2013). Efficient genome editing in zebrafish using a CRISPR-Cas system. Nat. Biotechnol. 31(3): 227-229.

- Nadège, V., Q. Zhengdong, P. A. S. Kipen, C. E. Hirst, M. Christophe et al., (2015). CRISPR mediated somatic cell genome engineering in the chicken. Dev. Biol. 407(1): 68-74.

- Bai, Y., L. He, P. Li, K. Xu, S. Shao et al., (2016). Efficient genome editing in chicken DF-1 cells using the CRISPR/Cas9 system. G3 (Bethesda) pii: g3.116.027706.

- Doran T. et al., (2016). Sex selection in layer chickens. ASAP Animal Production, Adelaide.

- Tizard M. and Doran T. (2014). Precision genome engineering in the chicken: Th gap between science and market place. A presentation at IWRAB - II, in Brasilia.

Acknowledgement of the above references herein is not to be inferred as meaning that these are in any way relevant to the patentability of the presently disclosed subject matter.

## BACKGROUND OF THE INVENTION

In the food industry, chicks are culled by billions on a daily basis via suffocation or grinding. The males are terminated since they are not useful for laying eggs or to be bread for meat and the weak or unhealthy females are being terminated as well. A method for *in-ovo,* or embryo sex-determination prior to hatching is thus highly desired due to both ethical and economic considerations.

Specifically, visually identifying poultry fertile eggs is important to allow removal of unfertile eggs to save hatching costs (by prevention of hatching an unfertile egg), and to lower the bio-security risks involved in the continuation of the incubation of these contamination-prone unfertile eggs alongside the fertile eggs.

Visually identifying egg fertility at an early stage of the embryo, while inside the unhatched egg, involves outer light source candling and can be difficult, virtually impossible in early embryonic stages. An even greater challenge is to identify the sex of embryos, and currently there is no available method for the discrimination between males and females in unhatched eggs that are found fertile. However, identification of fertility at an early embryonic stage and the sex determination of poultry are vital for aviculture, scientific research, and conservation. The determination of sex in young birds by morphological features is extremely challenging for most species. The gender may be determined by individual vent sexing which involves manually squeezing the feces out of the chick, which opens up the chicks' anal vent slightly, allowing to see if the chick has a small "bump", which would indicate that the chick is a male. However, this method represents high risk of bird injury and mistakes in sex determination, together with cumbersome work conducted manually by trained personal.

Vent sexing or chick sexing is the method of distinguishing the sex of chicken and other hatchlings, usually by a trained person called a chick sexer or chicken sexer. Chicken sexing is practiced mostly by large commercial hatcheries, who have to know the difference between the sexes in order to separate them into sex groups, and in order to take them into different programs, which can include the growing of the desired group and culling of the undesired group which does not meet the commercial needs. In example, a male hatched from an egg that comes from an egg layer commercial line of breed. That male will not have a good meat yield and will not lay eggs, and thus will be culled after sexing. Upon sexing, the relevant sex will continue its course to serve the purpose while the other sex or most of it will be culled within days of hatching being irrelevant to egg production.

In farms that produce eggs, males are unwanted, and chicks of an unwanted sex are killed almost immediately to reduce costs to the breeder. Chicks are moved down a conveyer belt, where chick sexers separate out the males and toss them into a chute where they are usually ground up alive in a meat grinder.

Identification and determination of the fertility of an egg and the sex of the embryos in eggs prior to their hatching, will enable the elimination of unfertile eggs, and the unwanted type of embryos while in their eggs, and thus will immensely reduce incubation costs (which includes the energy and efficiency costs alongside with air pollution and energy consumption). In addition, chicks' suffering will cease and pollution from culling will be prevented. An automated sexing device will additionally result in reduced eggs production costs by eliminating the need for chick sexers, as well as reduce the size of the hatcheries needed since at early stage 50% of the eggs will be reduced deducted from the process, thus reducing the costs of hatching these eggs, and later on the need for any elaborate killing procedures.

In all commercial types of birds intended for breeding, laying, or meat production, there is a need to determine fertility and the sex of the embryo. There are great economic returns; in energy saving, biosecurity risk reduction, garbage disposal, sexing labor costs and sexing errors, culling costs and disposal, and animal welfare.

WO 2010/103111 describes an invasive method comprising a series of steps, among them introducing into the egg a labeled antibody, specifically designed to match a sex-specific antigen on the embryo.

WO 2014/0296707 describes luminance composition designed to serve as a biomarker for quantifying or evaluating efficiency of vaccination being injected into the bird's egg. No sex determination is described or even hinted in this disclosure. In-ovo injection apparatus and detection methods was disclosed by US Patent 6, 244,214.

WO 06124456A2 discloses invasive methods of *in-ovo* sex determining of an avian embryo by determining the presence of an estrogenic steroid compound in a sample of embryonic fluid (e.g., allantoic fluid or blood) from the avian egg. Determining the presence of the compound is done by measuring analytes in samples obtained from said avian egg by competitive immunoassay utilizing fluorescence microscopy.

Spectroscopic approaches were also described, among them US2014069336A which is based on screening the avian embryo feather color (pre-hatching) and determining the sex of the avian embryo, based on the feather color or WO16005539 which disclose a device obtaining a shell-specific spectral response to an incident light signal.

Further genetic approaches addressing the *in ovo* sexing include DNA sequencing of DNA samples obtained from fertilized eggs for detecting two specific genes located on the Z and W chromosomes of birds (WO 96/39505), or the use of oligonucleotide probes which hybridize to specific sequences of the female W chromosome (WO 97/49806). These methods are invasive and therefore do not provide a safe strategy.

The clustered regularly interspaced short palindromic repeats (CRISPR)/CRISPR-associated (Cas) system is the state of the art gene editing system, allowing a simple construct design with high success rate (M. Jinek, 2012).

Niu et al. (2014) injected guide RNA (gRNA) and Cas9 RNA into monkey oocytes to modify three target genes, and Hwang et al. (2013) modified the drd3 and gsk3b genes in zebrafish embryos to obtain a two-locus mutant. Cong and Zhang (2015) have modified the CRISPR system to edit any gene in living cells.

Veron and coworkers (2015), demonstrated that expression levels of somatic cells in chicken embryos were modified by electroporation of CRISPR gRNA plasmids directed against the PAX7 transcription factor (Nadège et al. 2015), Bai and coworkers edited the PPAR-g, ATP synthase epsilon subunit (ATP5E).

Quansah, E. et. al., disclosed sperm mediated transgenesis in chicken using a PiggyBac transposon system. In particular, they disclose that aGFP plasmid and Lipofectamine LTXTM 9LPX) combination had no effect on viability, mobility or fertility of chicken sperm.

CA2264450 discloses pluripotent cells comprising a nucleic acid sequence encoding a fluorescent protein marker selectively integrated into a heterologous sex chromosome in the cell embryos and transgenic animals produced using the pluripotent cells and, the uses of such cells, embryos, and animals. This publication particularly relate to GFP in mammalian cells.

Doran T. et al., generally describes a strategy to differentiate between males and females pre-hatch, by adding a biological marker, the GFP reporter gene, to the sex chromosome, in ASAP Animal Production 2016, Adelaide. The integration of the GFP reporter gene into the sex chromosome of chicks using the CRISPR technology was also described in a presentation at IWRAB-II, in Brasilia, in 2014. However, as this general publication discloses that the labeled chromosome is visualized *in ovo* by exposing the egg to UV light, due to the auto-fluorescence of the egg, no detectable signal is expected.

Thus, effective and non-invasive methods for sex identification during the egg stage, prior to the hatching of the chick are currently not available. There is therefore a long-felt need for a method enabling accurate and safe sex identification of the embryos in unhatched eggs.

## SUMMARY OF THE INVENTION

A first aspect of the invention relates to a method of gender determination of an avian embryo in an unhatched egg comprising the embryo within a structurally integral shell, the method comprising the step of:
First, in step (a), providing at least one transgenic avian subject comprising at least one exogenous reporter gene integrated into at least one position or location (also referred to herein as locus) in at least one of gender chromosome Z and W, wherein said reporter gene is a Red Fluorescent Protein (RFP).

In a second step (b), obtaining at least one unhatched egg comprising said embryo within a structurally integral shell, from the transgenic avian subject.

The next step (c) involves determining in said embryo within said structurally integral shell of said unhatched egg if at least one detectable signal is detected wherein detection of said at least one detectable signal indicates the expression of said at least one reporter gene in said embryo within said structurally integral shell of said unhatched egg, thereby the presence of said Z chromosome or W chromosome in said embryo,
thereby determining the gender of an avian embryo in an unhatched egg comprising the embryo within a structurally integral shell.

In a second aspect, the invention relates to an avian transgenic subject comprising at least one exogenous reporter gene integrated into at least one position or location in at least one of gender chromosome Z and W, wherein said reporter gene is a Red Fluorescent Protein (RFP), and wherein the expression of said at least one reporter is suitable for identification of a gender of an avian embryo in an unhatched egg comprising the embryo within a structurally integral shell.

Also described herein is a cell comprising at least one exogenous reporter gene integrated into at least one position or locus in at least one of gender chromosome Z and W.

In yet a further aspect, the invention provides a kit comprising:

(a) at least one Cas9 protein, or at least one first nucleic acid sequence comprising at least one nucleic acid sequence encoding said at least one Cas9 protein; and at least one guide RNA (gRNA) that targets at least one protospacer located within at least one gender chromosome Z or W, or nucleic acid sequence encoding said at least one gRNA; and (b) at least one second nucleic acid sequence comprising at least one reporter gene, wherein said reporter gene is a Red Fluorescent Protein (RFP); wherein the expression of said at least one reporter is suitable for identification of a gender of an avian embryo in an unhatched egg comprising the embryo within a structurally integral shell, wherein said at least one reporter gene is integrated into at least one site at: (a) gender Z chromosome locus 42172748-42177748, wherein said at least one gRNA comprises the nucleic acid sequence as denoted by SEQ ID NO. 26 and said at least one reporter gene comprised within said second nucleic acid is flanked at 5' and 3' thereof by homologous arms comprising the nucleic acid sequence as denoted by SEQ ID NO. 31 and 32, respectively; or (b) gender W chromosome locus 1022859-1024215, wherein at least one of: (i) said gRNA comprises the nucleic acid sequence as denoted by SEQ ID NO. 1, and said at least one reporter gene comprised within said second nucleic acid sequence is flanked at 5' and 3' thereof by homologous arms comprising the amino acid sequence as denoted by SEQ ID NO. 4 and 5, respectively; and (ii) said gRNA comprises the nucleic acid sequence as denoted by SEQ ID NO. 2, and said at least one reporter gene comprised within said second nucleic acid sequence is flanked at 5' and 3' thereof by homologous arms comprising the amino acid sequence as denoted by SEQ ID NO. 6 and 7, respectively.

Still further, described herein is a method for determining and detecting fertilization in an unhatched egg. More specifically, such method comprises comprise the step of:
First, in step (a), providing or obtaining at least one transgenic avian subject or animal comprising at least one exogenous reporter gene integrated into at least one position or location in both gender chromosome Z and W, in case of a female subject and into both gender chromosomes Z in a male. In a second step (b) obtaining at least one fertilized egg from the transgenic avian subject, or of any cells thereof. The next step (c) involves determining in the egg if at least one detectable signal is detected. In more specific examples, detection of at least one detectable signal indicates the expression of said at least one reporter gene, thereby the presence of the labeled maternal W chromosome or Z chromosome (in case of a female or the labeled paternal Z chromosome in the avian embryo.

These and further embodiments of the invention will become apparent by the hand of the following figures.

## BRIEF DESCRIPTION OF THE DRAWINGS

In order to better understand the subject matter that is disclosed herein and to exemplify how it may be carried out in practice, embodiments will now be described, by way of non-limiting example only, with reference to the accompanying drawings, in which:

### Figure 1A-1B. Complete set up for assessing fluorescence of eggs

**Fig. 1A.** shows a photograph of the device used by the methods of the invention for *in ovo* gender determination. The complete setup is composed of a laser source (h), laser source holder (g), a stand for the egg (e), the egg (f), a lens (d), a filter (c), a stand for the detector (b) and a detector (a). The different components are placed on a solid support (i).
**Fig. 1B.** shows schematic illustration of the device of the invention shown in Fig. 1A. The device comprises a laser source (h), laser source holder (g), a stand for the egg (e), the egg (f), a lens (d), a filter (c), a stand for the detector (b) and a detector (a). The different components are placed on a solid support (i).

### Figure 2. Fluorescence intensity using a Blue laser (473 nm) with or without fluorescein
The fluorescence intensity [Pout] that was received on the detector when a complete egg was subjected to blue laser with or without $10\mu M$ or 1mM fluorescein (fl), and the intensity is represented as a function of the light source intensity [Pin].

### Figure 3. Fluorescence intensity using a Green laser (532 nm) with or without Rhodamin
The fluorescence intensity [Pout] that was received on the detector when a complete egg was subjected to green laser with or without Rhodamin, and the intensity is represented as a function of the light source intensity [Pin].

### Figure 4. Fluorescence intensity using a Green laser (532 nm) with or without dir
The fluorescence intensity [Pout] that was received on the detector, when a complete egg was subjected to green laser with or without dir, and the intensity is represented as a function of the light source intensity [Pin].

**Figure 5. Fluorescence intensity using a Red laser (632.8 nm) with or without dir**
The fluorescence intensity [Pout] that was received on the detector, when a complete egg was subjected to red laser with or without dir, and the intensity is represented as a function of the light source intensity [Pin].

**Figure 6. Cells expressing GFP**
Picture providing the fluorescent signal of HEK cells transfected with GFP-vector expressing GFP.

Figure 7. Cells expressing RFP
Picture providing the fluorescent signal HEK cells transfected with RFP-vector expressed RFP.

**Figure 8. Control measurements**
Fluorescence intensity using a Green laser and a red filter with different positions of the egg. The fluorescence intensity [Pout] that was received on the detector is represented as a function of the light source intensity [Pin].

**Figure 9. RFP Measurements**
Measurements of fluorescence intensity of eggs injected with different concentrations of RFP-expressing cells using a Green laser and the red filter. The fluorescence intensity [Pout] that was received on the detector is represented as a function of the light source intensity [Pin].

**Figure 10. RFP angle**
RFP fluorescence intensity measurements of excited eggs at the determined optimal position of the egg using the Green laser and the red filter. The fluorescence intensity [Pout] that was received on the detector is represented as a function of the light source intensity [Pin].

**Figure 11A-11B. RFP-expressing cells with PBS or Glycerol**
Figure shows fluorescence intensity of Eggs injected with different concentration of RFP-expressing cells with PBS or Glycerol using the Green laser.

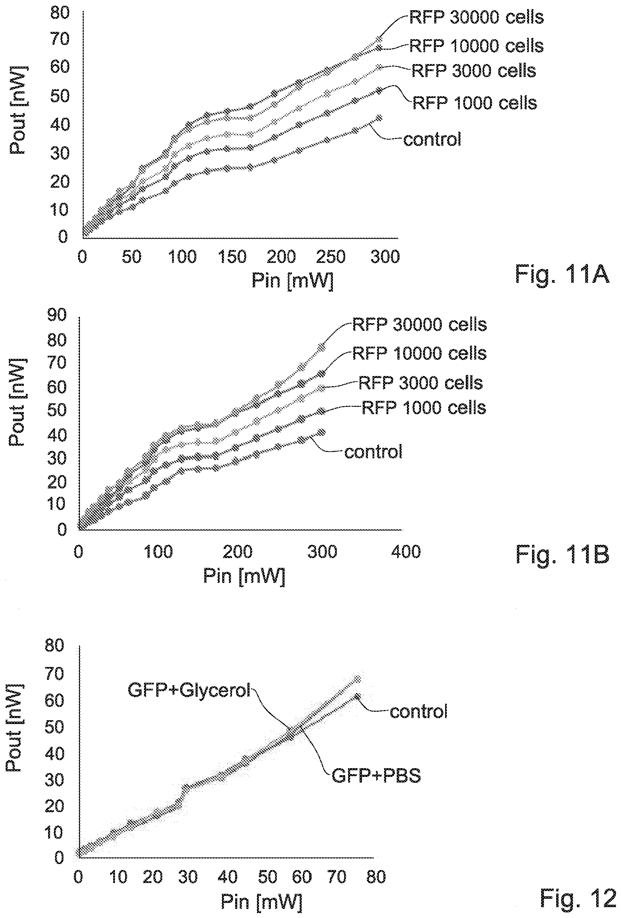

**Fig. 11A.** shows the fluorescence intensity of Eggs containing different concentrations of RFP-expressing cells together in PBS. **Fig. 11B** shows the fluorescence intensity of Eggs containing different concentrations of RFP-expressing cells in Glycerol. The fluorescence intensity [Pout] that was received on the detector is represented as a function of the light source intensity [Pin].

**Figure 12. GFP-expressing cells with PBS or Glycerol**
Figure shows fluorescence intensity of Eggs injected with 30,000 GFP-expressing cells with PBS or Glycerol using the Blue laser. The fluorescence intensity [Pout] that was received on the detector is represented as a function of the light source intensity [Pin].

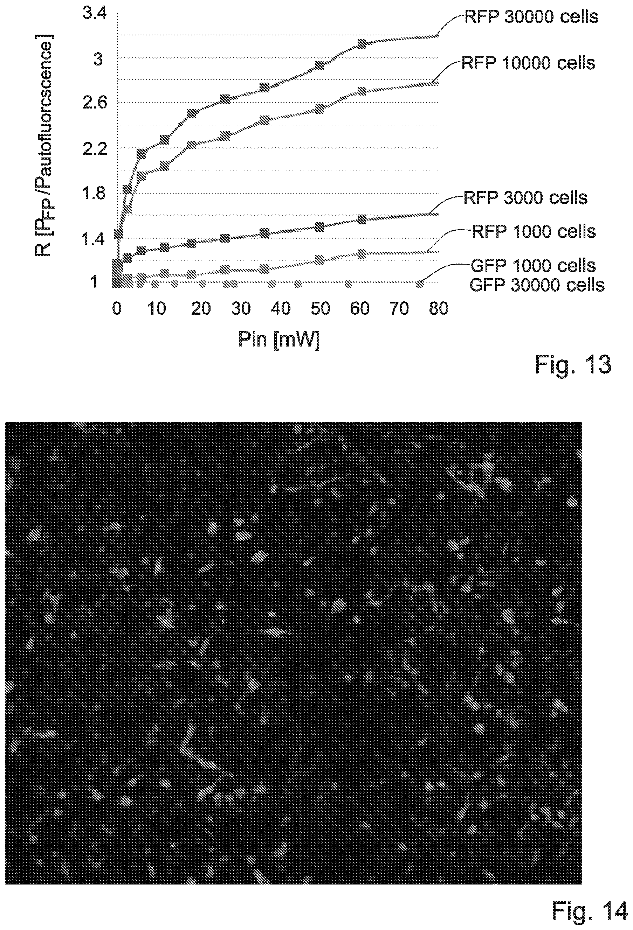

**Figure 13. RFP vs. GFP**

Figure shows ratio between the Fluorescence Protein intensity either from GFP-expressing cells or RFP-expressing cells and the auto fluorescence intensity.
Parameter R describing the ratio between Fluorescence and auto fluorescence is represented as a function of the light source intensity [Pin].

**Figure 14. Incorporation of RFP into female Z chromosome**
Figure shows RFP transfected female chicken cell line with pDsRed with chicken ChZ Left &Right arms and CMV-hspCas9-H1-gRNA.

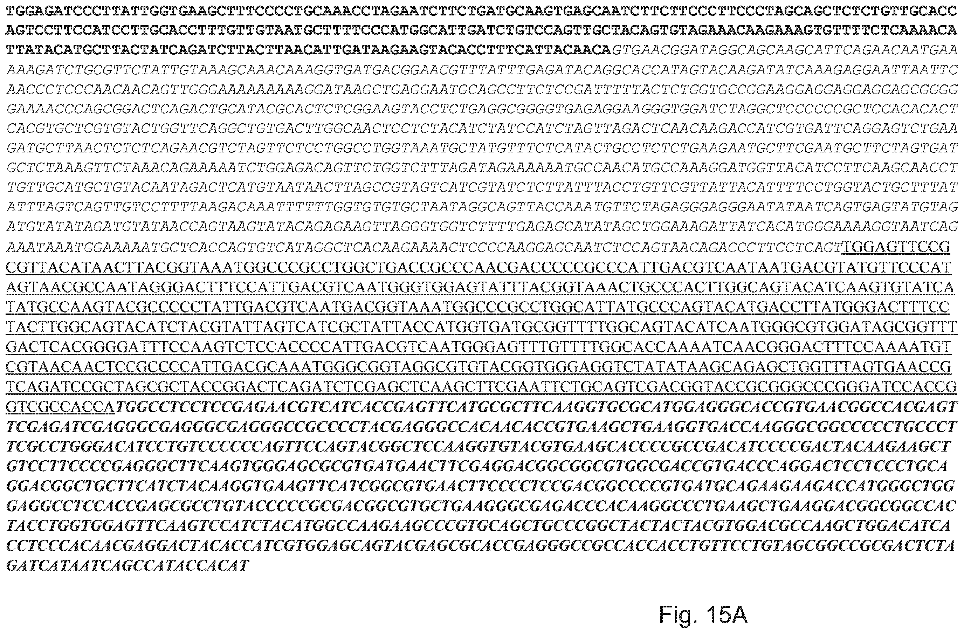

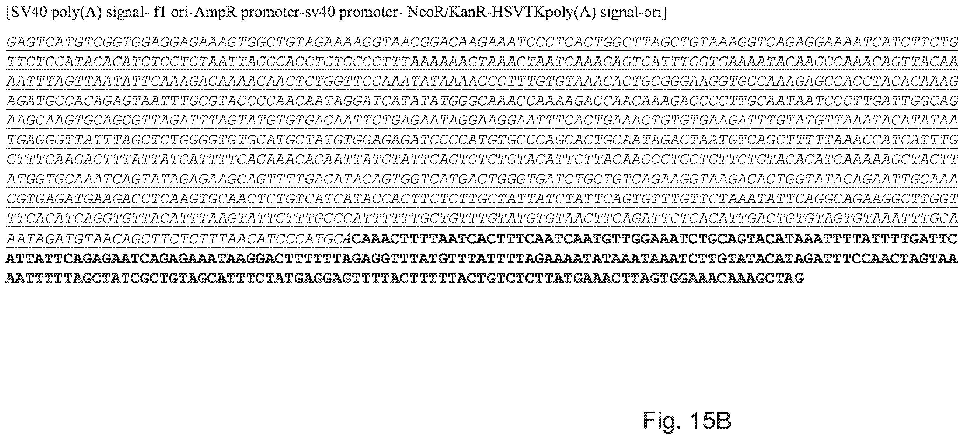

**Figure 15A-15B. Schematic representation of the integrated sequence into chromosome Z of female chicken cell line**
Bold sequences represent the flanking left and right arms of the Z chromosome and are denoted by SEQ ID NO: 60 and SEQ ID NO: 61 respectively; The sequence (1017bp) marked with Italics represents Left arm, as denoted by SEQ ID NO: 62; Underlined sequence (629bp) represents the CMV Enhancer, Promotor and MCS, as denoted by SEQ ID NO: 63; Bold Italics sequence (714bp) represents the dsRED2 reporter gene, as denoted by SEQ ID NO: 64; Sequence with no color-3332bp represents the dsRED plasmid content; Underlined Italics sequence (1026bp) represents the Right arm, as denoted by SEQ ID NO: 65. The left part of the sequence is presented by SEQ ID NO. 66 (all parts 5' to vector sequences, shown in Fig. 15A), and the right part of the sequence is denoted by SEQ ID NO. 67 (all parts 3' to vector sequences, shown in Fig. 15B).

## DETAILED DESCRIPTION OF THE INVENTION

Each day, billions of male chicks are being terminated via suffocation or grinding since they are not useful for laying eggs or to be bread for meat. The ability to determine the sex of the embryo before hatching is of high importance both ethically and financially. In the chicken- the genetic make-up of the sex chromosomes is ZZ for males and ZW for females. Meaning the W chromosome determines the gender of the female. This is unlike humans, in which it is the Y from the father that determines the male gender.

The invention provides a non-invasive efficient method for gender determination, using a reporter gene integrated in a gender specific chromosomes of transgenic avian subjects. Expression of this reporter gene in an embryo of an unhatched egg clearly and accurately identify the gender of said embryo.

Thus, a first aspect of the invention relates to a method of gender determination and optionally of selection of avian embryo in an unhatched egg comprising the embryo within a structurally integral shell. The method comprises the steps of:

First, in step (a), providing at least one transgenic avian subject comprising at least one exogenous reporter gene integrated into at least one position or location in at least one of gender chromosome Z and W, wherein said reporter gene is a Red Fluorescent Protein (RFP). In a second step (b) obtaining at least one unhatched egg comprising said embryo within a structurally integral shell, from the transgenic avian subject.

The next step (c), involves determining in the embryo within said structurally integral shell of said unhatched egg if at least one detectable signal is detected, wherein detection of at least one detectable signal indicates the expression of the at least one reporter gene in said embryo within said structurally integral shell of said unhatched egg, thereby the presence of the W chromosome or Z chromosome in the avian embryo. Thus, in case the reporter gene has been integrated into the Z chromosome of a female transgenic avian, identification of a detectable signal in the examined egg indicate that the embryo comprised therein has a maternal Z chromosome having a reporter gene integrated therein, and the embryo is thereby identified as male. In yet some other embodiments, in case the reporter gene has been integrated into the W chromosome of a female transgenic avian, identification of a detectable signal in the examined egg indicate that the embryo comprised therein carries a maternal W chromosome and is therefore determined as female, thereby providing gender determination of the embryo.

It should be appreciated that the transgenic avian provided by the invention may be either a female or a male, as described in more detail herein after. In yet some further specific embodiments, the transgenic animal may be the gender having two different gender chromosomes, specifically, the heterogametic subject. In some particular embodiments the heterogametic animal may be a female. In more specific embodiments, where the transgenic avian subject is a female, the egg identified by the method of the invention is laid by the transgenic female avian provided by the invention. In more specific embodiment, the transgenic female may be fertilized either by a transgenic male or in some other embodiments, by a wild type avian male. Still further, fertilization may occur either by mating or by insemination of the transgenic avian female with sperms obtained from a transgenic or wild type avian male. In yet some other embodiments, where the transgenic avian is a male, egg identified by the method of the invention may be laid by either a wild type or transgenic female mated with the transgenic male provided by the invention, or inseminated by any cells thereof, specifically sperm cells that comprise the exogenous reporter gene of the invention integrated into the gender chromosomes thereof.

The invention thus provides a method for detecting a gender of an avian embryo within an unhatched fertilized egg. It should be appreciated that the method of the invention may be applicable for unhatched eggs of any embryonic stage of an avian embryo.

It should be noted that **"Embryonic development stage or step of avian embryo",** as used herein refers to the stage of **day 1** wherein the germinal disc is at the blastodermal stage and the segmentation cavity takes on the shape of a dark ring; the stage of **day 2** wherein the first groove appears at the center of the blastoderm and the vitelline membrane appears; the stage of **day 3** wherein blood circulation starts, the head and trunk can be discerned, as well as the brain and the cardiac structures which begins to beat; the stage of **day 4** wherein the amniotic cavity is developing to surround the embryo and the allantoic vesicle appears; the stage of **day 5** wherein the embryo takes a C shape and limbs are extending; the stage of **day 6** wherein fingers of the upper and lower limbs becomes distinct; the stage of **day 7** wherein the neck clearly separates the head from the body, the beak is formed and the brain progressively enters the cephalic region; the stage of **day 8** wherein eye pigmentation is readily visible, the wings and legs are differentiated and the external auditory canal is opening; the stage of **day 9** wherein claws appears and the first feather follicles are budding; the stage of **day 10** wherein the nostrils are present, eyelids grow and the egg-tooth appears; the stage of **day 11** wherein the palpebral aperture has an elliptic shape and the embryo has the aspect of a chick; the stage of **day 12** wherein feather follicles surround the external auditory meatus and cover the upper eyelid whereas the lower eyelid covers major part of the cornea; the stage of **day 13** wherein the allantois becomes the chorioallantoic membrane while claws and leg scales becomes apparent; the stage of **days 14 to 16** wherein the whole body grows rapidly, vitellus shrinking accelerates and the egg white progressively disappears; the stage of **day 17** wherein the renal system produces urates, the beak points to the air cell and the egg white is fully resorbed; the stage of **day 18** wherein the vitellus internalized and the amount of amniotic fluid is reduced; the stage of **day 19** wherein vitellus resorption accelerates and the beak is ready to pierce the inner shell membrane; the stage of **day 20** wherein the vitellus is fully resorbed, the umbilicus is closed, the chick pierces the inner shell membrane, breathes in the air cell and is ready to hatch; the stage of **day 21** wherein the chick pierces the shell in a circular way by means of its egg-tooth, extricates itself from the shell in 12 to 18 hours and lets its down dry off.

More specifically, the method of the invention may be applicable in determining the gender of an avian embryo *in-ovo,* inside the egg, at every stage of the embryonic developmental process. More specifically, from day 1, from day 2, from day 3, from day 4, from day 5, from day 6, from day 7, from day 8, from day 9, from day 10, from day 11, from day 12, from day 13, from day 14, from day 15, from day 16, from day 17, from day 18, from day 19, from day 20 and from day 21. More specifically, the method of the invention may be applicable for early detection of the embryo's gender, specifically, from day 1 to day 10. In some embodiments the method of the invention may enable determining the embryo's gender at day one of the embryonic development. In some further embodiments, the method of the invention may enable determining the embryo's gender at day two. In some further embodiments, the method of the invention may enable determining the embryo's gender at day three. In further embodiments, the method of the invention may enable determining the embryo's gender at day four. In yet some further embodiments, the method of the invention may enable determining the embryo's gender at day five. In further embodiments, the method of the invention may enable determining the embryo's gender at day six. In further embodiments, the method of the invention may enable determining the embryo's gender at day seven. further embodiments, the method of the invention may enable determining the embryo's gender at day eight. In some further embodiments, the method of the invention may enable determining the embryo's gender at day nine. In yet some further embodiments, the method of the invention may enable determining the embryo's gender at day ten. In some specific the method of the invention may enable determining the embryo's gender between days 1 to 5 of the embryonic development.

As noted above, the method of the invention may be applicable for fertilized unhatched eggs. The term **"fertilized egg"** refers hereinafter to an egg laid by a hen wherein the hen has been mated by a rooster within two weeks, allowing deposit of male sperm into the female infundibulum and fertilization event to occur upon release of the ovum from the ovary. **"Unhatched egg"** as used herein, relates to an egg containing an embryo (also referred to herein as a fertile egg) within a structurally integral (not broken) shell.

The method of the invention is based on determination of a detectable signal formed by a reporter gene integrated into specific loci of the transgenic avian female or male laying the examined egg.

The **"Integration of foreign or exogenous DNA/gene into chromosome"** as used herein, refers hereinafter to a permanent modification of the nucleotide sequence of an organism chromosome. This modification is further transferred during cell division and if occurring in germinal cell lines, it will be transmitted also to offspring. In this case, the integrated reporter gene may be transferred to the embryo within the unhatched egg. The term **"exogenous"** as used herein, refers to originating from outside an organism that has been introduced into an organism for example by transformation or transfection with specifically manipulated vectors, viruses or any other vehicle. The integrated exogenous gene according to certain embodiments, may be a reporter gene. The term **"reporter gene"** relates to gene which encodes a polypeptide, whose expression can be detected in a variety of known assays and wherein the level of the detected signal indicates the presence of said reported.

As noted above, the exogenous reporter gene may be integrated into the avian gender chromosomes Z or W. The avian **"gender chromosome Z or W"** as used herein refers to the chromosomal system that determines the sex of offspring in chicken wherein males are the homogametic sex (ZZ), while females are the heterogametic sex (ZW). The presence of the W chromosome in the ovum determines the sex of the offspring while the Z chromosome is known to be larger and to possess more genes.

The method of the invention is based on the detection of a detectable signal that indicates and reflects the presence of the reporter gene and thereby the presence of a specific gender chromosome. **"Detectable signal"** refers hereinafter to a change in that is perceptible either by observation or instrumentally. Without limitations, the signal can be detected directly. In some embodiments, detectable response is an optical signal.

It should be appreciated that in some specific embodiments, at least one transgenic avian subject provided by the method of the invention, may comprise at least two different reporter genes, each reporter gene may be integrated into at least one position or location in one of gender chromosome Z or W. In case of at least two different reporter genes, in some embodiments, each of the gender chromosomes may be labeled differently. The evaluation of the detectable signal formed, may indicate the gender of the examined embryo.

In the method of the invention, the reporter gene comprised within the transgenic avian of the invention red Fluorescent Protein (RFP). Thus, in some embodiments, the assay measures the levels of light emitted upon excitation, specifically, with the appropriate light source.

The term **"fluorescence"** refers to the emission of light by a substance that has been illuminated, absorbed light or other electromagnetic radiation. It is a form of luminescence i.e. an emission of light by a substance not resulting from heat.

A **"fluorescent protein"** refers to a protein that exhibits bright fluorescence when exposed to light. Fluorescent proteins have particular wavelength for the peak of the illumination excitation intensity and wavelength for the peak of fluorescence emission intensity. Excitation refers to the illumination of a fluorescent protein. In some embodiment, the excitation may be provided by a laser. In yet some embodiments, the signal may be detected upon the usage of an appropriate optical filter.

In the method of the invention, the fluorescent reporter gene is red Fluorescent Protein (RFP). Other fluorescent reporter genes described herein include cyan fluorescent protein (CFP), yellow fluorescent protein (YFP), and auto-fluorescent proteins including blue fluorescent protein (BFP).

It should be noted that in some further examples, any fluorescent protein may be applicable in the methods described herein. More specifically, mutagenesis efforts in the original *Aequorea victoria* jellyfish green fluorescent protein have resulted in new fluorescent probes that range in color from blue to yellow, that may be applicable according to some examples. Longer wavelength fluorescent proteins, emitting in the orange and red spectral regions, have been developed from the marine anemone, *Discosoma striata,* and reef corals belonging to the class **Anthozoa.** Still other species have been mined to produce similar proteins having cyan, green, yellow, orange, and deep red fluorescence emission.

In some examples, the reporter gene that may be applicable in the methods, transgenic avian subjects, constructs, cells and kits may be the yellow fluorescent proteins. More specifically, the family of yellow fluorescent proteins was initiated after the crystal structure of green fluorescent protein revealed that threonine residue 203 **(Thr203)** was near the chromophore. Mutation of this residue to tyrosine was introduced to stabilize the excited state dipole moment of the chromophore and resulted in a 20-nanometer shift to longer wavelengths for both the excitation and emission spectra. Further refinements led to the development of the enhanced yellow fluorescent protein **(EYFP),** which is one of the brightest and most widely used fluorescent proteins.

The **Citrine** variant of yellow fluorescent protein is very bright relative to EYFP and may be also applicable in some examples. Another derivative, named **Venus,** is the fastest maturing and one of the brightest yellow variants developed to date. The coral reef protein, **ZsYellow1**, originally cloned from a *Zoanthus* species native to the Indian and Pacific oceans, produces true yellow emission and is also applicable in certain examples described herein.

In yet some further examples, the reporter gene that may be applicable in the methods, transgenic avian subjects, constructs, cells and kits may be the blue fluorescent proteins. The blue and cyan variants of green fluorescent protein resulted from direct modification of the tyrosine residue at position 66 **(Tyr66)** in the native fluorophore. Conversion of this amino acid to histidine results in blue emission having a wavelength maxima at 450 nanometers, whereas conversion to tryptamine results in a major fluorescence peak around 480 nanometers along with a shoulder that peaks around 500 nanometers. More specifically, among the improved cyan fluorescent proteins that have been introduced, **AmCyan1** and an enhanced cyan variant termed **Cerulean** show the most promise. Derived from the reef coral, *Anemonia majano*, the AmCyan1 fluorescent protein variant may be also applicable for certain examples described herein.

In some specific embodiments, the reporter gene that may be applicable in the methods, transgenic avian subjects, constructs, cells and kits of the invention may be the red fluorescent proteins. The first coral-derived fluorescent protein to be extensively utilized was derived from *Discosoma striata* and is commonly referred to as **DsRed.** Once fully matured, the fluorescence emission spectrum of DsRed features a peak at 583 nanometers whereas the excitation spectrum has a major peak at 558 nanometers and a minor peak around 500 nanometers. Still further, other variants that may be applicable in the present invention include but are not limited to **DsRed2, DsRed-Express** and **RedStar.** Several additional red fluorescent proteins showing a considerable amount of promise have been isolated from the reef coral organisms. One of these proteins that may be applicable in the present invention, may be the **HcRed1,** which was isolated from *Heteractis crispa* and is now commercially available. HcRed1 was originally derived from a non-fluorescent chromoprotein that absorbs red light through mutagenesis to produce a weakly fluorescent obligate dimer having an absorption maximum at 588 nanometers and an emission maximum of 618 nanometers.

Still further, in some embodiments, the reporter gene that may be applicable in the methods, transgenic avian subjects, constructs, cells and kits of the invention may be any fluorescent protein, provided that such protein is not the GFP, and in some more specific embodiments, not the Wild type GFP. However, in some embodiments, several mutants and variants of GFP that form the red-emitting spectral species with excitation and emission maxima at 555 and 585nm, may be also applicable in the present invention. In yet some further specific embodiments, such mutants or variants may comprise Serine 65 and/or Asparagine 68 residues. In yet some further embodiments, such mutants or variants of GFP may comprise mutation of at least one of F46L, V163A and I167V. In yet some further embodiments the variant may comprise any combination of Ser65, Asn68, F46L, V163A and I167V.

Examples of fluorescent proteins are disclosed in **Table 1** herein after.

**Table 1:** *fluorescent proteins:*

| Protein (Acronym) | Excitation Maximum (nm) | Emission Maximum (nm) |
|---|---|---|
| GFP (wt) | 395/475 | 509 |
| **Green Fluorescent Proteins** | | |
| EGFP | 484 | 507 |
| Emerald | 487 | 509 |
| Superfolder GFP | 485 | 510 |
| Azami Green | 492 | 505 |
| mWasabi | 493 | 509 |
| TagGFP | 482 | 505 |
| TurboGFP | 482 | 502 |
| AcGFP | 480 | 505 |
| ZsGreen | 493 | 505 |
| T-Sapphire | 399 | 511 |
| **Blue Fluorescent Proteins** | | |
| EBFP | 383 | 445 |
| EBFP2 | 383 | 448 |
| Azurite | 384 | 450 |
| mTagBFP | 399 | 456 |
| **Cyan Fluorescent Proteins** | | |
| ECFP | 439 | 476 |
| mECFP | 433 | 475 |
| Cerulean | 433 | 475 |
| mTurquoise | 434 | 474 |
| CyPet | 435 | 477 |
| AmCyan1 | 458 | 489 |
| Midori-Ishi Cyan | 472 | 495 |
| TagCFP | 458 | 480 |
| mTFP1 (Teal) | 462 | 492 |
| **Yellow Fluorescent Proteins** | | |
| EYFP | 514 | 527 |
| Topaz | 514 | 527 |
| Venus | 515 | 528 |
| mCitrine | 516 | 529 |
| YPet | 517 | 530 |
| TagYFP | 508 | 524 |
| PhiYFP | 525 | 537 |

(continued)

| Yellow Fluorescent Proteins | | |
|---|---|---|
| ZsYellow1 | 529 | 539 |
| mBanana | 540 | 553 |
| **Orange Fluorescent Proteins** | | |
| Kusabira Orange | 548 | 559 |
| Kusabira Orange2 | 551 | 565 |
| mOrange | 548 | 562 |
| mOrange2 | 549 | 565 |
| dTomato | 554 | 581 |
| dTomato-Tandem | 554 | 581 |
| TagRFP | 555 | 584 |
| TagRFP-T | 555 | 584 |
| DsRed | 558 | 583 |
| DsRed2 | 563 | 582 |
| DsRed-Express (T1) | 555 | 584 |
| DsRed-Monomer | 556 | 586 |
| mTangerine | 568 | 585 |
| **Red Fluorescent Proteins** | | |
| mRuby | 558 | 605 |
| mApple | 568 | 592 |
| mS trawberry | 574 | 596 |
| AsRed2 | 576 | 592 |
| mRFP1 | 584 | 607 |
| JRed | 584 | 610 |
| mCherry | 587 | 610 |
| HcRed1 | 588 | 618 |
| mRaspberry | 598 | 625 |
| dKeima-Tandem | 440 | 620 |
| HcRed-Tandem | 590 | 637 |
| mPlum | 590 | 649 |
| AQ143 | 595 | 655 |

As surprisingly shown by Examples 1 and 2, the auto fluorescent properties of the egg reveled by the present invention allow only the detection of embryos that carry cells expressing RFP.

Thus, the reporter gene of the method of the present invention is Red Fluorescent Protein (RFP). The term **"Red Fluorescent Protein"** or "RFP" refers hereinafter to a fluorescent protein that emits orange, red, and far-red fluorescence that has been isolated from anthozoans (corals) and anemones as well as any variants thereof. There are two main types of RFP proteins, DsRed and Kaede. DsRed-like RFPs are derived from *Discosoma striata* and include but are not limited to mCherry, zFP538, mKO, mOrange, mRouge, E2-Crimson, mNeptune, TagRFP657, Keima, mKate, mStrawberry, mBanana, mHoneydew, nitangerine, mRaspberry, mPlum, mRFPmars and mRFPruby.

On the other end, Kaede is a natural fluorescent protein found in the stony coral *Trachyphyllia geoffroyi,* which irreversibly changes its emission wavelength from green (518 nm) to red (582 nm) upon irradiation at ~400 nm. Kaede family members comprise for example EosFP, dendFP, mcavRFP, and rfloRFP, found respectively in the corals *Lobophyllia hemprichii, Dendronephthya, Monastrea cavernosa,* and *Ricordea florida.* It should be appreciated that any of the RFPs described herein, of any source known in the art, may be applicable for the methods, transgenic animals, cells, kits and devices of the invention.

In yet some specific embodiments, the RFP that may be used by the methods of the invention may be the RFP used in the pDsRed1-N1, that encodes a novel red fluorescent protein [RFP; Matz, M. V., et al. (1999) Nature Biotech. 17:969-973] that has been optimized for high expression in mammalian cells (excitation maximum = 558 nm; emission maximum = 583 nm). RFP was isolated from an IndoPacific sea anemone-relative, Discosoma sp; DsRed1's coding sequence contains 144 silent base pair changes, which correspond to human codon-usage preferences for high expression in mammalian cells.

In some embodiments, the RFP used by the methods and kits of the invention may be encoded by a nucleic acid sequence comprising the sequence as denoted by SEQ ID NO. 20. In yet some further embodiments, such RFP may comprise the amino acid sequence as denoted by SEQ ID NO. 21, or any homologs, variants, mutants or derivatives thereof. In yet some alternative specific embodiments, the RFP used by the invention may be the RFP encoded by the nucleic acid sequence as disclosed by GenBank: AF272711.1, having the amino acid sequence as disclosed by GenBank: AAG16224.1. In yet some further specific embodiments, the RFP used by the methods and kits of the invention may be encoded by a nucleic acid sequence comprising the sequence as denoted by SEQ ID NO. 22. In yet some further embodiments, such RFP may comprise the amino acid sequence as denoted by SEQ ID NO. 23, or any homologs, mutants or derivatives thereof.

It should be noted that in some examples, the reporter gene used by the methods described herein may be any fluorescent protein, provided that said fluorescent protein is not green fluorescent protein (GFP). In other words, in some specific examples the reporter gene integrated into the gender chromosome of the transgenic animal provided by the methods described herein may be any fluorescent protein with the proviso that said fluorescent protein is not GFP.

Still further, in some specific and particular embodiments, when the reporter gene is RFP, it should be noted that in some embodiments, the excitation wavelengths of RFPs are about 500-650 nm while the emission wavelengths may be about 550-650 nm.

In some other embodiments, detecting a detectable signal by the method of the invention may further comprise the step of subjecting said egg to an appropriate light source. In yet some further embodiments, exposure of the egg to an appropriate light source comprising wavelength of between about 400 to about 650. In yet some further embodiments, light of any wavelength may be used with the proviso that said light source is not Ultra-Violet (UV) light (wavelength 10-400 nm).

In some specific embodiment, the step of subjecting said egg to a light source is excitation of the fluorescent protein. In some particular embodiments, the excitation wavelength is between about 500 nm and about 650 nm. In some further embodiments, the excitation wavelength is about 510, 515, 520, 525, 530, 535, 540, 545, 550, 555, 560, 565, 570, 575, 580, 585, 590, 595, 600, 605, 610, 615, 620, 625, 630, 635, 640 nm.

In some more specific embodiment, the excitation wavelength may range between about 515 to about 555. In yet some further embodiments, the wavelength may be 532 nm. In some specific and non-limiting embodiment, the light source may be provided by a laser.

As used herein, the term "laser" refers to electromagnetic radiation of any frequency that is amplified by stimulated emission of radiation. A laser also refers to a device that emits electromagnetic radiation through a process called stimulated emission. Laser light is usually spatially coherent, which means that the light either is emitted in a narrow, low-divergence beam, or can be converted into one with the help of optical components such as lenses.

In some other embodiments, the laser may be a blue laser, or a red laser. In more specific embodiments, the light source is a green laser.

In certain embodiments, the laser may be employed with a filter.

As used herein, the term "filter" refers to "optical filter" which corresponds to a device that selectively transmits light of different wavelengths, usually implemented as plane glass or plastic devices in the optical path which are either dyed in the bulk or have interference coatings.

In some embodiments, the filter may be a green filter that transmits above 500 nm, or a dark green filter that transmits between 540 nm and 580 nm, or a red filter that transmits between 590 nm and 650 nm, or a red filter that transmits above 650 nm or above 660 nm.

In some particular embodiments, the light source may be a green laser with a wavelength of 532 nm. In some further embodiments, such laser may be provided with a red filter that transmits above 650 nm.

The chicken germ-line develops from a small population of primordial germ cells, migrating to the genital ridge from an extragonadal site, while undergoing phases of active migration, as well as passive circulation in the bloodstream. PGCs are located in the center of the epiblast of freshly laid, un-incubated egg, a developmental stage referred as stage X. During incubation PGCs migrate anteriorly and accumulate at the germinal crescent of stage 10HH embryo (approximately 33-38 hours of incubation), considered the main site for intravasation. Later, PGCs can be found in the circulation from stage 12HH to 17HH (approximately from 45 to 64 hours of incubation), reaching a peak concentration in stage 14HH (approximately 50-53 hours of incubation). PGCs leave the circulation at a site adjacent to the gonad anlage at the intermediate mesoderm, where they can be found as early as stage 15HH (55-55 hours of incubation). PGCs reach the genital ridge by active migration along the dorsal mesentery, and colonize both gonads, where they later differentiate into spermatogonia or oogonia.

Embryo is positioned on top of the yolk sac. The yolk is freely rotating in the egg, and the embryo always be facing the upper side of the yolk. During incubation, the blunt end of the egg (where the air sac is located) is facing up, and eggs are occasionally rotated in 90°. As the embryo develops, formation of extra-embryonic tissues retracts the embryo away from the eggshell, and the embryo is less accessible.

Thus, in some specific embodiments, the egg is exposed to said light source. In yet some further embodiments, the egg is placed in a position facilitating exposure of the embryo at any stage to the light source. In yet some further embodiments, a region containing the upper face of the egg yolk at stage X of the egg is excited with the light source.

In some further embodiments, said step of subjecting said egg to a light source is provided by a system, apparatus or a device that may comprise a laser source, a stand for the egg, a lens, a filter, a stand for a detector and a detector.

As used herein, the term "detector" refers to any type of device that detects and/or measures light. In some embodiments, it should be noted that the detectable signal, specifically, the fluorescent signal may be detected using suitable fluorescent means. In some embodiments, the detectable signal formed by the RFP reporter gene may be detected by light sensitive apparatus such as modified optical microscopes or Charge Coupled Device (CCD), a highly sensitive photon detector.

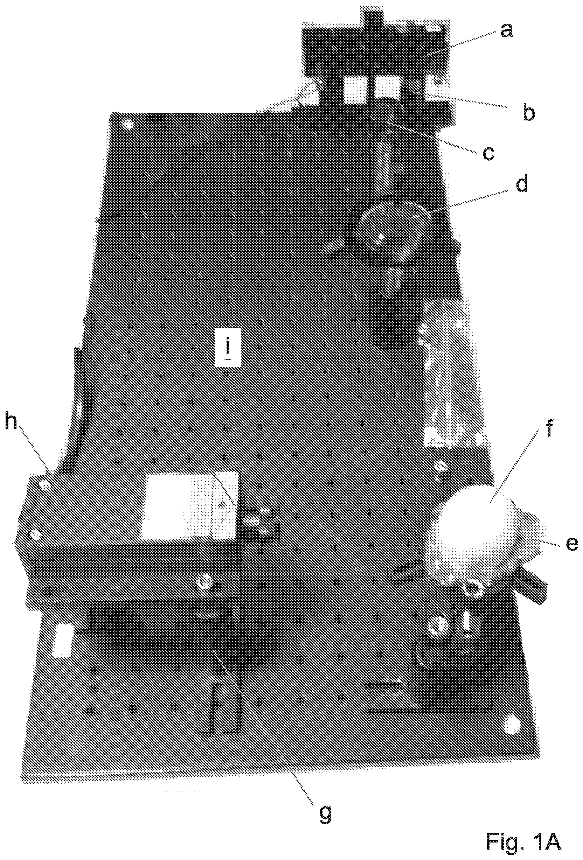

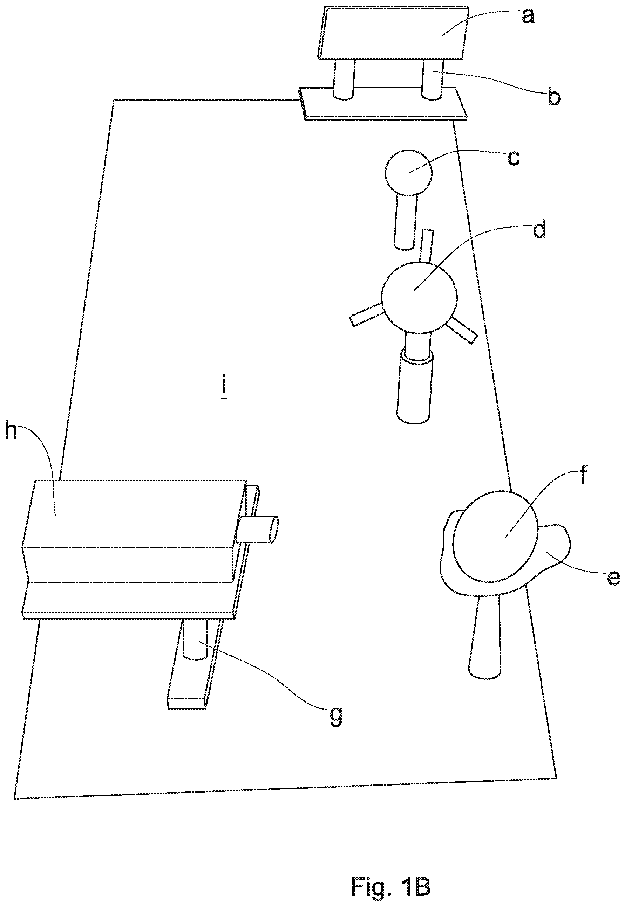

In yet some further embodiments, the methods of the invention may be performed using any device, apparatus or system that provides at least one light source, at least one detector, at least one filter and at least one holding arm that places the egg in an appropriate position facilitating the exposure of cells expressing the reporter gene of the invention to said light source. In some particular and non-limiting embodiments, the method of the invention may use a device as illustrated in Figure 1. In yet some further embodiments, the distance between the egg holder and lens and/or lens and detector may range between about 1 to 100 cm, specifically, 1, 5, 10, 15, 20, 25, 30, 35, 40, 45, 50, 55, 60, 65, 70, 75, 80, 85, 90, 95 and 100 cm, specifically between about 5 to 25 cm, more specifically, about 20 cm.

It should be therefore appreciated that in some embodiments, the methods of the invention may further comprise the step of providing a system or device, for example, as described herein, that is adapted for determining the existence of a detectable signal in an examined egg. Specifically, an apparatus or device comprising a laser source, a stand for the egg, a lens, a filter, a stand for a detector and a detector. As indicated above, in some embodiments for such device, a green laser source that display an excitation wavelength of about 532nm may be used. In yet some further embodiments, a filter, specifically a red filter, that transmits above 650nm, may be used.

In still further embodiments, the at least one transgenic avian subject or animal provided by the method of the invention may be a female avian subject or animal. In more specific embodiments, where the at least one reporter gene is integrated into at least one position of female chromosome Z, detection of a detectable signal indicates that the embryo in the unhatched egg is a male. More specifically, an embryo containing the paternal unlabeled Z gender chromosome and further contains the maternal labeled Z chromosome, contains two Z chromosomes and therefore is a male.

In yet some further embodiments, at least one transgenic avian subject or animal provided by the methods of the invention may be a female avian subject or animal. In some specific embodiments, where the at least one reporter gene is integrated into at least one position of female chromosome W, detection of a detectable signal, indicates that the embryo in the unhatched egg is female. More specifically, the embryo contains a parental unlabeled z chromosome and a labeled maternal W chromosome is an heterogametic embryo containing the WZ chromosomes that is a female.

In yet some further embodiments, the transgenic animal provided by the methods of the invention may be a male subject having the reporter gene integrated into the Z chromosomes thereof. In such case, a detectable signal determined in an egg fertilized by such transgenic male or any sperms thereof, indicates that the embryo carries a paternal Z chromosome comprising the transgenic reporter gene, and is therefore male. In still further embodiments, detection of a detectable signal in an egg laid by a transgenic female avian fertilized by a transgenic male avian, both carrying the reporter gene of the invention integrated into the Z chromosomes thereof, may indicate in case of an intense signal that the embryo carries two copies of a reporter gene integrated into the female and male Z chromosomes thereof. In case of a less intense signal, for example, a signal with reduced intensity of about 50%. The egg may be determined as a female.

As indicated herein before, the method of the invention involves the provision of transgenic avian animals. The preparation of transgenic avian animals, requires the use of genetic engineering approach that may use specific gene editing compound or component. Non-limiting examples for gene editing components may comprise in some embodiments, the use of nucleases.

In some specific embodiments, the nucleases applicable in the methods of the invention may be RNA guided DNA binding protein nucleases.

As used herein, an **RNA guided DNA binding protein nuclease** is a nuclease which is guided to its cleavage site (or alternatively, a site for any other alternative activity), by an RNA molecule. This RNA molecule is referred as a guide RNA.

Thus, in yet more specific embodiments, the at least one reporter gene may be integrated into the gender chromosome of the transgenic avian subject or animal provided by the method of the invention using at least one programmable engineered nuclease (PEN). The term **"programmable engineered nucleases (PEN)"** as used herein, refers to synthetic enzymes that cut specific DNA sequences, derived from natural occurring nucleases involved in DNA repair of double strand DNA lesions and enabling direct genome editing.

In some more specific embodiments, PEN used by the methods of the invention may be any one of a clustered regularly interspaced short palindromic repeat (CRISPR) Class 2 or Class 1 system.

More specifically, the Clustered Regularly Interspaced Short Palindromic Repeats (CRISPR) Type II system is a bacterial immune system that has been modified for genome engineering.

It should be appreciated however that other genome engineering approaches, like zinc finger nucleases (ZFNs) or transcription-activator-like effector nucleases (TALENs) that relay upon the use of customizable DNA-binding protein nucleases that require design and generation of specific nuclease-pair for every genomic target may be also applicable herein.

**CRISPR-Cas systems fall into two classes.** Class 1 systems use a complex of multiple Cas proteins to degrade foreign nucleic acids. Class 2 systems use a single large Cas protein for the same purpose. More specifically, Class 1 may be divided into types I, III, and IV and class 2 may be divided into types II, V, and VI.

As used herein, CRISPR arrays also known as SPIDRs (Spacer Interspersed Direct Repeats) constitute a family of recently described DNA loci that are usually specific to a particular bacterial species. The CRISPR array is a distinct class of interspersed short sequence repeats (SSRs) that were first recognized in *E. coli.* In subsequent years, similar CRISPR arrays were found in *Mycobacterium tuberculosis, Haloferax mediterranei, Methanocaldococcus jannaschii, Thermotoga maritima* and other bacteria and archaea. It should be understood that the invention contemplates the use of any of the known CRISPR systems, particularly and of the CRISPR systems disclosed herein. The CRISPR-Cas system has evolved in prokaryotes to protect against phage attack and undesired plasmid replication by targeting foreign DNA or RNA. The CRISPR-Cas system, targets DNA molecules based on short homologous DNA sequences, called spacers that exist between repeats. These spacers guide CRISPR-associated (Cas) proteins to matching (and/or complementary) sequences within the foreign DNA, called proto-spacers, which are subsequently cleaved. The spacers can be rationally designed to target any DNA sequence. Moreover, this recognition element may be designed separately to recognize and target any desired target. With respect to CRISPR systems, as will be recognized by those skilled in the art, the structure of a naturally occurring CRISPR locus includes a number of short repeating sequences generally referred to as "repeats". The repeats occur in clusters and are usually regularly spaced by unique intervening sequences referred to as "spacers." Typically, CRISPR repeats vary from about 24 to 47 base pair (bp) in length and are partially palindromic. The spacers are located between two repeats and typically each spacer has unique sequences that are from about 20 or less to 72 or more bp in length. Thus, in certain embodiments the CRISPR spacers used in the sequence encoding at least one gRNA of the methods and kits of the invention may comprise between 10 to 75 nucleotides (nt) each. More specifically, about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75 or more. In some specific embodiments the spacers comprise about 20 to 25 nucleotides, more specifically, about 20 nucleobases.

In addition to at least one repeat and at least one spacer, a CRISPR locus also includes a leader sequence and optionally, a sequence encoding at least one tracrRNA. The leader sequence typically is an AT-rich sequence of up to 550 bp directly adjoining the 5' end of the first repeat.

In some specific embodiment, the PEN used by the methods of the invention may be a CRISPR Class 2 system. In yet some further particular embodiments, such class 2 system may be a CRISPR type II system.

More specifically, three major types of CRISPR-Cas system are delineated: Type I, Type II and Type III.

**The type II CRISPR-Cas systems** include the 'HNH'-type system (Streptococcus-like; also known as the Nmeni subtype, for *Neisseria meningitidis serogroup A str.* Z2491, or CASS4), in which Cas9, a single, very large protein, seems to be sufficient for generating crRNA and cleaving the target DNA, in addition to the ubiquitous Cas1 and Cas2. Cas9 contains at least two nuclease domains, a RuvC-like nuclease domain near the amino terminus and the HNH (or McrA-like) nuclease domain in the middle of the protein, but the function of these domains remains to be elucidated. However, as the HNH nuclease domain is abundant in restriction enzymes and possesses endonuclease activity responsible for target cleavage.

Type II systems cleave the pre-crRNA through an unusual mechanism that involves duplex formation between a tracrRNA and part of the repeat in the pre-crRNA; the first cleavage in the pre-crRNA processing pathway subsequently occurs in this repeat region. Still further, it should be noted that type II system comprise at least one of *cas9, cas1, cas2 csn2, and cas4* genes. It should be appreciated that any type II CRISPR-Cas systems may be applicable in the present invention, specifically, any one of type II-A or B. Thus, in yet some further and alternative embodiments, the at least one *cas* gene used in the methods and kits of the invention may be at least one *cas* gene of type II CRISPR system (either typeII-A or typeII-B). In more particular embodiments, at least one *cas* gene of type II CRISPR system used by the methods and kits of the invention may be the *cas9* gene. It should be appreciated that such system may further comprise at least one of *cas1, cas2, csn2* and *cas4* genes.

Double-stranded DNA (dsDNA) cleavage by Cas9 is a hallmark of ***"type II CRISPR-Cas"*** immune systems. The CRISPR-associated protein Cas9 is an RNA-guided DNA endonuclease that uses RNA:DNA complementarity to identify target sites for sequence-specific double stranded DNA (dsDNA) cleavage, creating the double strand brakes (DSBs) required for the HDR that results in the integration of the reporter gene into the specific target sequence, for example, a specific target within the avian gender chromosomes W and Z. The targeted DNA sequences are specified by the CRISPR array, which is a series of about 30 to 40 bp spacers separated by short palindromic repeats. The array is transcribed as a pre-crRNA and is processed into shorter crRNAs that associate with the Cas protein complex to target complementary DNA sequences known as proto-spacers. These proto-spacer targets must also have an additional neighboring sequence known as a proto-spacer adjacent motif (PAM) that is required for target recognition. After binding, a Cas protein complex serves as a DNA endonuclease to cut both strands at the target and subsequent DNA degradation occurs via exonuclease activity.

CRISPR type II system as used herein requires the inclusion of two essential components: a "guide" RNA (gRNA) and a non-specific CRISPR-associated endonuclease (Cas9). The gRNA is a short synthetic RNA composed of a "scaffold" sequence necessary for Cas9-binding and about 20 nucleotide long "spacer" or "targeting" sequence which defines the genomic target to be modified. Thus, one can change the genomic target of Cas9 by simply changing the targeting sequence present in the gRNA. Guide RNA (gRNA), as used herein refers to a synthetic fusion of the endogenous bacterial crRNA and tracrRNA, providing both targeting specificity and scaffolding/binding ability for Cas9 nuclease. Also referred to as "single guide RNA" or "sgRNA". CRISPR was originally employed to "knock-out" target genes in various cell types and organisms, but modifications to the Cas9 enzyme have extended the application of CRISPR to "knock-in" target genes, selectively activate or repress target genes, purify specific regions of DNA, and even image DNA in live cells using fluorescence microscopy. Furthermore, the ease of generating gRNAs makes CRISPR one of the most scalable genome editing technologies and has been recently utilized for genome-wide screens.

The target within the genome to be edited, specifically, the specific target loci within the gender chromosomes Z or W, where the reporter gene of the invention is to be integrated, should be present immediately upstream of a Protospacer Adjacent Motif (PAM).

The PAM sequence is absolutely necessary for target binding and the exact sequence is dependent upon the species of Cas9 (5' NGG 3' for *Streptococcus pyogenes* Cas9). In certain embodiments, Cas9 from S. *pyogenes* is used in the methods and kits of the invention. Nevertheless, it should be appreciated that any known Cas9 may be applicable. Non-limiting examples for Cas9 useful in the present disclosure include but are not limited to *Streptococcus pyogenes* (SP), also indicated herein as SpCas9, *Staphylococcus aureus* (SA), also indicated herein as SaCas9, *Neisseria meningitidis* (NM), also indicated herein as NmCas9, *Streptococcus thermophilus* (ST), also indicated herein as StCas9 and *Treponema denticola* (TD), also indicated herein as TdCas9. In some specific embodiments, the Cas9 of Streptococcus pyogenes M1 GAS, specifically, the Cas9 of protein id: AAK33936.1, may be applicable in the methods and kits of the invention. In some embodiments, the Cas9 protein may be encoded by the nucleic acid sequence as denoted by SEQ ID NO. 24. In further specific and non-limiting embodiments, the Cas9 protein may comprise the amino acid sequence as denoted by SEQ ID NO. 25, or any derivatives, mutants or variants thereof. Once expressed, the Cas9 protein and the gRNA, form a riboprotein complex through interactions between the gRNA "scaffold" domain and surface-exposed positively-charged grooves on Cas9. Cas9 undergoes a conformational change upon gRNA binding that shifts the molecule from an inactive, non-DNA binding conformation, into an active DNA-binding conformation. Importantly, the "spacer" sequence of the gRNA remains free to interact with target DNA. The Cas9-gRNA complex binds any genomic sequence with a PAM, but the extent to which the gRNA spacer matches the target DNA determines whether Cas9 will cut. Once the Cas9-gRNA complex binds a putative DNA target, a "seed" sequence at the 3' end of the gRNA targeting sequence begins to anneal to the target DNA. If the seed and target DNA sequences match, the gRNA continues to anneal to the target DNA in a 3' to 5' direction.

Cas9 will only cleave the target if sufficient homology exists between the gRNA spacer and target sequences. Still further, the Cas9 nuclease has two functional endonuclease domains: RuvC and HNH. Cas9 undergoes a second conformational change upon target binding that positions the nuclease domains to cleave opposite strands of the target DNA. The end result of Cas9-mediated DNA cleavage is a double strand break (DSB) within the target DNA that occurs about 3 to 4 nucleotides upstream of the PAM sequence.

The resulting DSB may be then repaired by one of two general repair pathways, the efficient but error-prone Non-Homologous End Joining (NHEJ) pathway and the less efficient but high-fidelity Homology Directed Repair (HDR) pathway. In some embodiments, the insertion that results in the specific integration of the reporter gene of the invention to the specific target loci within the gender chromosomes W or Z, is a result of repair of DSBs caused by Cas9. In some specific embodiments, the reporter gene of the invention is integrated, or knocked-in the target loci by HDR.

The term **"Homology directed repair (HDR)",** as used herein refers to a mechanism in cells to repair double strand DNA lesions. The most common form of HDR is homologous recombination. The HDR repair mechanism can only be used by the cell when there is a homologue piece of DNA present in the nucleus, mostly in G2 and S phase of the cell cycle. When the homologue DNA piece is absent, another process called non-homologous end joining (NHEJ) can take place instead. Programmable engineered nucleases (PEN) strategies for genome editing, are based on cell activation of the HDR mechanism following specific double stranded DNA cleavage.

As discussed previously, Cas9 generates double strand breaks (DSBs) through the combined activity of two nuclease domains, RuvC and HNH. The exact amino acid residues within each nuclease domain that are critical for endonuclease activity are known (D10A for HNH and H840A for RuvC in *S. pyogenes* Cas9) and modified versions of the Cas9 enzyme containing only one active catalytic domain (called "Cas9 nickase") have been generated. Cas9 nickases still bind DNA based on gRNA specificity, but nickases are only capable of cutting one of the DNA strands, resulting in a "nick", or single strand break, instead of a DSB. DNA nicks are rapidly repaired by HDR (homology directed repair) using the intact complementary DNA strand as the template. Thus, two nickases targeting opposite strands are required to generate a DSB within the target DNA (often referred to as a "double nick" or "dual nickase" CRISPR system). This requirement dramatically increases target specificity, since it is unlikely that two off-target nicks will be generated within close enough proximity to cause a DSB. It should be therefore understood, that the invention further encompasses the use of the dual nickase approach to create a double nick-induced DSB for increasing specificity and reducing off-target effects.

Thus, in certain embodiments, the at least one reporter gene may be integrated into the gender chromosome of the transgenic avian subject, specifically animal by homology directed repair (HDR) mediated by at least one CRISPR/CRISPR-associated endonuclease 9 (Cas9) system.

As indicated above, the CRISPR type II system comprises at least two elements, the nuclease, specifically, the Cas9 and the guide RNA. Therefore, for incorporating and integrating the reporter gene used by the invention, into the gender chromosomes of the transgenic avian subject, in addition to Cas9 or any nucleic acid sequence encoding Cas9, the invention further provides guide RNA or any nucleic acid sequence encoding such gRNA that targets the Cas9 to the target site within a specific gender chromosome. In some further embodiments, the gRNA of the kit of the invention may comprise at least one CRISPR RNA (crRNA) and at least one trans-activating crRNA (tracrRNA).

In some alternative embodiments the kit of the invention may comprise nucleic acid sequence encoding the at least one gRNA. Such nucleic acid sequence may comprise a CRISPR array comprising at least one spacer sequence that targets and is therefore identical to at least one protospacer in a target genomic DNA sequence. It should be noted that the nucleic acid sequence further comprises a sequence encoding at least one tracrRNA.

Still further, in some embodiments, the at least one reporter gene may be integrated into a gender chromosome of the transgenic avian subject, specifically animal by contacting or co-transfecting at least one cell of the avian subject, or animal or at least one cell introduced into the avian subject, or animal, with: (a) elements of the CRISPR Cas9 system, specifically, at least one Cas9 protein or at least one first nucleic acid sequence comprising at least one nucleic acid sequence encoding said at least one Cas9 protein; and at least one guide RNA (gRNA) that targets a protospacer within the gender chromosome Z or W, or at least one nucleic acid sequence encoding said at least one gRNA; and (b) at least one second nucleic acid sequence comprising at least one reporter gene. It should be noted that in some embodiments, in case the elements of the CRISPR Cas9 system of (a) are provided in the form of nucleic acid sequence, they may be provided either in one nucleic acid molecule that comprises the different elements (referred to herein as the "first nucleic acid sequence", or in two or more nucleic acid molecules, each comprises one of the elements indicated above, specifically, sequences encoding the gRNA and Cas9.

Thus, for the preparation of a transgenic avian animal used by the methods of the invention, at least two nucleic acid molecules should be provided.

Still further, a **"gRNA"** or **"targeting RNA"** is an RNA that, when transcribed from the portion of the CRISPR system encoding it, comprises at least one segment of RNA sequence that is identical to (with the exception of replacing T for U in the case of RNA) or complementary to (and thus "targets") a DNA sequence in the target genomic DNA, referred to herein as a protospacer. The CRISPR systems of the present disclosure may optionally encode more than one targeting RNA, and the targeting RNAs be directed to one or more target sequences in the genomic DNA. A **"proto-spacer",** as used herein, refers to the target sequence within the target chromosome. Such proto-spacers comprise nucleic acid sequence having sufficient complementarity to a targeting RNA encoded by the CRISPR spacers comprised within the nucleic acid sequence encoding the gRNA of the methods and kits of the invention.

The methods of the invention, as well as the kits described herein after, provide nucleic acid sequences. As used herein, **"nucleic acids or nucleic acid molecules"** is interchangeable with the term **"polynucleotide(s)"** and it generally refers to any polyribonucleotide or poly-deoxyribonucleotide, which may be unmodified RNA or DNA or modified RNA or DNA or any combination thereof. "Nucleic acids" include, without limitation, single- and double-stranded nucleic acids. As used herein, the term "nucleic acid(s)" also includes DNAs or RNAs as described above that contain one or more modified bases. As used herein, the term **"oligonucleotide"** is defined as a molecule comprised of two or more deoxyribonucleotides and/or ribonucleotides, and preferably more than three. Its exact size will depend upon many factors which in turn, depend upon the ultimate function and use of the oligonucleotide. The oligonucleotides may be from about 8 to about 1,000 nucleotides long. More specifically, the oligonucleotide molecule/s used by the kit of the invention may comprise any one of 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 75, 80, 85, 90, 95, 100, 200, 300, 400, 500, 600, 700, 800, 900, 1000 or more bases in length.

Nucleic acid molecules can be composed of monomers that are naturally-occurring nucleotides (such as DNA and RNA), or analogs of naturally-occurring nucleotides (e.g., alpha-enantiomeric forms of naturally-occurring nucleotides), or modified nucleotides or any combination thereof. Herein this term also encompasses a cDNA, i.e. complementary or copy DNA produced from an RNA template by the action of reverse transcriptase (RNA-dependent DNA polymerase).

In this connection an **"isolated polynucleotide"** is a nucleic acid molecule that is separated from the genome of an organism. For example, a DNA molecule that encodes the reporter gene used by the methods and kits of the invention or any derivatives or homologs thereof, as well as the sequences encoding the CRISPR/Cas9 and gRNAs of the methods and kits of the invention, that has been separated from the genomic DNA of a cell is an isolated DNA molecule. Another example of an isolated nucleic acid molecule is a chemically-synthesized nucleic acid molecule that is not integrated in the genome of an organism. A nucleic acid molecule that has been isolated from a particular species is smaller than the complete DNA molecule of a chromosome from that species.

In some embodiments, the nucleic acid sequences used by the methods and kits (described herein after) of the invention, specifically, nucleic acid sequences comprising sequences encoding the Cas9 and gRNA, or alternatively the reporter gene of the invention, may be provided constructed within a vector, cassette or any other vehicle. The invention thus further relates to recombinant DNA constructs comprising the polynucleotides of the invention, and optionally, further additional elements such as promoters, regulatory and control elements, translation, expression and other signals, operably linked to the nucleic acid sequence of the invention. A non-limiting example for a vector provided by the invention may be any vector that comprises the nucleic acid sequences that encode the Cas9 and specific gRNA required for integration of the reporter gene into the target gender chromosome. In some specific embodiments, the invention provides DNA constructs or vectors that comprise the nucleic acid sequences that encode Cas9 as denoted by SEQ ID NO. 25, and the gRNA7 as denoted by SEQ ID NO. 26, that targets the reporter gene to the gender chromosome Z. In yet some further embodiments, the invention further provides a vector or any DNA construct that comprises the nucleic acid sequence that encodes the reporter gene of the invention, specifically, the RFP, flanked by left and right arms required for recombination and integration into the target locus within the gender chromosome, specifically, the gender chromosome Z. A non-limiting example for such vector may be a vector that comprises the nucleic acid sequences as disclosed in Figure 15. In yet some further embodiments, the nucleic acid sequence that comprise the nucleic acid sequence as denoted by at least one of SEQ ID NO. 66 and/or SEQ ID NO. 67.

Still further, the invention further encompasses any host cell that comprises and/or express the vectors and DNA constructs of the invention, specifically, any of the vectors described by the invention.

As used herein, the terms **"recombinant DNA"**, **"recombinant nucleic acid sequence"** or **"recombinant gene"** refer to a nucleic acid comprising an open reading frame encoding one of the CRISPR system of the invention, specifically, the CRISPR/Cas9 type II, along with the gRNA of the invention that target the Cas9 to the corresponding protospacer in a specific locus within avian chromosomes Z and/or W. In yet some other embodiments, recombinant DNA as used herein further refers to a nucleic acid comprising an open reading frame encoding the reporter gene of the invention, specifically, transgene.

As referred to herein, by the term **"gene"** or **"transgene"** is meant a nucleic acid, either naturally occurring or synthetic, which encodes a protein product. The term "nucleic acid" is intended to mean natural and/or synthetic linear, circular and sequential arrays of nucleotides and nucleosides, e.g., cDNA, genomic DNA (gDNA), mRNA, and RNA, oligonucleotides, oligonucleosides, and derivatives thereof.

The phrase **"operatively-linked"** is intended to mean attached in a manner which allows for transgene transcription. As noted above, the transgene used by the method of the invention is prepared by providing nucleic acid sequence encoding the transgene that is to be incorporated into the gender chromosomes Z or W, specifically, the reporter gene, and a gene editing system (e.g., the CRISP/Cas9 system). The term **"encoding"** is intended to mean that the subject nucleic acid may be transcribed and translated into either the desired polypeptide or the subject protein in an appropriate expression system, e.g., when the subject nucleic acid is linked to appropriate control sequences such as promoter and enhancer elements in a suitable vector (e.g., an expression vector) and when the vector is introduced into an appropriate system or cell.

It should be appreciated that in some embodiments, at least one of the first and the second nucleic acid sequences provided and used by the methods and kits of the invention may be constructed and comprised within a vector. **"Vectors"** or **"Vehicles",** as used herein, encompass vectors such as plasmids, phagemides, viruses, integratable DNA fragments, and other vehicles, which enable the integration of DNA fragments into the genome of the host, or alternatively, enable expression of genetic elements that are not integrated. Vectors are typically self-replicating DNA or RNA constructs containing the desired nucleic acid sequences, and operably linked genetic control elements that are recognized in a suitable host cell and effect the translation of the desired spacers. Generally, the genetic control elements can include a prokaryotic promoter system or a eukaryotic promoter expression control system. Such system typically includes a transcriptional promoter, transcription enhancers to elevate the level of RNA expression. Vectors usually contain an origin of replication that allows the vector to replicate independently of the host cell. In yet some alternative embodiments, the expression vectors used by the invention may comprise elements necessary for integration of the desired reporter gene of the invention into the avian gender specific chromosomes W and/or Z.

Accordingly, the term **"control and regulatory elements"** includes promoters, terminators and other expression control elements. Such regulatory elements are described in Goeddel; [Goeddel., et al., Gene Expression Technology: Methods in Enzymology 185, Academic Press, San Diego, Calif. (1990)]. For instance, any of a wide variety of expression control sequences that control the expression of a DNA sequence when operatively linked to it may be used in these vectors to express DNA sequences encoding any desired protein using the method of this invention.

A vector may additionally include appropriate restriction sites, antibiotic resistance or other markers for selection of vector-containing cells. Plasmids are the most commonly used form of vector but other forms of vectors which serve an equivalent function and which are, or become, known in the art are suitable for use herein. See, e.g., Pouwels et al., Cloning Vectors: a Laboratory Manual (1985 and supplements), Elsevier, N.Y.; and Rodriquez, et al. (eds.) Vectors: a Survey of Molecular Cloning Vectors and their Uses, Buttersworth, Boston, Mass (1988).

To create the transgenic avian animal used by the methods of the invention, an avian cell comprising the reporter gene integrated into specific loci within the gender chromosomes Z or W thereof must be prepared. Such cell may be prepared in some embodiments, by contacting, introducing or co-transfecting the cell of the avian subject or any cell introduced to said subject, with the first and second nucleic acid sequences provided by the methods and kits of the invention or with any construct, vector, vehicle, comprising the same. In some embodiments, nucleic acid sequences encoding the gene editing elements, and nucleic acid sequences encoding the targeted reporter gene, specifically, RFP. **"Transfection"** as used herein is meant the process of inserting genetic material, such as DNA and double stranded RNA, into mammalian cells. The insertion of DNA into a cell enables the expression, or production, of proteins using the cells own machinery. Thus, co-transfection as used herein refers to simultaneous transfection of at least two different nucleic acid molecules or any vector comprising the same to each single cell. Still further, the nucleic acid sequences to be transfected can be transiently expressed for a short period of time, or become incorporated into the genomic DNA, where the change is passed on from cell to cell as it divides.

The invention therefore provides methods for an *in-ovo* gender determination of an avian embryo *in-ovo* based on expression of a reporter gene, specifically, Red Fluorescent Protein (RFP) integrated in the gender chromosome/s thereof (specifically, Z and/or W). **"Expression"** generally refers to the process by which gene-encoded information is converted into the structures present and operating in the cell. Therefore, according to the invention "expression" of a reporter gene, specifically, may refer to transcription into a polynucleotide, translation into a protein, or even posttranslational modification of the protein.

The insertion and specific integration of the reporter gene to the specific target locus within the gender chromosomes of the transgenic avian subject, may involve in some embodiments the provision of the CRISPR/Cas9 system that includes specific gRNA and the nucleic acid sequence of the reporter gene that should be integrated. To facilitate and enable integration, the reporter gene must be flanked in some embodiments, with sequences that may be homolog to the sequences flanking the targeted integration site and thereby enable recombination. Thus, in yet some further specific embodiments, the at least one reporter gene in the second nucleic acid sequence may be flanked at 5' and 3' thereof by homologous arms. It should be appreciated that in some embodiments, these arms are required and therefore facilitate HDR of the reporter gene at the integration site. In more specific embodiments, the reporter gene in the second nucleic acid sequence used by the method of the invention, may be flanked with two arms that are homologous or show homology or identity of about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% to the whole, entire, or complete at least one nucleic acid sequence comprised within the target loci within the gender chromosomes Z or W, that serves as the integration site to facilitate specific integration via HDR. In certain embodiments, the target sequence is also referred to herein as at least one "proto-spacer" that is recognized by the "spacer" sequences that are part of the gRNA used by the invention, and provided by the first nucleic acid sequence.

The term **"Homologous arms",** as used herein refers to HDR templates introduced into specific vectors or viruses, used to create specific mutations or insertion of new elements into a gene, that possess a certain amount of homology surrounding the target sequence to be modified (depending on which PEN is used). In yet some further specific embodiments, where CRISPR is used as a PEN, the arms sequences (left, upstream and right, downstream) may comprise between about 10 to 5000 bp, specifically, between about 50 to 1000 bp, between 100 to 500, specifically, 50, 100, 200, 300, 400, 500, 600, 700, 800, 900, 1000bp.

In yet some further embodiments, the targeting sequence within the gRNA encoded by the first nucleic acid sequence provided by the methods and kits of the invention, also referred to herein as the "spacer" sequence, exhibits homology or identity of about 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% to the entire, complete or whole at least one nucleic acid sequence comprised within the target loci within the gender chromosomes Z or W, referred to herein as the "proto-spacer".

In some embodiments, the at least one reporter gene in the second nucleic acid sequence may be operably linked to any one of a gender specific promoter, an embryonal specific promoter (for example α-Globin Promoter) and an inducible promoter (for example light-inducible promoters derived from the soybean SSU gene, or derived from parsley chalcone synthase CHS promoter, or an engineered version of EL222, a bacterial Light-Oxygen-Voltage protein that activates expression when illuminated with blue light). In yet more specific embodiments, the reporter gene may be under the control of an embryonic promoter, thereby limiting the expression of the transgenic reporter gene to the embryonal stage, with no expression in the adult chick. In such embodiment, the reporter transgene may be used and expressed only at the embryonal stage, for diagnostic purposes.

More specifically, **"Promoter"** as used herein, refers to a particular region of the DNA that has the ability to control the expression of the gene which is placed downstream. Thus, **"Promoter specific for gender in chicks"** refers hereinafter to a promoter that will activate the expression of a gene, only in a specific chick gender (i.e. male or female). Still further, **"Promoter specific for development in chicks"** refers to a promoter that will activate the expression of a gene, only at specific stages of the chick development.

In some specific embodiments, the at least one reporter gene may be inserted and thereby integrated into at least one non-coding region of the target gender chromosome. Such approach avoids the disruption of genes that may be required for development and maturation of the unhatched embryo.

**"Non-coding region"** as used herein, refers to components of an organism's DNA that do not encode protein sequences. Some noncoding DNA region is transcribed into functional non-coding RNA molecules, other functions of noncoding DNA regions include the transcriptional and translational regulation of protein-coding sequences, scaffold attachment regions, origins of DNA replication, centromeres and telomeres. The hypothesized non-functional portion (or DNA of unknown function) has often been referred to as "junk DNA".

In yet some specific embodiments, the at least one reporter gene used by the method of the invention for preparing the transgenic avian animal, may be integrated into at least one site at gender Z chromosome. In more specific embodiments, the specific loci in the Z chromosome may be any one of regions 9156874-9161874, as denoted by SEQ ID NO:15, 27764943-27769943, as denoted by SEQ ID NO: 16, 42172748-42177748, as denoted by SEQ ID NO:17, 63363656-63368656, as denoted by SEQ ID NO:18 and 78777477-78782477, as denoted by SEQ ID NO:19 of Chromosome Z of female chicken. In some specific embodiments, at least one reporter gene may be integrated into at least one site at gender Z chromosome locus 42172748-42177748, specifically, as denoted by SEQ ID NO. 17. In yet some further specific embodiments, a gRNA sequence suitable for integration into specific loci within the Z chromosome, may include but is not limited to gRNA7 directed to a protospacer located within Z chromosome locus chrZ_42174515_-1. In some specific embodiments, the gRNA may be the gRNA designated gRNA7. In more specific embodiments, such gRNA7 may comprise the nucleic acid sequence GTAATACAGAGCTAAACCAG, as also denoted by SEQ ID NO:26. In yet some further embodiments, for integrating the reporter gene of the invention into the specific locus within the Z chromosome, the sequence encoding the reporter gene, specifically, the RFP, provided by the invention, may be flanked by a left arm at the 5' thereof, and a right arm at the 3' thereof. In yet some further specific embodiments, the left arm may comprise the nucleic acid sequence as denoted by SEQ ID NO. 31, and right arm comprising the nucleic acid sequence as denoted by SEQ ID NO. 32, may be used to integrate the reporter gene of the invention to the specific loci directed by gRNA7 of SEQ ID NO:26.

In yet some further specific embodiments, the at least one gRNA required to target the reporter gene to such specific location within the Z chromosome may comprises the nucleic acid sequence as denoted by any one of gRNA3: ACAGACCTATGATATGT, as denoted by SEQ ID NO. 11; gRNA4: CGATTATCACTCACAAG, as denoted by SEQ ID NO. 12; gRNA5: CTGGTTAGCATGGGGAC, as denoted by SEQ ID NO. 13; gRNA6: GTAAAGAGTCAGATACA, as denoted by SEQ ID NO. 14.

In yet some further embodiments, for integrating the reporter gene of the invention into the specific locus within the Z chromosome, left arm comprising the nucleic acid sequence as denoted by SEQ ID NO. 41 and right arm comprising the nucleic acid sequence as denoted by SEQ ID NO. 42 may be used to integrate the reporter gene of the invention to the specific loci directed by gRNA3 of SEQ ID NO: 11. In further embodiments, for integrating the reporter gene of the invention into the specific locus within the Z chromosome, left arm comprising the nucleic acid sequence as denoted by SEQ ID NO. 43, and right arm comprising the nucleic acid sequence as denoted by SEQ ID NO. 44, may be used to integrate the reporter gene of the invention to the specific loci directed by gRNA4 of SEQ ID NO: 12. In still further embodiments, for integrating the reporter gene of the invention into the specific locus within the Z chromosome, left arm comprising the nucleic acid sequence as denoted by SEQ ID NO. 45, and right arm comprising the nucleic acid sequence as denoted by SEQ ID NO. 46, may be used to integrate the reporter gene of the invention to the specific loci directed by gRNA5 of SEQ ID NO: 13. In some further embodiments, for integrating the reporter gene of the invention into the specific locus within the Z chromosome, left arm comprising the nucleic acid sequence as denoted by SEQ ID NO. 47, and right arm comprising the nucleic acid sequence as denoted by SEQ ID NO. 48, may be used to integrate the reporter gene of the invention to the specific loci directed by gRNA6 of SEQ ID NO:14.

Further non-limiting examples for gRNA sequences suitable for integration into specific loci within the Z chromosome, may include but are not limited to gRNA8 of Z chromosome locus chrZ_9157091_1, comprising the nucleic acid sequence ACAGACCTATGATATGTGAG, as also denoted by SEQ ID NO:27, gRNA9 of Z chromosome locus chrZ_27767602_-1, comprising the nucleic acid sequence GAGCTTGTGAGTGATAATCG, as also denoted by SEQ ID NO:28, gRNA10 of Z chromosome locus chrZ_78779927_1, comprising the nucleic acid sequence GTAAAGAGTCAGATACACAG, as also denoted by SEQ ID NO: 29, and gRNAll of Z chromosome locus chrZ_63364946_-1, comprising the nucleic acid sequence CAGTGGGTACTGAAGCTGTG as also denoted by SEQ ID NO: 30.

In further embodiments, for integrating the reporter gene of the invention into the specific locus within the Z chromosome, left arm comprising the nucleic acid sequence as denoted by SEQ ID NO. 33, and right arm comprising the nucleic acid sequence as denoted by SEQ ID NO. 34, may be used to integrate the reporter gene of the invention to the specific loci directed by gRNA8 of SEQ ID NO:27. In still further embodiments, for integrating the reporter gene of the invention into the specific locus within the Z chromosome, left arm comprising the nucleic acid sequence as denoted by SEQ ID NO. 35, and right arm comprising the nucleic acid sequence as denoted by SEQ ID NO. 36, may be used to integrate the reporter gene of the invention to the specific loci directed by gRNA9 of SEQ ID NO:28. In further embodiments, for integrating the reporter gene of the invention into the specific locus within the Z chromosome, left arm comprising the nucleic acid sequence as denoted by SEQ ID NO. 37, and right arm comprising the nucleic acid sequence as denoted by SEQ ID NO. 38, may be used to integrate the reporter gene of the invention to the specific loci directed by gRNA10 of SEQ ID NO:29. In yet a further embodiment, for integrating the reporter gene of the invention into the specific locus within the Z chromosome, left arm comprising the nucleic acid sequence as denoted by SEQ ID NO. 39, and right arm comprising the nucleic acid sequence as denoted by SEQ ID NO. 40, may be used to integrate the reporter gene of the invention to the specific loci directed by gRNA11 of SEQ ID NO:30.

In yet some alternative embodiments, the at least one reporter gene may be integrated into at least one site at gender W chromosome. In more specific embodiments, the specific locus in the W chromosome may be location 1022859-1024215. In some specific embodiments, the target locus may comprise the nucleic acid sequence as denoted by SEQ ID NO. 3.

In more specific embodiments, the at least one gRNA required to target the reporter gene to such specific location within the W chromosome may comprises the nucleic acid sequence as denoted by any one of SEQ ID NO. 1 and 2, these gRNAs are designated herein as gRNA1 and gRNA2, respectively.

In yet some more specific embodiments, the gRNA used by the method of the invention to prepare the transgenic avian female may comprise the nucleic acid sequence as denoted by SEQ ID NO. 1 (gRNA1). In such case, the at least one reporter gene comprised within said second nucleic acid sequence may be flanked at 5' and 3' thereof by homologous arms comprising the amino acid sequence as denoted by SEQ ID NO. 4 and 5, that facilitate the integration thereof to said specific loci in W chromosome, respectively. It should be appreciated that these arms are also referred to herein as left and right arms, respectively.

In yet some alternative embodiments, the gRNA used for preparing the transgenic avian female of the invention may comprise the nucleic acid sequence as denoted by SEQ ID NO. 2 (gRNA2). In such case the at least one reporter gene comprised within the second nucleic acid sequence is flanked at 5' and 3' thereof by homologous arms comprising the amino acid sequence as denoted by SEQ ID NO. 6 and 7, respectively. It should be appreciated that these arms are also referred to herein as left and right arms, respectively.

When genetic loci of zygote cells of an avian host, have been targeted and/or transfected with exogenous sequences, specifically, the reporter gene used by the invention, it may be desirable to use such cells to generate transgenic animals. For such a procedure, following the introduction of the targeting construct into the embryonic stem (ES) cells, the cells may be plated onto a feeder layer in an appropriate medium, for example, DMEM supplemented with growth factors and cytokines, fetal bovine serum and antibiotics. The embryonic stem cells may have a single targeted locus (heterozygotic) or both loci targeted (homozygotic). Cells containing the construct may be detected by employing a selective medium and after sufficient time for colonies to grow, colonies may be picked and analyzed for the occurrence of gene targeting. In some specific embodiments, PCR may be applied to verify the integration of the desired exogenous sequences into the target loci, using primers within and outside the construct sequence. Colonies which show gene targeting may then be used for injection into avian embryos. The ES cells can then be trypsinized and the modified cells can be injected through an opening made in the side of the egg. After sealing the eggs, the eggs can be incubated under appropriate conditions until hatching. Newly hatched avian can be tested for the presence of the target construct sequences, for example by examining a biological sample thereof, e.g., a blood sample. After the avian have reached maturity, they are bred and their progeny may be examined to determine whether the exogenous integrated sequences are transmitted through the germ line.

Chimeric avian are generated which are derived in part from the modified embryonic stem cells or zygote cells, capable of transmitting the genetic modifications through the germ line. Mating avian strains containing exogenous sequences, specifically, the reporter gene used by the invention, or portions thereof, with strains in which the avian wild type loci, or portions thereof, is restored, should result in progenies displaying an *in-ovo* detectable gender.

Still further, transgenic avian can also be produced by other methods, some of which are discussed below. Among the avian cells suitable for transformation for generating transgenic animals are primordial germ cells (PGC), sperm cells and zygote cells (including embryonic stem cells). Sperm cells can be transformed with DNA constructs by any suitable method, including electroporation, microparticle bombardment, lipofection and the like. The sperm can be used for artificial insemination of avian. Progeny of the inseminated avian can be examined for the exogenous sequence as described above.

Alternatively, primordial germ cells may be isolated from avian eggs, transfected with the exogenous reporter gene of the invention by any appropriate method, and transferred or inserted into new embryos, where they can become incorporated into the developing gonads. Hatched avian and their progeny can be examined for the exogenous reporter gene sequence as described by the invention.

In yet another approach, dispersed blastodermal cells isolated from eggs can be transfected by any appropriate means with the exogenous reporter gene sequence, or portions thereof, integrated to the gender specific chromosomes Z or W, followed by injection into the subgerminal cavity of intact eggs. Hatched avian subjects and their progeny may be examined for the exogenous reporter gene as described above.

Chicken primordial germ cells (PGCs) are the precursors for ova and spermatozoa. Thus, in some aspects thereof, the invention provides the production of transgenic chickens via a germline transmission system using PGCs co-transfected with the reporter gene construct and with the CRISPR/Cas9 gRNA construct that directs the integration of the reporter gene into the gender specific chromosomes W and/or Z. PGCs are sorted and transferred into the bloodstream of 2.5-day recipient embryos for germline transmission.

PGCs are the precursors of gametes and show unique migration activity during early embryogenesis in birds, when compared to mammals. In avian embryos, PGCs first arise from the epiblast and migrate to the hypoblast of the germinal crescent at stage 4 (HH), approximately 18-19 h after incubation. Between stages 10 and 12 (HH), PGCs move from the germinal crescent into the bloodstream and migrate through the circulatory system until they reach the genital ridges and colonize the developing gonads. This contrasts with mammals in which PGCs migrate through the embryonic tissues to reach the developing gonads. It is this unique feature of avian PGC migration through the blood stream that has facilitated the major advance in the genetic modification of chickens.

Thus, in some specific embodiments, the "Preparation of transgenic avian animal" refers to a multi-step method involving genetic engineering techniques for production of chicken with genomic modifications wherein a) Primordial Germ Cells (PGCs) are isolated from the blood of two days-old chick embryos; b) a transgene construct is incorporated into cultured PGCs, for example, by using lentiviral system, Piggybac transposon vectors, TALENS or CRISPR/Cas9 techniques; (c) transgenic PGCs are identified and injected into the circulatory system of embryos and migrate to the developing gonads; d) recipient embryos are incubated at 37°C until hatching (d) hatched males are reared to sexual maturity and crossed with wild-type hens (e) offspring are screened to identify those derived from the transgenic PGCs.

Thus, in a second aspect, the invention relates to an avian transgenic animal or subject comprising, in at least one cell thereof, at least one exogenous reporter gene integrated into at least one position or location (also referred to herein as locus) in at least one of gender chromosome Z and W.

The term "avian" relates to any species derived from birds characterized by feathers, toothless beaked jaws, the laying of hard-shelled eggs, a high metabolic rate, a four-chambered heart, and a lightweight but strong skeleton. Avian species includes, without limitation, chicken, quail, turkey, duck, Gallinacea sp, goose, pheasant and other fowl. The term "hen" includes all females of the avian species. A "transgenic avian" generally refers to an avian that has had a heterologous DNA sequence, or one or more additional DNA sequences normally endogenous to the avian (collectively referred to herein as "transgenes") chromosomally integrated into the germ cells of the avian. As a result of such transfer and integration, the transferred sequence may be transmitted through germ cells to the offspring of a transgenic avian. The transgenic avian (including its progeny) also have the transgene integrated into the gender chromosomes of somatic cells.

In some specific embodiments, the at least one transgenic animal of the invention may comprise at least two different reporter genes. In such case, each reporter gene may be integrated into at least one position or location in one of gender chromosome Z and/or W. In yet some further embodiments, each reporter gene may be integrated into at least two, at least three, at least four, at least five, at least six, at least seven, at least eight, at least nine, at least ten or more, positions or locations in one of gender chromosome Z and/or W.

In certain embodiments, the at least one transgenic avian animal provided by the invention, may be female. In more specific embodiments, the at least one reporter gene in such transgenic avian female may be integrated into at least one position of the female chromosome Z.

In yet some alternative embodiments, the at least one transgenic avian animal may be female, having at least one reporter gene integrated into at least one position of the female chromosome W.

In yet some further alternative embodiments, the transgenic animal provided by the invention may be a transgenic male subject that carry the transgene reporter gene integrated into at least one of its two gender Z chromosomes. In some further embodiments, the transgenic animal provided by the invention may be a transgenic male subject that carry the transgene reporter gene integrated into one of its two gender Z chromosomes. In further alternative embodiments, the transgenic animal provided by the invention may be a transgenic male subject that carry the transgene reporter gene integrated into two of its two gender Z chromosomes.

In some specific embodiments, the at least one reporter gene may be integrated into the gender chromosome of the transgenic animal of the invention using at least one PEN. As noted above in connection with the methods of the invention, any genetic engineering system or any PEN may be used, specifically, any RNA guided system, for example, one of the CRISPR, TALEN, ZFN and the like.

More specifically, such PEN may be in certain embodiments, a CRISPR system may be used. In more specific embodiments, a CRISPR Class 2 system may be used in creating the transgenic animal of the invention. In yet some further specific embodiments, a CRISPR type II system may be used.

In yet more specific embodiments, the at least one reporter gene may be integrated into the gender chromosome of the transgenic avian animal of the invention by HDR mediated by at least one CRISPR/Cas9 system.

In more specific embodiments, the at least one reporter gene may be integrated into a gender chromosome of the transgenic avian animal of the invention by contacting or co-transfecting at least one cell of this avian animal/subject, or at least one cell that is to be introduced into said avian animal with at least two nucleic acid sequences. More specifically, such cell may be contacted or co-transfected with (a) at least one component of a gene editing system, specifically, at least one Cas9 protein or at least one first nucleic acid sequence comprising at least one nucleic acid sequence encoding said at least one Cas9 protein; and at least one gRNA targeting at least one protospacer within at least one gender chromosome, Z or W, or at least one nucleic acid sequence encoding said at least one gRNA, thereby providing a CRISPR mediated integration; and (b) at least one second nucleic acid sequence comprising at least one reporter gene or any fragments thereof.

In more specific embodiments, the at least one reporter gene in the second nucleic acid sequence may be flanked at 5' and 3' thereof by homologous arms. These arms exhibit homology to the integration target site within the target gender chromosome, thereby facilitating HDR at the integration site.

In yet more specific embodiments, the at least one reporter gene in the second nucleic acid sequence may be operably linked to any one of a gender specific promoter, an embryonal specific promoter and an inducible promoter. Such promoter may in some embodiments, limit the expression of the reporter gene of the invention to the specific desired gender (in case of gender specific promoter), the specific embryonic stage (embryonic specific promoter) or specific conditions (inducible conditions).

In yet some further specific embodiments, the at least one reporter gene comprised within the transgenic avian animal of the invention may be integrated into at least one non-coding region of one of its gender chromosomes.

In yet some further alternative embodiments, the transgenic avian animal of the invention may comprise at least one reporter gene integrated into at least one site at gender Z chromosome. In some particular and non-limiting embodiments, such avian transgenic animal may be female that carry the transgenic reporter gene integrated into the Z chromosome. In more specific embodiments, the specific loci in the Z chromosome may be any one of regions 9156874-9161874, as denoted by SEQ ID NO:15, 27764943-27769943, as denoted by SEQ ID NO:16, 42172748-42177748, as denoted by SEQ ID NO:17, 63363656-63368656, as denoted by SEQ ID NO:18 and 78777477-78782477, as denoted by SEQ ID NO:19 of Chromosome Z of female chicken.

In some specific embodiments, at least one reporter gene may be integrated into at least one site at gender Z chromosome locus 42172748-42177748, specifically, as denoted by SEQ ID NO. 17. In yet some further specific embodiments, a gRNA sequence suitable for integration into specific loci within the Z chromosome, may include but is not limited to gRNA7 directed to a protospacer located within Z chromosome locus chrZ_42174515_-1. In some specific embodiments, the gRNA may be the gRNA designated gRNA7. In more specific embodiments, such gRNA7 may comprise the nucleic acid sequence GTAATACAGAGCTAAACCAG, as also denoted by SEQ ID NO:26. In yet some further embodiments, for integrating the reporter gene of the invention into the specific locus within the Z chromosome, the sequence encoding the reporter gene, specifically, the RFP, provided by the invention, may be flanked by a left arm at the 5' thereof, and a right arm at the 3' thereof. In yet some further specific embodiments, the left arm may comprise the nucleic acid sequence as denoted by SEQ ID NO. 31, and right arm comprising the nucleic acid sequence as denoted by SEQ ID NO. 32, may be used to integrate the reporter gene of the invention to the specific loci directed by gRNA7 of SEQ ID NO:26.

In yet some more specific embodiments, the at least one gRNA required to target the reporter gene to such specific location within the Z chromosome in the transgenic avian subject, may comprises the nucleic acid sequence as denoted by any one of gRNA3: ACAGACCTATGATATGT, as denoted by SEQ ID NO. 11; gRNA4: CGATTATCACTCACAAG, as denoted by SEQ ID NO. 12; gRNA5: CTGGTTAGCATGGGGAC, as denoted by SEQ ID NO. 13 ; gRNA6: GTAAAGAGTCAGATACA, as denoted by SEQ ID NO. 14.

In yet some further embodiments, for integrating the reporter gene of the invention into the specific locus within the Z chromosome, left arm comprising the nucleic acid sequence as denoted by SEQ ID NO. 41, and right arm comprising the nucleic acid sequence as denoted by SEQ ID NO. 42, may be used to integrate the reporter gene of the invention to the specific loci directed by gRNA3 of SEQ ID NO:11. In further embodiments, for integrating the reporter gene of the invention into the specific locus within the Z chromosome, left arm comprising the nucleic acid sequence as denoted by SEQ ID NO. 43, and right arm comprising the nucleic acid sequence as denoted by SEQ ID NO. 44, may be used to integrate the reporter gene in the transgenic avian subject of the invention to the specific loci directed by gRNA4 of SEQ ID NO:12. In still further embodiments, for integrating the reporter gene of the invention into the specific locus within the Z chromosome in the transgenic avian subject, left arm comprising the nucleic acid sequence as denoted by SEQ ID NO. 45, and right arm comprising the nucleic acid sequence as denoted by SEQ ID NO. 46, may be used to integrate the reporter gene of the invention to the specific loci directed by gRNA5 of SEQ ID NO:13. In some further embodiments, for integrating the reporter gene of the invention into the specific locus within the Z chromosome, left arm comprising the nucleic acid sequence as denoted by SEQ ID NO. 47, and right arm comprising the nucleic acid sequence as denoted by SEQ ID NO. 48, may be used to integrate the reporter gene of the invention to the specific loci directed by gRNA6 of SEQ ID NO:14.

Further non-limiting examples for gRNA sequences suitable for integration into specific loci within the Z chromosome of the transgenic avian subject of the invention, may include but are not limited to gRNA8 of Z chromosome locus chrZ_9157091_1, comprising the nucleic acid sequence ACAGACCTATGATATGTGAG, as also denoted by SEQ ID NO:27, gRNA9 of Z chromosome locus chrZ_27767602_-1, comprising the nucleic acid sequence GAGCTTGTGAGTGATAATCG, as also denoted by SEQ ID NO:28, gRNA10 of Z chromosome locus chrZ_78779927_1, comprising the nucleic acid sequence GTAAAGAGTCAGATACACAG, as also denoted by SEQ ID NO: 29, and gRNA11 of Z chromosome locus chrZ_63364946_-1, comprising the nucleic acid sequence CAGTGGGTACTGAAGCTGTG as also denoted by SEQ ID NO: 30.

In further embodiments, for integrating the reporter gene of the invention into the specific locus within the Z chromosome of the transgenic avian subject of the invention, left arm comprising the nucleic acid sequence as denoted by SEQ ID NO. 33, and right arm comprising the nucleic acid sequence as denoted by SEQ ID NO. 34, may be used to integrate the reporter gene of the invention to the specific loci directed by gRNA8 of SEQ ID NO:27. In still further embodiments, for integrating the reporter gene of the invention into the specific locus within the Z chromosome, left arm comprising the nucleic acid sequence as denoted by SEQ ID NO. 35, and right arm comprising the nucleic acid sequence as denoted by SEQ ID NO. 36, may be used to integrate the reporter gene of the invention to the specific loci directed by gRNA9 of SEQ ID NO:28. In further embodiments, for integrating the reporter gene of the invention into the specific locus within the Z chromosome, left arm comprising the nucleic acid sequence as denoted by SEQ ID NO. 37, and right arm comprising the nucleic acid sequence as denoted by SEQ ID NO. 38, may be used to integrate the reporter gene of the invention to the specific loci directed by gRNA10 of SEQ ID NO:29. In yet a further embodiment, for integrating the reporter gene of the invention into the specific locus within the Z chromosome, left arm comprising the nucleic acid sequence as denoted by SEQ ID NO. 39, and right arm comprising the nucleic acid sequence as denoted by SEQ ID NO. 40, may be used to integrate the reporter gene of the invention to the specific loci directed by gRNA11 of SEQ ID NO:30.

In yet some further alternative embodiments, the at least one reporter gene may be integrated into at least one site at gender W chromosome of the transgenic avian subject of the invention. In some particular embodiments, the integration site may be located at locus 1022859-1024215 at the W chromosome, specifically, galGal5_dna range of chromosome W:1022859-1024215. In yet some further specific embodiments, such loci comprises the nucleic acid sequence as denoted by SEQ ID NO. 3.

For specific integration of the reporter gene of the invention at any position within the loci described above, specific gRNAs may be required. Therefore, in some particular and non-limiting embodiments, appropriate gRNAs used for the preparation of the transgenic avian animal of the invention may comprise the nucleic acid sequence as denoted by any one of SEQ ID NO. 1 and 2. In some specific embodiments, these gRNAs are referred to herein as gRNA1 and gRNA2, respectively.

In some particular embodiments, the transgenic avian animal provided by the invention has been prepared using a gRNA1 that comprises the nucleic acid sequence as denoted by SEQ ID NO. 1. To enable integration of the reporter gene of the invention in such specific location, the reporter gene that should be integrated, must carry in certain embodiments, particular arms facilitating incorporation thereof in the target integration site directed by the gRNA used. Thus, in some specific embodiments, the at least one reporter gene may be comprised within the second nucleic acid sequence, where this reporter gene is flanked at 5' and 3' thereof by homologous arms comprising the amino acid sequence as denoted by SEQ ID NO. 4 and 5, respectively.

In yet some alternative embodiments, the transgenic avian animal provided by the invention may be prepared using a gRNA2 that comprises the nucleic acid sequence as denoted by SEQ ID NO. 2. In such case, to enable integration of the reporter gene of the invention at the specific site recognized by said gRNA2, the at least one reporter gene comprised within the second nucleic acid sequence may be according to specific embodiments, flanked at 5' and 3' thereof by homologous arms comprising the amino acid sequence as denoted by SEQ ID NO. 6 and 7, respectively.

It should be further appreciated that the invention further encompasses any of the transgenic avian subjects as described herein for use in any of the methods of the invention, specifically, methods for *in ovo* gender determination as discussed herein.

In yet some additional embodiments, the invention further encompasses the use of any egg laid by any of the transgenic avian subjects provided by the invention, specifically, as disclosed above, for use in any of the methods of the invention. Also described herein is a cell comprising at least one exogenous reporter gene integrated into at least one position or location in at least one of gender chromosome Z and W.

In some specific examples, the cell may be an avian cell.

In some particular examples, the avian cell may be a primordial germ cell (PGC).

The term **"germ cells"** refers to an embryonic cell that upon uniting with another germ cells develops into a gamete. **"Primordial germ cells (PGCs)",** as used herein relates to germline stem cells that serve as progenitors of the gametes and give rise to pluripotent embryonic stem cells. The cells in the gastrulating embryo that are signaled to become PGCs during embryogenesis, migrate into the genital ridges which becomes the gonads, and differentiate into mature gametes.

In some examples, the reporter gene integrated in at least one gender chromosome of the cell may be a fluorescent protein, specifically, RFP, YFP, BFP, and the like or any of the fluorescent proteins disclosed by Table 1. In yet some further examples, the reporter gene integrated in the cell may be any fluorescent protein, provided that said fluorescent protein is not green fluorescent protein (GFP). In other words, in some specific examples the reporter gene integrated into the gender chromosome of the transgenic cell may be any fluorescent protein with the proviso that said fluorescent protein is not GFP.

In yet more specific embodiments, the at least one reporter gene may be integrated into the gender chromosome of the transgenic avian subject or animal provided by the cell using at least one programmable engineered nuclease (PEN). The term **"programmable engineered nucleases (PEN)"** as used herein, refers to synthetic enzymes that cut specific DNA sequences, derived from natural occurring nucleases involved in DNA repair of double strand DNA lesions and enabling direct genome editing. In more specific embodiments, PEN may be a clustered regularly interspaced short palindromic repeat (CRISPR) class 2, specifically, type II system.

In yet some further embodiments, the cell may comprise at least one reporter gene integrated into a gender chromosome of the cell. In more specific embodiments, such specific integration of the reporter gene may be enabled by contacting or co-transfecting the cell with: (a) at least one gene editing system, comprising at least one Cas9 protein or at least one first nucleic acid sequence comprising at least one nucleic acid sequence encoding said at least one Cas9 protein; and at least one gRNA that targets at least one protospacer within at least one gender chromosome Z and/or W, or at least one nucleic acid sequence encoding said at least one gRNA; and (b) at least one second nucleic acid sequence comprising at least one said reporter gene.

In certain embodiments, the at least one reporter gene in the second nucleic acid sequence co-transfected to the cell, may be flanked at 5' and 3' thereof by homologous arms for HDR at the integration site.

In yet some specific embodiments, the cell may be prepared by integrating the at least one reporter gene into the Z chromosome of the cell. In certain embodiments, for preparing the cell, the at least one reporter gene may be integrated into at least one site at gender Z chromosome. In more specific embodiments, the specific loci in the Z chromosome may be any one of regions 9156874-9161874, as denoted by SEQ ID NO:15, 27764943-27769943, as denoted by SEQ ID NO:16, 42172748-42177748, as denoted by SEQ ID NO:17, 63363656-63368656, as denoted by SEQ ID NO:18 and 78777477-78782477, as denoted by SEQ ID NO: 19 of Chromosome Z of female chicken.

In yet some further alternative embodiments, the cell may be prepared by using gRNAs that facilitate integration into at least one site at gender Z chromosome locus 42172748-42177748, specifically, SEQ ID NO. 17. In yet some further specific embodiments, a gRNA sequence suitable for integration into specific loci within the Z chromosome, may include but is not limited to gRNA7 of Z chromosome locus chrZ_42174515_-1, comprising the nucleic acid sequence GTAATACAGAGCTAAACCAG, as also denoted by SEQ ID NO:26, In yet some further embodiments, for integrating the reporter gene into the specific locus within the Z chromosome, left arm comprising the nucleic acid sequence as denoted by SEQ ID NO. 31, and right arm comprising the nucleic acid sequence as denoted by SEQ ID NO. 32, may be used to integrate the reporter gene to the specific loci directed by gRNA7 of SEQ ID NO:26.

In more specific embodiments, the at least one gRNA required to target the reporter gene to such specific location within the Z chromosome of the cell may comprises the nucleic acid sequence as denoted by any one of gRNA3: ACAGACCTATGATATGT, as denoted by SEQ ID NO. 11; gRNA4: CGATTATCACTCACAAG, as denoted by SEQ ID NO. 12; gRNA5: CTGGTTAGCATGGGGAC, as denoted by SEQ ID NO. 13; gRNA6: GTAAAGAGTCAGATACA, as denoted by SEQ ID NO. 14.

In yet some further embodiments, for integrating the reporter gene into the specific locus within the Z chromosome of the cell, left arm comprising the nucleic acid sequence as denoted by SEQ ID NO. 41, and right arm comprising the nucleic acid sequence as denoted by SEQ ID NO. 42, may be used to integrate the reporter gene to the specific loci directed by gRNA3 of SEQ ID NO:11. In further embodiments, for integrating the reporter gene into the specific locus within the Z chromosome, left arm comprising the nucleic acid sequence as denoted by SEQ ID NO. 43, and right arm comprising the nucleic acid sequence as denoted by SEQ ID NO. 44, may be used to integrate the reporter gene to the specific loci directed by gRNA4 of SEQ ID NO:12. In still further embodiments, for integrating the reporter gene into the specific locus within the Z chromosome of the cell, left arm comprising the nucleic acid sequence as denoted by SEQ ID NO. 45, and right arm comprising the nucleic acid sequence as denoted by SEQ ID NO. 46, may be used to integrate the reporter gene invention to the specific loci directed by gRNA5 of SEQ ID NO:13. In some further embodiments, for integrating the reporter gene into the specific locus within the Z chromosome, left arm comprising the nucleic acid sequence as denoted by SEQ ID NO. 47, and right arm comprising the nucleic acid sequence as denoted by SEQ ID NO. 48, may be used to integrate the reporter gene to the specific loci directed by gRNA6 of SEQ ID NO:14.

Further non-limiting examples for gRNA sequences suitable for integration into specific loci within the Z chromosome, may include but are not limited to gRNA8 of Z chromosome locus chrZ_9157091_1, comprising the nucleic acid sequence ACAGACCTATGATATGTGAG, as also denoted by SEQ ID NO:27, gRNA9 of Z chromosome locus chrZ_27767602_-1, comprising the nucleic acid sequence GAGCTTGTGAGTGATAATCG, as also denoted by SEQ ID NO:28, gRNA10 of Z chromosome locus chrZ_78779927_1, comprising the nucleic acid sequence GTAAAGAGTCAGATACACAG, as also denoted by SEQ ID NO: 29, and gRNA11 of Z chromosome locus chrZ_63364946_-1, comprising the nucleic acid sequence CAGTGGGTACTGAAGCTGTG as also denoted by SEQ ID NO: 30.

In further embodiments, for integrating the reporter gene into the specific locus within the Z chromosome of the cell, left arm comprising the nucleic acid sequence as denoted by SEQ ID NO. 33, and right arm comprising the nucleic acid sequence as denoted by SEQ ID NO. 34, may be used to integrate the reporter gene to the specific loci directed by gRNA8 of SEQ ID NO:27. In still further embodiments, for integrating the reporter gene into the specific locus within the Z chromosome of the cell, left arm comprising the nucleic acid sequence as denoted by SEQ ID NO. 35, and right arm comprising the nucleic acid sequence as denoted by SEQ ID NO. 36, may be used to integrate the reporter gene to the specific loci directed by gRNA9 of SEQ ID NO:28. In further embodiments, for integrating the reporter gene into the specific locus within the Z chromosome, left arm comprising the nucleic acid sequence as denoted by SEQ ID NO. 37, and right arm comprising the nucleic acid sequence as denoted by SEQ ID NO. 38, may be used to integrate the reporter gene to the specific loci directed by gRNA10 of SEQ ID NO:29. In yet a further embodiment, for integrating the reporter gene into the specific locus within the Z chromosome, left arm comprising the nucleic acid sequence as denoted by SEQ ID NO. 39, and right arm comprising the nucleic acid sequence as denoted by SEQ ID NO. 40, may be used to integrate the reporter gene to the specific loci directed by gRNA11 of SEQ ID NO:30.

In some particular embodiments, to target the integration of the reporter gene to chromosome W in the cell provided by the invention, specific gRNAs should be used. In further particular embodiments, the gRNA may comprise the nucleic acid sequence as denoted by SEQ ID NO. 1 referred to herein as gRNA1. In such case, the at least one reporter gene comprised within the second nucleic acid sequence, may be flanked at 5' and 3' thereof by homologous arms comprising the amino acid sequence as denoted by SEQ ID NO. 4 and 5, respectively.

In yet some further alternative embodiments, the cell provided by the invention may be prepared by using gRNA referred to herein as gRNA2. In certain embodiments, gRNA2 may comprise the nucleic acid sequence as denoted by SEQ ID NO. 2. In such specific embodiments, the at least one reporter gene comprised within the second nucleic acid sequence may be flanked at 5' and 3' thereof by homologous arms comprising the amino acid sequence as denoted by SEQ ID NO. 6 and 7, respectively.

Still further, the invention provides a method for detecting fertilization of an unhatched egg. In some specific embodiments, such method may comprise the step of (a), providing or obtaining at least one transgenic avian subject or animal comprising at least one exogenous reporter gene integrated into at least one position or location in both gender chromosome Z and W, in case the provided transgenic animal is a female subject and into both gender chromosomes Z in case the provided transgenic animal is a male. In a second step (b) obtaining at least one fertilized egg from the transgenic avian subject, specifically animal or of any cells thereof. The next step (c) involves determining in the egg if at least one detectable signal is detected. In more specific embodiments, detection of at least one detectable signal indicates the expression of said at least one reporter gene, thereby the presence of the labeled maternal W chromosome or Z chromosome (in case of a female or the labeled paternal Z chromosome in the avian embryo.

In yet some further embodiments, the transgenic animal provided by the method of the invention may be a male subject having the reporter gene integrated into both Z chromosomes thereof. In such case, a detectable signal determined in an egg fertilized by such transgenic male or any sperms thereof, indicates that the embryo carries a paternal Z chromosome comprising the transgenic reporter gene, and the egg is therefore fertilized. In still further embodiments, the transgenic animal provided by the method of the invention may be a female subject having the reporter gene integrated into both, Z and W chromosomes thereof. In such case, a detectable signal determined in an egg carried by such transgenic female, indicates that the embryo carries a maternal Z or W chromosome comprising the transgenic reporter gene, and the egg is therefore fertilized. It should be appreciated that in further embodiments, for detecting a fertilized egg, the reporter gene, specifically, the RFP gene may be inserted into the two copies of any chromosomes, to create a homozygous transgenic avian subject, specifically, a male subject. A fertilized egg will be detected as carrying the RFP reporter gene.

In yet a further aspect, the invention provides a kit comprising:

(a) at least one component of at least one gene editing system, specifically, the CRSPR/Cas9 system that may comprise at least one Cas9 protein or at least one first nucleic acid sequence comprising at least one nucleic acid sequence encoding said at least one Cas9 protein; and at least one gRNA that targets at least one protospacer within at least one gender chromosome Z and/or W, or at least one nucleic acid sequence encoding said at least one gRNA; and (b) at least one second nucleic acid sequence comprising at least one said reporter gene, wherein said reporter gene is a Red Fluorescent Protein (RFP); wherein the expression of said at least one reporter is suitable for identification of a gender of an avian embryo in an unhatched egg comprising the embryo within a structurally integral shell,

wherein said at least one reporter gene is integrated into at least one site at: (a) gender Z chromosome locus 42172748-42177748, wherein said at least one gRNA comprises the nucleic acid sequence as denoted by SEQ ID NO. 26 and said at least one reporter gene comprised within said second nucleic acid is flanked at 5' and 3' thereof by homologous arms comprising the nucleic acid sequence as denoted by SEQ ID NO. 31 and 32, respectively; or (b) gender W chromosome locus 1022859-1024215, wherein at least one of: (i) said gRNA comprises the nucleic acid sequence as denoted by SEQ ID NO. 1, and said at least one reporter gene comprised within said second nucleic acid sequence is flanked at 5' and 3' thereof by homologous arms comprising the amino acid sequence as denoted by SEQ ID NO. 4 and 5, respectively; and (ii) said gRNA comprises the nucleic acid sequence as denoted by SEQ ID NO. 2, and said at least one reporter gene comprised within said second nucleic acid sequence is flanked at 5' and 3' thereof by homologous arms comprising the amino acid sequence as denoted by SEQ ID NO. 6 and 7, respectively.

In some further embodiments, the gRNA used by the method, as well by kit of the invention (described herein after) may comprise at least one CRISPR RNA (crRNA) and at least one trans-activating crRNA (tracrRNA).

In some alternative embodiments the kit of the invention may comprise nucleic acid sequence encoding the at least one gRNA. Such nucleic acid sequence may comprise a CRISPR array comprising at least one spacer sequence that targets and is therefore homolog or identical to at least one protospacer in a target genomic DNA sequence. It should be noted that the nucleic acid sequence may further comprise a sequence encoding at least one tracrRNA.

In some embodiments the CRISPR array according to the present disclosure comprises at least one spacer and at least one repeat. In yet another embodiment, the invention further encompasses the option of providing a pre-crRNA that can be processed to several final gRNA products that may target identical or different targets.

In yet some more specific embodiments, the crRNA comprised within the gRNA of the invention may be a single-stranded ribonucleic acid (ssRNA) sequence complementary to a target genomic DNA sequence. In some specific embodiments, the target genomic DNA sequence may be located immediately upstream of a protospacer adjacent motif (PAM) sequence and further.

As indicated herein, the gRNA of the kits and methods of the invention may be complementary, at least in part, to the target genomic DNA, in this case, the target locus within the gender chromosomes Z or W, referred to herein as "protospacer". In certain embodiments, "**Complementarity**" refers to a relationship between two structures each following the lock-and-key principle. In nature complementarity is the base principle of DNA replication and transcription as it is a property shared between two DNA or RNA sequences, such that when they are aligned antiparallel to each other, the nucleotide bases at each position in the sequences will be complementary (e.g., A and T or U, C and G). As indicated above, the genomic DNA sequence targeted by the gRNA of the kit of the invention is located immediately upstream to a PAM sequence. In some embodiments, such PAM sequence may be of the nucleic acid sequence NGG.

In certain embodiments, the PAM sequence referred to by the invention may comprise N, that is any nucleotide, specifically, any one of Adenine (A), Guanine (G), Cytosine (C) or Thymine (T). In yet some further embodiments the PAM sequence according to the invention is composed of A, G, C, or T and two Guanines.

According to one embodiment, the polynucleotide encoding the gRNA of the invention may comprise at least one spacer and optionally, at least one repeat. In yet some further embodiments, the DNA encoding the gRNA of the invention may comprise at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or more, specifically, 110, 120, 130, 140, 150, 160, 170, 180, 190, 200 or more spacers. In some embodiments, each spacer is located between two repeats. It should be further understood that the spacers of the nucleic acid sequence encoding the gRNA of the invention may be either identical or different spacers. In more embodiments, these spacers may target either an identical or different target genomic DNA. In yet some other embodiments, such spacer may target at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or more target genomic DNA sequence. These target sequences may be derived from a single locus or alternatively, from several target loci.

As used herein, the term "**spacer**" refers to a non-repetitive spacer sequence that is designed to target a specific sequence and is located between multiple short direct repeats (i.e., CRISPR repeats) of CRISPR arrays. In some specific embodiments, spacers may comprise between about 15 to about 30 nucleotides, specifically, about 15, 16, 17, 18, 19, 20, 21, 22, 23, 24, 25, 26, 27, 28, 29, 30 or more nucleotides. More specifically, about 20-25 nucleotides.

The guide or targeting RNA encoded by the CRISPR system of the invention may comprise a CRISPR RNA (crRNA) and a trans activating RNA (tracrRNA). The sequence of the targeting RNA encoded by the CRISPR spacers is not particularly limited, other than by the requirement for it to be directed to (i.e., having a segment that is the same as or complementarity to) a target sequence in avian genomic DNA, specifically, in a target locus within the gender chromosomes Z or W, that is also referred to herein as a "**proto-spacer**". Such proto-spacers comprise nucleic acid sequence having sufficient complementarity to a targeting RNA encoded by the CRISPR spacers comprised within the nucleic acid sequence encoding the gRNA of the methods and kits of the invention.

In some embodiments, a crRNA comprises or consists of 20, 21, 22, 23, 24, 25, 26, 27, 28, 29, 30, 31, 32, 33, 34, 35, 36, 37, 38, 39 or 40 nt of the spacer (targeting) sequence followed by 19-36 nt of repeat sequence. In specific and non-limiting embodiments, the targeting spacer may comprise or consist of a segment that targets any one of the genomic DNA sequence for which representative spacer sequences are indicated herein.

It should be noted that in some specific embodiments, the spacers of the CRISPR system of the invention may encode a targeting guide RNA (gRNA). A "**gRNA**" or "**targeting RNA**" is an RNA that, when transcribed from the portion of the CRISPR system encoding it, comprises at least one segment of RNA sequence that is identical to (with the exception of replacing T for U in the case of RNA) or complementary to (and thus "targets") a DNA sequence in the target genomic DNA. The CRISPR systems of the present disclosure may optionally encode more than one targeting RNA, and the targeting RNAs be directed to one or more target sequences in the genomic DNA.

In some embodiments, the Cas9 protein provided by the kit of the invention may be encoded by the nucleic acid sequence as denoted by SEQ ID NO. 24. In further specific and non-limiting embodiments, the Cas9 protein may comprise the amino acid sequence as denoted by SEQ ID NO. 25, or any derivatives, mutants or variants thereof.

It should be noted that "Amino acid sequence" or "peptide sequence" is the order in which amino acid residues connected by peptide bonds, lie in the chain in peptides and proteins. The sequence is generally reported from the N-terminal end containing free amino group to the C-terminal end containing amide. Amino acid sequence is often called peptide, protein sequence if it represents the primary structure of a protein, however one must discern between the terms "Amino acid sequence" or "peptide sequence" and "protein", since a protein is defined as an amino acid sequence folded into a specific three-dimensional configuration and that had typically undergone post-translational modifications, such as phosphorylation, acetylation, glycosylation, manosylation, amidation, carboxylation, sulfhydryl bond formation, cleavage and the like.

By "fragments or peptides" it is meant a fraction of said Cas9 or RFP (described herein after) molecules. A "fragment" of a molecule, such as any of the amino acid sequences of the present invention, is meant to refer to any amino acid subset of the Cas9 or RFP molecules. This may also include "variants" or "derivatives" thereof. A "peptide" is meant to refer to a particular amino acid subset having functional activity. By "functional" is meant having the same biological function, for example, having the ability to perform gene editing as the Cas 9 or as producing a detectable signal as the RFP.

It should be appreciated that the invention encompasses any variant or derivative of the RFP or Cas9 molecules of the invention and any polypeptides that are substantially identical or homologue. The term "*derivative*" is used to define amino acid sequences (polypeptide), with any insertions, deletions, substitutions and modifications to the amino acid sequences (polypeptide) that do not alter the activity of the original polypeptides. In this connection, a derivative or fragment of the Cas9 or RFP molecules of the invention may be any derivative or fragment of the Cas9 or RFP molecules, specifically as denoted by SEQ ID NO. 25, 21, 23, that do not reduce or alter the activity of the Cas9 or RFP molecules. By the term "derivative" it is also referred to homologues, variants and analogues thereof. Proteins orthologs or homologues having a sequence homology or identity to the proteins of interest in accordance with the invention, specifically that may share at least 50%, at least 60% and specifically 65%, 70%, 75%, 80%, 85%, 90%, 91%, 92%, 93%, 94%, 95%, 96%, 97%, 98%, 99%, 100%, or higher, specifically as compared to the entire sequence of the proteins of interest in accordance with the invention, for example, any of the proteins that comprise the amino acid sequence as denoted by SEQ ID NO. 25, 21, 23. Specifically, homologs that comprise or consists of an amino acid sequence that is identical in at least 50%, at least 60% and specifically 65%, 70%, 75%, 80%, 85%, 90%, 91%, 92%, 93%, 94%, 95%, 96%, 97%, 98%, 99%, or higher to SEQ ID NO. 21, 23, 25 specifically, the entire sequence as denoted by SEQ ID NO. 21, 23, 25. In some embodiments, derivatives refer to polypeptides, which differ from the polypeptides specifically defined in the present invention by insertions, deletions or substitutions of amino acid residues. It should be appreciated that by the terms "**insertion/s**", "**deletion/s**" or "**substitution/s**", as used herein it is meant any addition, deletion or replacement, respectively, of amino acid residues to the polypeptides disclosed by the invention, of between 1 to 50 amino acid residues, between 20 to 1 amino acid residues, and specifically, between 1 to 10 amino acid residues. More particularly, insertion/s, deletion/s or substitution/s may be of any one of 1, 2, 3, 4, 5, 6, 7, 8, 9, or 10 amino acids. It should be noted that the insertion/s, deletion/s or substitution/s encompassed by the invention may occur in any position of the modified peptide, as well as in any of the N' or C' termini thereof.

With respect to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologues, and alleles of the invention. For example, substitutions may be made wherein an aliphatic amino acid (G, A, I, L, or V) is substituted with another member of the group, or substitution such as the substitution of one polar residue for another, such as arginine for lysine, glutamic for aspartic acid, or glutamine for asparagine. Each of the following eight groups contains other exemplary amino acids that are conservative substitutions for one another:

    1) Alanine (A), Glycine (G);
    2) Aspartic acid (D), Glutamic acid (E);
    3) Asparagine (N), Glutamine (Q);
    4) Arginine (R), Lysine (K);
    5) Isoleucine (I), Leucine (L), Methionine (M), Valine (V);
    6) Phenylalanine (F), Tyrosine (Y), Tryptophan (W);
    7) Serine (S), Threonine (T); and
    8) Cysteine (C), Methionine (M).

Thus, in some embodiments, the invention encompasses Cas9 or RFP molecules or any derivatives thereof, specifically a derivative that comprise at least 1, 2, 3, 4, 5, 6, 7, 8, 9, 10 or more conservative substitutions to the amino acid sequences as denoted by any one of SEQ ID NO. 21, 23, 25. More specifically, amino acid "substitutions" are the result of replacing one amino acid with another amino acid having similar structural and/or chemical properties, i.e., conservative amino acid replacements. Amino acid substitutions may be made on the basis of similarity in polarity, charge, solubility, hydrophobicity, hydrophilicity, and/or the amphipathic nature of the residues involved. For example, nonpolar "***hydrophobic***" amino acids are selected from the group consisting of Valine (V), Isoleucine (I), Leucine (L), Methionine (M), Phenylalanine (F), Tryptophan (W), Cysteine (C), Alanine (A), Tyrosine (Y), Histidine (H), Threonine (T), Serine (S), Proline (P), Glycine (G), Arginine (R) and Lysine (K); "***polar***" amino acids are selected from the group consisting of Arginine (R), Lysine (K), Aspartic acid (D), Glutamic acid (E), Asparagine (N), Glutamine (Q); "***positively charged***" amino acids are selected form the group consisting of Arginine (R), Lysine (K) and Histidine (H) and wherein "***acidic***" amino acids are selected from the group consisting of Aspartic acid (D), Asparagine (N), Glutamic acid (E) and Glutamine (Q).

Variants of the polypeptides of the invention may have at least 80% sequence similarity or identity, often at least 85% sequence similarity or identity, 90% sequence similarity or identity, or at least 95%, 96%, 97%, 98%, or 99% sequence similarity or identity at the amino acid level, with the protein of interest, such as the various polypeptides of the invention.

In some embodiments, the at least one reporter gene in the second nucleic acid sequence comprised within the kit of the invention, may be flanked at 5' and 3' thereof by homologous arms for HDR at the integration site.

In yet some further specific embodiments, the at least one reporter gene in the second nucleic acid sequence of the kit of the invention may be operably linked to any one of a gender specific promoter, an embryonic specific promoter and an inducible promoter.

In yet some further alternative embodiments, the at least one reporter gene may be integrated into at least one site at gender Z chromosome. In more specific embodiments, the specific loci in the Z chromosome may be any one of regions 9156874-9161874, as denoted by SEQ ID NO:15, 27764943-27769943, as denoted by SEQ ID NO:16, 42172748-42177748, as denoted by SEQ ID NO:17, 63363656-63368656, as denoted by SEQ ID NO:18 and 78777477-78782477, as denoted by SEQ ID NO:19 of Chromosome Z of female chicken.

In some specific embodiments, at least one reporter gene may be integrated into at least one site at gender Z chromosome locus 42172748-42177748. Non-limiting examples of the first nucleic acid sequence of the kit of the invention may comprise a gRNA, being the at least one of gRNA7 of Z chromosome locus chrZ_42174515_-1, comprising the nucleic acid sequence GTAATACAGAGCTAAACCAG, as also denoted by SEQ ID NO:26. More specifically, for integrating the reporter gene of the invention into the specific locus within the Z chromosome, left arm comprising the nucleic acid sequence as denoted by SEQ ID NO. 31, and right arm comprising the nucleic acid sequence as denoted by SEQ ID NO. 32, may be used to integrate the reporter gene of the invention to the specific loci directed by gRNA7 of SEQ ID NO:26.

Thus, in more specific embodiments, the first nucleic acid sequence of the kit of the invention may comprise a gRNA, being the at least one of gRNA3: ACAGACCTATGATATGT, as denoted by SEQ ID NO. 11; gRNA4: CGATTATCACTCACAAG, as denoted by SEQ ID NO. 12; gRNA5: CTGGTTAGCATGGGGAC, as denoted by SEQ ID NO. 13; gRNA6: GTAAAGAGTCAGATACA, as denoted by SEQ ID NO. 14.

In further examples, the at least one reporter gene comprised within the second nucleic acid sequence of the kit described herein, may be flanked at 5' and 3' thereof by homologous arms comprising the amino acid sequence as denoted by any one of SEQ ID NO. 41 to 48. More specifically, for integrating the reporter gene into the specific locus within the Z chromosome, left arm comprising the nucleic acid sequence as denoted by SEQ ID NO. 41, and right arm comprising the nucleic acid sequence as denoted by SEQ ID NO. 42, may be used to integrate the reporter gene to the specific loci directed by gRNA3 of SEQ ID NO:11. In further examples, for integrating the reporter gene into the specific locus within the Z chromosome, left arm comprising the nucleic acid sequence as denoted by SEQ ID NO. 43, and right arm comprising the nucleic acid sequence as denoted by SEQ ID NO. 44, may be used to integrate the reporter gene to the specific loci directed by gRNA4 of SEQ ID NO:12. In still further examples, for integrating the reporter gene into the specific locus within the Z chromosome, left arm comprising the nucleic acid sequence as denoted by SEQ ID NO. 45, and right arm comprising the nucleic acid sequence as denoted by SEQ ID NO. 46, may be used to integrate the reporter gene to the specific loci directed by gRNA5 of SEQ ID NO:13. In some further examples, for integrating the reporter gene into the specific locus within the Z chromosome, left arm comprising the nucleic acid sequence as denoted by SEQ ID NO. 47, and right arm comprising the nucleic acid sequence as denoted by SEQ ID NO. 48, may be used to integrate the reporter gene to the specific loci directed by gRNA6 of SEQ ID NO:14.

Further non-limiting examples of the first nucleic acid sequence of the kit described herein may comprise a gRNA, being the at least one of gRNA8 of Z chromosome locus chrZ_9157091_1, comprising the nucleic acid sequence ACAGACCTATGATATGTGAG, as also denoted by SEQ ID NO:27, gRNA9 of Z chromosome locus chrZ_27767602_-1, comprising the nucleic acid sequence GAGCTTGTGAGTGATAATCG, as also denoted by SEQ ID NO:28, gRNA10 of Z chromosome locus chrZ_78779927_1, comprising the nucleic acid sequence GTAAAGAGTCAGATACACAG, as also denoted by SEQ ID NO: 29, and gRNA11 of Z chromosome locus chrZ_63364946_-1, comprising the nucleic acid sequence CAGTGGGTACTGAAGCTGTG as also denoted by SEQ ID NO: 30.

In further examples, the at least one reporter gene comprised within the second nucleic acid sequence of the kit described herein, may be flanked at 5' and 3' thereof by homologous arms comprising the amino acid sequence as denoted by any one of SEQ ID NO. 33 to 40. More specifically, for integrating the reporter gene into the specific locus within the Z chromosome, left arm comprising the nucleic acid sequence as denoted by SEQ ID NO. 33, and right arm comprising the nucleic acid sequence as denoted by SEQ ID NO. 34, may be used to integrate the reporter gene to the specific loci directed by gRNA8 of SEQ ID NO:27. In still further examples, for integrating the reporter gene into the specific locus within the Z chromosome, left arm comprising the nucleic acid sequence as denoted by SEQ ID NO. 35, and right arm comprising the nucleic acid sequence as denoted by SEQ ID NO. 36, may be used to integrate the reporter gene to the specific loci directed by gRNA9 of SEQ ID NO:28. In further examples, for integrating the reporter gene into the specific locus within the Z chromosome, left arm comprising the nucleic acid sequence as denoted by SEQ ID NO. 37, and right arm comprising the nucleic acid sequence as denoted by SEQ ID NO. 38, may be used to integrate the reporter gene to the specific loci directed by gRNA10 of SEQ ID NO:29. In yet a further example, for integrating the reporter gene into the specific locus within the Z chromosome, left arm comprising the nucleic acid sequence as denoted by SEQ ID NO. 39, and right arm comprising the nucleic acid sequence as denoted by SEQ ID NO. 40, may be used to integrate the reporter gene to the specific loci directed by gRNA11 of SEQ ID NO:30.

In certain embodiments, the at least one reporter gene provided by the kits of the invention may be integrated into at least one non-coding region of the gender chromosome, specifically, to chromosome W. In such case, the first nucleic acid sequence of the kit of the invention may encode at least one gRNA comprising the nucleic acid sequence as denoted by any one of SEQ ID NO. 1 and 2, also referred to herein as gRNA1 and gRNA2, respectively.

In some specific embodiments, the first nucleic acid sequence of the kit of the invention may comprise a gRNA, being gRNA1. In some embodiments, such gRNA1 may comprise the nucleic acid sequence as denoted by SEQ ID NO. 1. In such case, the reporter gene comprised within said second nucleic acid sequence of the kit of the invention, may be flanked at 5' and 3' thereof by homologous arms comprising the amino acid sequence as denoted by SEQ ID NO. 4 and 5, respectively.

In yet some further alternative embodiments, the kit of the invention may comprise in the first nucleic acid sequence thereof, a sequence encoding gRNA2. In some specific embodiments, such sequence encodes the nucleic acid sequence as denoted by SEQ ID NO. 2. In yet some further embodiments, the least one reporter gene comprised within the second nucleic acid sequence of the kit of the invention , may be flanked at 5' and 3' thereof by homologous arms comprising the amino acid sequence as denoted by SEQ ID NO. 6 and 7, respectively.

The reporter gene comprised within the second nucleic acid sequence of the kit of the invention is a Red Fluorescent Protein (RFP). In more specific embodiments, such RFP may comprise the amino acid sequence as denoted by SEQ ID NO. 21, or any homologs, variants, mutants or derivatives thereof. In yet some alternative specific embodiments, the RFP used by the invention may be the RFP that may comprise the amino acid sequence as denoted by SEQ ID NO. 23, or any homologs, mutants or derivatives thereof.

In yet some further embodiments, the kit of the invention may be suitable for use in the preparation of a transgenic avian animal comprising at least one exogenous reporter gene integrated into at least one position or location in at least one of gender chromosome Z and W.

In some embodiments, the method of the invention may use any of the kits of the invention as described herein.

Still further, it must be appreciated that the kits of the invention may further comprise any reagent, buffer, media or material required for the preparation of the transgenic avian animals of the invention. The kit of the invention may further comprise instructions as well as containers for the different components thereof.

In some embodiments, the kits of the invention may be used in the preparation of any of the transgenic avian subjects of the invention or of any cell thereof in accordance with the invention. In yet some further embodiments, the transgenic avian subjects and cells prepared using the kits of the invention, may be used by any of the methods of the invention, using any of the systems and devices as disclosed herein. Still further, in some embodiments, the kit of the invention may further comprise any apparatus, system, and/or device adapted for determining the presence of the reporter gene within the *in ovo* embryo examined by the methods of the invention. In some specific embodiments, such apparatus or device may be any of the devices disclosed by the invention, specifically, as shown in Figure 1.

In yet another aspect thereof, the invention provides a system, apparatus or a device that is specifically adapted for detecting a reporter gene within an *in ovo* embryo. In some embodiments, the device of the invention may comprise a laser source, a stand for the egg, a lens, a filter, a stand for a detector and a detector. The device of the invention may optionally, in some embodiments, comprise a solid support that holds all elements of the system of the invention, for example, as shown in Figure 1. As used herein, the term "**detector**" refers to any type of device that detects and/or measures light.

In some embodiments, it should be noted that the detectable signal, specifically, the fluorescent signal may be detected by the device of the invention using suitable fluorescent means. In some embodiments, the detectable signal formed by the RFP reporter gene may be detected by light sensitive apparatus such as modified optical microscopes or Charge Coupled Device (CCD), a highly sensitive photon detector.

In yet some further embodiments, the device, apparatus or system of the invention may comprise at least one light source, at least one detector, at least one filter and at least one holding arm that places the egg in an appropriate position facilitating the exposure of cells expressing the reporter gene of the invention to said light source. In some embodiments, such egg may be a fertilized unhatched avian egg. In yet some further embodiments, such egg may be laid by any of the transgenic avian subjects of the invention. In some particular and non-limiting embodiments, such device of the invention may be as illustrated in Figure 1. In yet some further embodiments, the distance between the egg holder and lens and/or lens and detector may range between about 1 to 100 cm, specifically, 1, 5, 10, 15, 20, 25, 30, 35, 40, 45, 50, 55, 60, 65, 70, 75, 80, 85, 90, 95 and 100 cm, specifically between about 5 to 25 cm, more specifically, about 20 cm.

It should be therefore appreciated that in some embodiments, the system or device of the invention may be adapted for performing any of the methods disclosed by the invention. In some specific embodiments, the device of the invention may comprise an appropriate light source that enables detection of the reporter gene in the examined egg. In yet some further embodiments, the light source may comprise wavelength of between about 400 to about 650. In yet some further embodiments, light of any wavelength may be used with the proviso that said light source is not Ultra-Violet (UV) light (wavelength 10-400 nm). In some specific embodiment, the appropriate light source is suitable for excitation of the fluorescent protein. In some particular embodiments, the excitation wavelength may be between about 500 nm and about 650 nm. In some further embodiments, the excitation wavelength is about 510, 515, 520, 525, 530, 535, 540, 545, 550, 555, 560, 565, 570, 575, 580, 585, 590, 595, 600, 605, 610, 615, 620, 625, 630, 635, 640 nm.

In some more specific embodiment, the excitation wavelength may range between about 515 to about 555. In yet some further embodiments, the wavelength may be 532 nm. In some specific and non-limiting embodiment, the light source may be provided by a laser. In some other embodiments, the laser of the device of the invention may be a blue laser, or a red laser. In more specific embodiments, the light source is a green laser.

In certain embodiments, the laser may be employed with a filter. In some embodiments, the filter may be a green filter that transmits above 500 nm, or a dark green filter that transmits between 540 nm and 580 nm, or a red filter that transmits between 590 nm and 650 nm, or a red filter that transmits above 650 nm or above 660 nm.

In some particular embodiments, the light source may be a green laser with a wavelength of 532 nm. In some further embodiments, such laser may be provided with a red filter that transmits above 650 nm.

It should be thus further appreciated that the invention further encompasses the device as described herein for use in any of the methods of the invention, specifically, methods for *in ovo* gender determination as discussed herein, using any of the transgenic avian subjects of the invention.

It should be appreciated that in certain embodiments, the oligonucleotide/s or polynucleotide/s used by the kit/s and method/s of the invention are isolated and/or purified molecules. As used herein, "**isolated**" or "**purified**" when used in reference to a nucleic acid means that a naturally occurring sequence has been removed from its normal cellular (e.g., chromosomal) environment or is synthesized in a non-natural environment (e.g., artificially synthesized). Thus, an "isolated" or "purified" sequence may be in a cell-free solution or placed in a different cellular environment. The term "purified" does not imply that the sequence is the only nucleotide present, but that it is essentially free (about 90-95% pure) of non-nucleotide material naturally associated with it, and thus is distinguished from isolated chromosomes.

All scientific and technical terms used herein have meanings commonly used in the art unless otherwise specified. The definitions provided herein are to facilitate understanding of certain terms used frequently herein and are not meant to limit the scope of the present disclosure.

Before specific aspects and embodiments of the invention are described in detail, it is to be understood that this invention is not limited to particular methods, and experimental conditions described, as such methods and conditions may vary. It is also to be understood that the terminology used herein is for the purpose of describing particular embodiments only, and is not intended to be limiting, since the scope of the present invention will be limited only by the appended claims.

As used in this specification and the appended claims, the singular forms "a", "an", and "the" include plural references unless the context clearly dictates otherwise. Thus for example, references to "a method" includes one or more methods, and/or steps of the type described herein and/or which will become apparent to those persons skilled in the art upon reading this disclosure and so forth.

Unless defined otherwise, all technical and scientific terms used herein have the same meaning as commonly understood by one of ordinary skill in the art to which this invention belongs. Although any methods and materials similar or equivalent to those described herein can be used in the practice or testing of the present invention, the preferred methods and materials are now described.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps. More specifically, the terms "comprises", "comprising", "includes", "including", "having" and their conjugates mean "including but not limited to". This term encompasses the terms "consisting of" and "consisting essentially of". The phrase "consisting essentially of" means that the composition or method may include additional ingredients and/or steps, but only if the additional ingredients and/or steps do not materially alter the basic and novel characteristics of the claimed composition or method.

The term "about" as used herein indicates values that may deviate up to 1%, more specifically 5%, more specifically 10%, more specifically 15%, and in some cases up to 20% higher or lower than the value referred to, the deviation range including integer values, and, if applicable, non-integer values as well, constituting a continuous range. As used herein the term "about" refers to $\pm$ 10 %.

It should be noted that various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible sub ranges as well as individual numerical values within that range. For example, description of a range such as from 1 to 6 should be considered to have specifically disclosed sub ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals there between.

The following examples are put forth so as to provide those of ordinary skill in the art with a complete disclosure and description of how to make and use the methods and compositions of the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

The examples are representative of techniques employed by the inventors in carrying out aspects of the present invention.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable sub combination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

Disclosed and described, it is to be understood that this invention is not limited to the particular examples, methods steps, and compositions disclosed herein as such methods steps and compositions may vary somewhat. It is also to be understood that the terminology used herein is used for the purpose of describing particular embodiments only and not intended to be limiting since the scope of the present invention will be limited only by the appended claims and equivalents thereof.

It must be noted that, as used in this specification and the appended claims, the singular forms "a", "an" and "the" include plural referents unless the content clearly dictates otherwise.

## EXAMPLES

Without further elaboration, it is believed that one skilled in the art can, using the preceding description, utilize the present invention to its fullest extent. The following preferred specific embodiments are, therefore, to be construed as merely illustrative, and not limitative of the claimed invention in any way.

Standard molecular biology protocols known in the art not specifically described herein are generally followed essentially as in Sambrook et al., Molecular cloning: A laboratory manual, Cold Springs Harbor Laboratory, New-York (1989,1992), and in Ausubel et al., Current Protocols in Molecular Biology, John Wiley and Sons, Baltimore, Maryland (1988).

### *Reagents*

Animals:

Commercial White Leghorn chickens are obtained from Hendrix ISA, and Minnesota. Marker Line chickens are from the Pacific Agri-Food Research Centre, Agassiz, British Columbia, Canada.

Animal experiments were done in strict accordance to IACUC approved protocols and under supervision of the Crystal Bioscience IACUC committee ensuring that no animal suffers from illness nor dies during the course of the experiments.

Vectors:

PrecisionX™ Cas9 SmartNuclease Vector System is ordered from System Bioscience Inc., catalogue number CAS8/9xx series.

pDsRed1-N1 from Clontech#6921-1.

GFP plasmid- FUGW-H1-GFP-neomycin (Addgene), Catalog number 37632.

Cell lines: Female cells are of *Gallus gallus,* lymphoblast female cell line Con A - C1 - VICK, ordered from ATCC® Number: CRL-12135™. For the chicken cell culture experiment chicken fibroblast female cell line **UMNSAH/DF-1 (ATCC® CRL-12203™)** from ATCC were used. Male cells are of *Gallus gallus,* epithelial male cell line LMH/2A ordered from ATCC® Number: CRL-2118™.

Primordial Germ Cell (PGC) line.
human embryonic kidney 293 (HEK-293) cell line (ATTC).

### *Reagents and materials required for preparing the avian transgenic subjects*

### PGC Isolation

    1. Microelectrode pipette puller (Shutter Instrument Co.).
    2. Micro grinder (NARISHIGE).
    3. Small-diameter (25 $\mu$m) glass micropipette.

4. Mouth-controlled pipette (Sigma-Aldrich).

5. 1x Hank's balanced salt solution (HBSS) without CaCl2 or MgCl2 (Hyclone, Logan, UT).

6. 1x Dulbecco's phosphate-buffered saline (PBS), without Ca2 or Mg2 (Hyclone).

7. Trypsin/ethylenediaminetetraacetic acid (EDTA) (Gibco).

8. Chicken embryos in Hamburger and Hamilton (HH) stages 14-17.

9. Magnetic-activated cell sorting (MACS) buffer. 1x PBS supplemented with 0.5% bovine serum albumin (BSA) and 2 mM EDTA.

10. MiniMACS column (Miltenyi Biotec).

11. Stage-specific embryonic antigen 1 (SSEA-1) antibody (Santa Cruz Biotechnology, Santa Cruz, CA).

12. Anti-mouse IgM MicroBeads (Miltenyi Biotec).

**PGC Culture**

1. PBS (Hyclone).

2. HBSS (Hyclone).

3. PGC culture medium (50 mL). 3.75 mL fetal bovine serum (FBS, Hyclone), 1.25 mL chicken serum (Sigma-Aldrich, St. Louis, MO), 500 $\mu$L GlutaMAX-I Supplement (100x, Invitrogen), 500 $\mu$L nucleosides (100x,Millipore), 500 $\mu$L antibiotic/antimycotic (ABAM; 100x, Invitrogen), 500 $\mu$L insulin-transferrin-selenium supplement (100x, Gibco), 500 $\mu$Lnonessential amino acids (NEAA; 100x, Invitrogen), 50 $\mu$L $\beta$-mercaptoethanol (1000x, Sigma-Aldrich), 10 $\mu$L of 50 ng/$\mu$L human basic fibroblast growth factor (bFGF, Sigma-Aldrich), and add Knockout Dulbecco's modified Eagle's medium (DMEM; Invitrogen) up to 50 mL. Store at 4 °C.

4. Accutase (Millipore).

**Germline Chimera Production and Transgenesis**

Gene Transfer into Chicken PGC

1. Lipofectamine 2000 (Invitrogen).

2. Opti-MEM medium (Invitrogen).

3. Transfection medium (PGC culture media without ABAM; 50 mL): 3.75 mL FBS (Hyclone), 1.25 mL chicken serum (Sigma-Aldrich), 500 $\mu$L GlutaMAX-I supplement (100x,Invitrogen), 500 $\mu$L nucleosides (100x, Millipore), 500 $\mu$L insulin-transferrin-selenium supplement (100x, Gibco), 500 $\mu$L NEAA (100x, Invitrogen), 50 $\mu$L $\beta$-mercaptoethanol (1000 x, Sigma-Aldrich), and 10 $\mu$L of 50 ng/$\mu$L bFGF (Sigma-Aldrich), and add Knockout DMEM (Invitrogen) up to 50 mL. Store at 4 °C.

4. PBS, without Ca2 or Mg2.

5. HBSS, without Ca2 or Mg2.

6. Prepare plasmid DNA at 1 $\mu$g/$\mu$L.

In Vitro Proliferation and Selection of Transgenic PGCs

1. HBSS, without Ca2 or Mg2 (Hyclone).

2. Hemocytometer.

3. Trypan blue, 0.4% (Invitrogen).

4. Inverted fluorescence microscope (Leica Microsystems).

5. Geneticin® Selective Antibiotic (Gibco).

PGC Transplantation into Recipient Eggs

1. Small-diameter (25 $\mu$m) glass micropipette (made with microelectrode pipette puller (Shutter Instrument Co.) and a micro grinder (NARISHIGE).

2. Mouth-controlled pipette.
3. HBSS, without CaCl2 or MgCl2 (Hyclone).
4. Recipient chicken embryos at HH stages 14-17.
5. PBS, without Ca2 or Mg2 (Hyclone).
6. Pipette washing solution. 0.1% hydrogen peroxide (Sigma-Aldrich) in autoclaved distilled water.
7. Forceps.
8. Stereomicroscope with illuminator.
9. Hot-melt glue sticks and glue gun.
10. Parafilm.
11. Donor PGC prepared at 3 _ 103 cells/μL in HBSS.

Testcross

1. Wild-type Korean Ogye (KO) female chicken.
2. 1 mL syringe.
3. PBS, without Ca2 or Mg2 (Hyclone).

Donor-Derived Progeny and TransgenicChicken Validation

1. DNeasy Blood & Tissue kit (Qiagen).
2. Donor PGC-specific primer set.
3. Transgene-specific primer set.
4. PCR machine.
5. PCR reagents: PCR buffer, dNTP, Taq polymerase.
6. Agarose.
7. TAE buffer.
8. Electrophoresis machine.

### *Experimental procedures*

Fluorescence assay

The complete setup of the experiment comprises a laser source, a holder or stand enabling positioning of the egg in an appropriate position allowing the exposure of cells expressing the reporting gene within the egg, to said laser source, a lens, a filter, a stand for the detector and a detector as illustrated in **Figure 1**. The distance between the sample and the lens and between the lens and the detector is about 20cm. The focal plane of the lens is about 10 cm and the filter is deposited there.

The optimal conditions were detected by placing the filter before the detector and by covering all the surrounding area of the detector to ensure that a scattering light will not bypass the filter.

Three types of lasers were used with initial power $I_0$, and filters as indicate in the following **Table 2**:

**Table 2**: light source used:

| Laser type (Wavelength) | Power [W] | Filters |
|---|---|---|
| **Blue laser (473±1nm)** | $I_o = 17.3 mW$ | above 500 nm |
| **Green laser (532nm)** | $I_o = 3.9 mW$ | 540 nm-580nm |
| **Red laser (632.8nm)** | $I_o = 3.3 mW$ | above 660 nm |

Injection of transfected GFP or RFP-expressing cells into eggs

The HEK cells were maintained in DMEM medium, supplemented with 1% L-glutamine, 10% fetal bovine serum (FBS), and 1% PenStrep (penicillin + streptomycin) (Biological industries, Israel). The cells were grown in a humidified incubator at 37°C with 5% $CO_2$. Medium was renewed 2-3 times a week, and cell count was kept between $1 \times 10^5$ and $3 \times 10^5$ cells/mL. Cells were subcultured and resuspended in fresh medium as follow:

    1. Supernatant was removed from the flask for the cells to adhere to the flask surface.
    2. The dislodged cells were resuspended in 5 mL of fresh medium.

Followed the 2nd subculture, cell were transfected with 2 µg DNA of plasmid using PolyJet™ In Vitro DNA Transfection Reagent (SignaGen Laboratories, MD, USA) according to manufacturer protocol. Two Plasmids were transfected into the cells separately; a GFP plasmid and a RFP plasmid, as specified above.

Following, transfected cells (RFP) were prepared at a concentration of $10^6$ cells/ml. A total volume of 100 µl of cells was then injected to fresh chicken egg at the center, according to **Tables 3 and 4.**

**Table 3: RFP injection**

| Egg number | Egg condition | Saline (ul) | Cell line (ul) of 1M cells/ul | Cell line follow transfection of GFP or RFP |
|---|---|---|---|---|
| 1. | Untouched | 0 | 0 | --------------- |
| 2. | Small hole at top | 100 | 0 | 0.1ml saline |
| 11. | Small hole at top | 97 | 3 | ,3000 cells + RFP |
| 12. | Small hole at top | 97 | 3 | ,3000 cells + RFP |
| 13. | Small hole at top | 90 | 10 | ,10000 cells + RFP |
| 14. | Small hole at top | 90 | 10 | ,10000 cells + RFP |
| 15. | Small hole at top | 70 | 30 | ,30000 cells + RFP |
| 16. | Small hole at top | 70 | 30 | ,30000 cells + RFP |
| 17. | Small hole at top | 0 | 100 | ,100000 cells + RFP |
| 18. | Small hole at top | 0 | 100 | ,100000 cells + RFP |

In the second experiment cell were grow and transfected as described earlier. Cells were then transferred to either PBS or 50% glycerol (to control disperse) and injected into fresh chicken egg at the center. The different injected eggs are disclosed by **Table 4.**

**Table 4: RFP or GFP injection**

| Egg number | Egg condition | PBS (ul) or 50% glycerol | Cell line (ul) of 1M cells/ul | Cell line follow transfection of GFP or RFP |
|---|---|---|---|---|
| 1. | Untouched | 0 | | Control-NON transfected |
| 2. | Small hole at top | 100 | 0 | 0.2 ml PBS |
| 3. | Small hole at top | 100 | 0 | 0.2 ml 50% Glycerol |
| 4. | Small hole at top | 99 | 1 | 1000 cells in PBS |
| 5. | Small hole at top | 99 | 1 | 1000 cells in 50% Gly |
| 6. | Small hole at top | 97 | 3 | 3000 cells in PBS |
| 7. | Small hole at top | 97 | 3 | 3000 cells in 50% Gly |
| 8. | Small hole at top | 90 | 10 | 10000 cells in PBS |
| 9. | Small hole at top | 90 | 10 | 10000 cells in 50% Gly |
| 10. | Small hole at top | 70 | 30 | 30000 cells in PBS |

(continued)

| Egg number | Egg condition | PBS (ul) or 50% glycerol | Cell line (ul) of 1M cells/ul | Cell line follow transfection of GFP or RFP |
|---|---|---|---|---|
| 11. | Small hole at top | 70 | 30 | 30000 cells in 50% Gly |
| 12. | Small hole at top | 99 | 1 | 1000 cells in PBS |
| 13. | Small hole at top | 99 | 1 | 1000 cells in 50% Gly |
| 14. | Small hole at top | 97 | 3 | 3000 cells in PBS |
| 15. | Small hole at top | 97 | 3 | 3000 cells in 50% Gly |
| 16. | Small hole at top | 90 | 10 | 10000 cells in PBS |
| 17. | Small hole at top | 90 | 10 | 10000 cells in 50% Gly |
| 18. | Small hole at top | 70 | 30 | 30000 cells in PBS |
| 19. | Small hole at top | 70 | 30 | 30000 cells in 50% Gly |

Male chicken cell line

The Male cells, specifically, *Gallus gallus,* chicken (liver; chemically induced; transfected with chicken estrogen receptor gene), were grown in a waymouth's MB 752/1 medium supplemented with 1% L-glutamine, 10% fetal bovine serum (FBS), and 1% PenStrep (penicillin + streptomycin) (Biological industries, Israel). Culture flask (T25) was coated with 0.1% gelatin). Medium was renewed every 3 days. The cells were grown in a humidified incubator at 37°C with 5% $CO_2$.

Female chicken cell line

Chicken fibroblast female cell line **UMNSAH/DF-1 (ATCC® CRL-12203™**) from ATCC were used. More details on the cell is presented in Table 5:

**Table 5: Female chicken cell line**

| Organism | *Gallus gallus*, chicken |
|---|---|
| Cell type | fibroblast spontaneously transformed |
| Product Format | frozen |
| Morphology | fibroblast |
| Culture Properties | adherent |
| Age | 10 days gestation |
| Gender | Female |

The female cells were grown in DMEM medium supplemented with 1% L-glutamine, 10% fetal bovine serum (FBS), and 1% PenStrep (penicillin + streptomycin) (Biological industries, Israel). The cells were grown in a humidified incubator at 37°C with 5% $CO_2$. Medium was renew 2-3 times a week.

PGCs cell culture

Embryonic gonads are retrieved from White Leghorn (WL) embryos at 6.5 days of incubation and then the retrieved gonadal cells are treated with magnetic activated cell sorter (MACS) for separation of PGCs in total embryonic gonadal cells. PGCs are grown in KO-DMEM (Life Technologies), of which 40% is preconditioned on buffalo rat liver cells (BRL, ATCC), and supplemented with 7.5% fetal bovine serum (Hyclone), 2.5% irradiated chicken serum, 1X non-essential amino acids, 2mM glutamine, 1mM sodium pyruvate, 0.1mMβ-mercaptoethanol (all from Life Technologies), 4ng/ml recombinant human fibroblast growth factor, 6ng/ml recombinant mouse stem cell factor (both from R&D Systems) and grow on an irradiated feeder layer of BRL cells. The cells are passaged 3 times per week onto fresh feeder layers.

Restriction-free (RF) cloning

The insertion of gRNAs into Cas9-SmartNuclease™ vector is performed by applying the Restriction Free method [Peleg Y et al., J. Struct. Biol. 172(1):34-44 (2010) 2010]. Primers are ordered from Sigma-Genosys (Rehovot, Israel) and subsequent RF reactions were carried out using Phusion polymerase (Thermo Scientific, Hudson, NH, USA). Plasmid purification is carried out using the MEGAspin kit and DNA-spin plasmid DNA purification kit, respectively (Intron Biotechnology, Daejoen, South Korea).

Transfection into chromosomes W or Z of chickens

Cell are co-transfected with 2 μg DNA of each plasmid (see details below) by using PolyJet™. In Vitro DNA Transfection Reagent (SignaGen Laboratories, MD, USA) according to manufacturer protocol. Co-transfection of two plasmid is performed as follow:

1. pDsRed with chicken Chromosome Z Left and Right arms or Chromosome W Left and Right arms and CMV-hspCas9-H1-gRNA (assay).
2. pCMVGluc with chicken Chromosome Z Left &Right arms or Chromosome W Left & Right arms and CMV-hspCas9-H1-gRNA (control).

Cells are then plated with Neomycin-resistant and seeded in a 48-well plate at a density of $10^5$ cells per well. After 3 days, 40μg/ml Neomycin is added to select for cells with a stable integration of the reporter genes.

DNA analysis for integration into chromosome Z or W

For verification of chromosomal integration, the following strategy was used. Cells were isolated and DNA of half of them was extracted and run on an agar gel (1%). The genomic DNA (leaving the plasmid DNA on the gel) was cut and cleaned. A PCR assay was run with the following primers. The products were separated on a gel and then sequenced. Since the insert was too long to be detected by the PCR assay, sliding window PCR assay was designed that embark on initial PCR product.

**a. Assay for cell line with the insert** (includes part of the Left arm, CMV Enhancer, Promotor and MCS and part of the dsRED2):
Left primer-TTGGTGTGTGCTAATAGGCAGT as denoted by SEQ ID NO 49; right primer-TAGTCCTCGTTGTGGGAGGT as denoted by SEQ ID NO 50, the product size: 1489bp.
**b. Assay for cell line with the insert** (includes part of the flanking (to the arms) Z chromosome, Left arm, and part of the CMV Enhancer, Promotor):
Left primer- GCATTGATCTGTCCAGTTGC as denoted by SEQ ID NO 51, right primer- TACTGCCAAAACCGCATCAC as denoted by SEQ ID NO 52, product size: 1462bp.

Preparation of transgenic chickens

Concentrated vehicle (that may be either lentivirus at a titer of about $10^7$ MOI) or plasmid DNA) is injected to 25 embryos in new laid eggs. Injections are carried out weekly three injections. The injected embryos hatch 3 weeks after injection. These are G0 birds. Immediately after hatch, the DNA is extracted from CAM samples of the hatched chicks and detection of the presence/absence of vector DNA is carried out by semi-quantitative PCR. Blood sample G0 chicks at 2-3 weeks of age and repeat PCR screen. G0 birds are raised to sexual maturity, 16-20 weeks for males, 20-24 weeks for females. Cockerels are tested for semen production from approximately 16 weeks.
Hens are inseminated, fertile eggs collected daily. The G1 chicks are hatch 3 weeks later and each individual chick wing banded and a chick chorioallantoic membrane (CAM) sample taken from the shell. Extract DNA from CAM samples and carry out PCR screen for presence of transgene, predicted to be single copy level. Repeat screen to confirm and sex chicks on DNA from blood sample 2-3 weeks later.
At a few weeks of age a blood sample is taken from G1 birds to prepare genomic DNA for PCR analysis. G1 birds are used for breeding G2.

**PGC Isolation**

1. Incubate fresh chicken eggs (EGK stage X), for example, Korean Ogye (KO), at 37 °C for 50-54 h (HH stage 14-17) for blood PGC isolation. Place incubated eggs horizontally on the egg plate and gently wipe eggshell using sanitized cotton with 70% ethanol.

2. Using sharpened forceps, cautiously crack the eggshell (<1 cm diameter) for the isolation of whole blood cells.

3. Collect about 2-3 $\mu$L whole blood cells from the embryonic dorsal aorta using a 25 $\mu$m thinly ground glass needle and mouth pipette. Mix with 500 $\mu$L PBS. For microinjection of PGCs, a mouth pipette and a glass micropipette of 20-25 $\mu$m diameter may be used. The glass micropipette is made with a microelectrode pipette puller (Shutter Instrument Co.) and ground at 25 degrees using a micro grinder (NARISHIGE).

4. Transfer whole-blood cells to a 1.5 mL sterile tube, centrifuge (250 x g, 5 min), and remove the supernatant.

5. For gonadal PGC isolation, incubate fresh chicken eggs at 37 °C for 5.5 days (HH 20-26). Extract embryonic gonads from embryos at HH stages 20-26 with sharpened forceps, and incubate with 500 $\mu$L of 0.05% trypsin/EDTA at 37 °C incubator for 5 min.

6. Add 50 $\mu$L FBS for inactivation, and centrifuge (250 x g, 5 min) and remove the supernatant.

7. Resuspend whole blood cells or dissociated gonadal cells in 1 mL PBS and apply anti-SSEA-1 antibody (Santa Cruz Biotechnology, SC-21702) at 1:200 dilution, and then incubate the mixture for 15 min at room temperature (RT).

8. Wash cells to remove unbound primary antibody by adding 5 mL MACS buffer (0.5% BSA and 2 mM EDTA in PBS, pH 7.2) per 107 total cells and centrifuge (250 x g, 5 min).

9. Aspirate supernatant completely and re-suspend cell pellet in 80 $\mu$L MACS buffer per $10^7$ total cells.

10. Add 20 $\mu$L rat anti-mouse IgM MicroBeads (Miltenyi Biotec. 130-047-301) per $10^7$ total cells. For higher cell numbers, scale up buffer volume accordingly.

11. Incubate cells with antibody at 2-8 °C for 20 min.

12. Wash cells by adding 2 mL MACS buffer per $10^7$ total cells and centrifuge (250 x g, 5 min).

13. Aspirate the supernatant completely and re-suspend up to 108 total cells in 500 $\mu$L MACS buffer.

14. Place column in the magnetic field of a suitable MACS separator.

15. Prepare column by rinsing with 500 $\mu$L MACS buffer.

16. Apply cell suspension onto the column, and wash the column with 500 $\mu$L buffer three times. Add new buffer when the column reservoir is empty.

17. Remove column from the separator and place it on a suitable collection tube.

18. Add 1 mL MACS buffer to the column and immediately flush out the magnetically labeled cells by firmly pushing the plunger into the column.

19. Transfer the isolated cells containing PGCs to the pre-warmed PGC culture medium (1 mL PGC medium per 1 x $10^5$ purified PGCs).

### PGC Culture

1. Transfer the isolated cells containing PGCs to the pre-warmed PGC culture medium (1 mL PGC medium per 1 $\times$10 purified PGCs) and incubate at 37°C.

2. After 7-14 days of growth, most of the primary cultured PGCs form colonies and lose aggregates of cell colonies.

3. The suspended PGC colonies can be withdrawn gently with medium after gentle pipetting and centrifuging (200 x g, 5 min).

4. Disaggregate the cell pellet with Accutase (1 mL Accutase solution per ~5 $\times10^5$ PGCs).

5. Centrifuge (200 x g, 5 min) and re-suspend the cell pellet in a PGC culture medium.

6. Seed the suspended PGCs in a 12-well plate (1 x $10^5$ cultured PGCs in 1 mL PGC culture medium per well of a 12-well plate).

7. PGCs can be routinely subcultured every 3-4 days.

### Gene Transfer into Chicken PGCs, Germline Chimera Production, and Transgenesis

### Gene Transfer into Chicken PGCs

1. Mix 4 $\mu$g helper plasmid containing CAGG-PBase (pCyL43B) and 6 $\mu$g the piggyBac transposon (pCyL50) containing the reporter gene (RFP) and optionally, selectable marker (e.g., neomycin resistance gene) with 100 $\mu$L Opti-MEM (Gibco) and incubate for 5 min at RT. For CRISPR/Cas transfection, mix 2.5 $\mu$gCMVRFP expression plasmid vector, 2.5 $\mu$g Cas9 and guide RNA expression plasmid vectors with 100 $\mu$L Opti-MEM (Gibco) and incubate for 5 min at RT.

2. Mix 10 μL Lipofectamine 2000 reagent (Thermo Fisher-Invitrogen) with 100 μL Opti-MEM and incubate for 5 min at RT.

3. Mix the plasmids with Opti-MEM and Lipofectamine 2000 reagent with Opti-MEM and incubate for 20 min at RT.

4. During incubation, harvest cultured PGCs and centrifuge (200 x g, 5 min). Discard the supernatants.

5. Add 1 mL Accutase (Millipore) to the harvested PGCs and incubate for 10 min at 37 °C.

6. Determine the number of PGCs using a hemocytometer (Marienfeld) and seed $5 \times 10^5$ PGCs in a 12-well plate with 1 mL PGC culture medium without antibiotics.

7. Apply DNA-Lipofectamine complex to PGCs and incubate for 1 day at 37 °C in a CO2 incubator.

8. Harvest the transfected PGCs and centrifuge (200 x g, 5 min). Remove the supernatants.

9. Wash the PGCs with 1 mL HBSS three times and suspend them with the PGC culture medium with antibiotics.

### In Vitro Proliferation and Selection of Transgenic PGCs

1. Select the transfected PGCs in the PGC culture medium containing 100 μg/mL G418 (for the neomycin resistance gene) the day after transfection.

2. Monitor reporter gene expression using fluorescence microscopy.

3. Subculture the PGCs every 3-4 days. A complete selection period requires up to 3 weeks.

4. If there is no drug-selection marker in the transfected plasmid vectors, PGCs that express fluorescent protein can be sorted by FACS.

### PGC Transplantation into Recipient Eggs

1. Incubate recipient eggs up to HH stages 14-17 at 37 °C in air with 60-70% relative humidity.

2. Harvest the transfected PGCs and centrifuge (200 x g, 5 min) and remove the supernatants.

3. Add 1 mL Accutase (Millipore) to the harvested PGCs and incubate for 10 min at 37 °C.

4. Centrifuge (200 x g, 5 min). Discard the supernatants.

5. Suspend the PGCs in HBSS (Hyclone).

6. Make a small window on the pointed end of the recipient egg and microinject a 2 μL aliquot containing more than 3,000 PGCs with a micropipette into the dorsal aorta of the recipient embryo.

7. Seal the egg window of the recipient embryo with parafilm using the glue gun, and incubate the egg with the pointed end down until hatching at 37 °C in air with 60-70% relative humidity.

### Monitoring Genetically Modified PGCs in Embryonic Gonads

1. Incubate recipient eggs up to HH stages 28-30 at 37 °C in air with 60-70% relative humidity.

2. Dissect the gonad at embryonic day 6.

3. Monitor fluorescent protein expression in the embryonic gonads using fluorescence microscopy.

Testcross

1. Collect semen twice in 1 week from sexually mature recipients and wild-type (WT) chickens.

2. Introduce 50 μL semen from mature male recipients and WT roosters to WT laying hens and mature female recipients, respectively.

3. Collect eggs from WT laying hens and mature female recipients the day after artificial insemination, and incubate the egg with the pointed end down until hatching at 37 °C in air with 60-70% relative humidity.

## EXAMPLE 1

### *Fluorescence analysis using different colored-lasers and filters for visual gender identification in poultry*

Optical fluorescent system

In order to demonstrate the feasibility of visually identify gender of in-ovo poultry, the use of green fluorescence as compared to blue fluorescence or red fluorescence reporter genes was evaluated.

The autofluorescence of complete eggs or of eggs separated into egg white, egg yolk and shell was determined using a blue laser, a green laser or a red laser. Comparison of an empty egg (shell) to a complete egg indicates no significant difference in autofluorescence level (data not shown).

The scattering and autofluorescence intensity provided by the different components and parts of the egg is described in **Table 6.**

Importantly, it was noted that the background noise (without laser) was about 4nW, while in the presence of the laser (532nm), it was about 12nW due to light scattering that bypass the filter (low pass filter that passes above 660nm).

**Table 6. Scattering and autofluorescence intensity of the parts of the egg using 3 different fluorescent lasers**

| Laser | Scattering Intensity | | | Autofluorescence Intensity | | |
|---|---|---|---|---|---|---|
| Experiment | Empty container | Container + egg white | Container + egg yellow | Empty container | Container + egg white | Container +egg yellow |
| **Blue laser (473±1nm)** | *2mW* | 1.02*mW* | 1.2*mW* | 16.5*nW* | 26.12 ± 6.25*nW* | 10.33 ±1.5*nW* |
| **Green laser (532nm)** | *370,uW* | 484,uW | 415$\mu W$ | 13.6*nW* | 14.5*nW* | 16*nW* |
| **Red laser (632.8nm)** | 300$\mu W$ | 160$\mu W$ | 156$\mu W$ | 13*nW* | 8.33±1.86*nW* | 10.82±3.42*nW* |

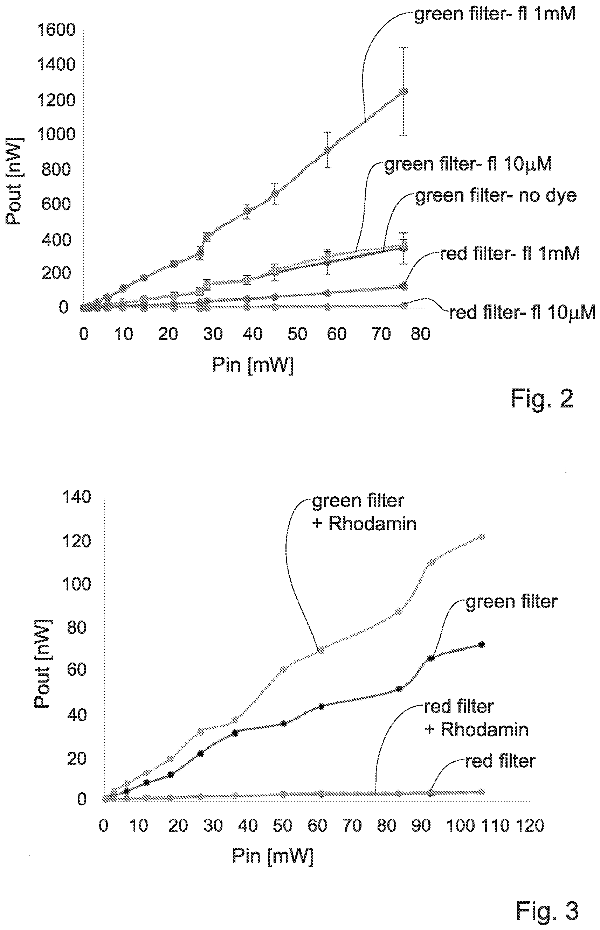

The use of a blue laser (473 nm) together with a green (+500 nm) or red filter (590-650 nm) with or without addition of different concentrations of fluorescein (1$\mu$M, 10mM) into a complete egg was assayed and is summarized in **Figure 2.** It appears that there is a large green autofluorescence, however fluorescence intensity could be detected upon using high fluorescein concentration.

The use of a green laser (532 nm) together with a dark green (540-580 nm) or red filter (+660 nm) with or without addition of Rhodamine B (10 $\mu$M) into a complete egg was assayed and is summarized in **Figure 3.** It appears that above 40mW, the fluorescent emission of the dye is considerably detectable with the green filter (almost 2 times larger). However, with the red filter, no difference could be observed.

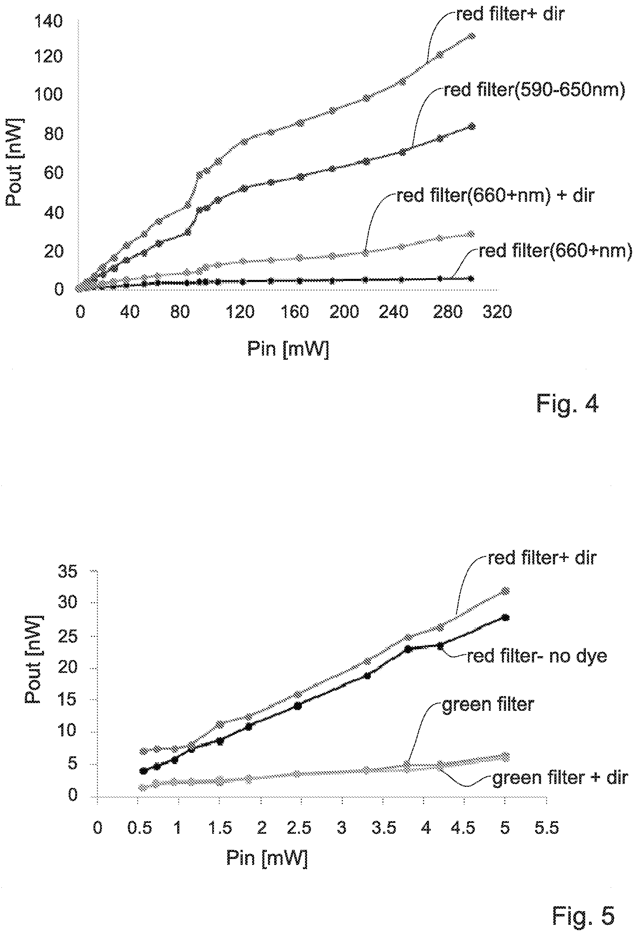

The use of a green laser (532 nm) together with a red filter A (590-650 nm) or a red filter B (+660 nm) with or without addition of dir (10 $\mu$M) into a complete egg was assayed and is summarized in **Figure 4.** It appears that above 80mW, the fluorescent emission of the dye is detectable using Red filter A while by using the Red filter B (+660nm), only a modest difference can be observed.

The use of a red laser (632.8 nm) together with a red filter (+660 nm) or a green filter (540-580 nm) with or without addition of dir (10 $\mu$M) into a complete egg was assayed and is summarized in **Figure 5.** Almost no intensity difference was shown with or without fluorescent dye.

All of the experiments were repeated using only the egg shell. Similar results were observed (data not shown).

These observations demonstrate the feasibility of detecting RFP through the egg shell.

## EXAMPLE 2

### *Fluorescence of RFP or GFP transfected cells into eggs*

To illustrate the feasibility of *in-ovo* detection of RFP expressing cells in an embryo, the inventors next examined if cells transfected with a fluorescent protein, specifically, RFP or GFP, can be detected upon injection thereof into a complete egg.

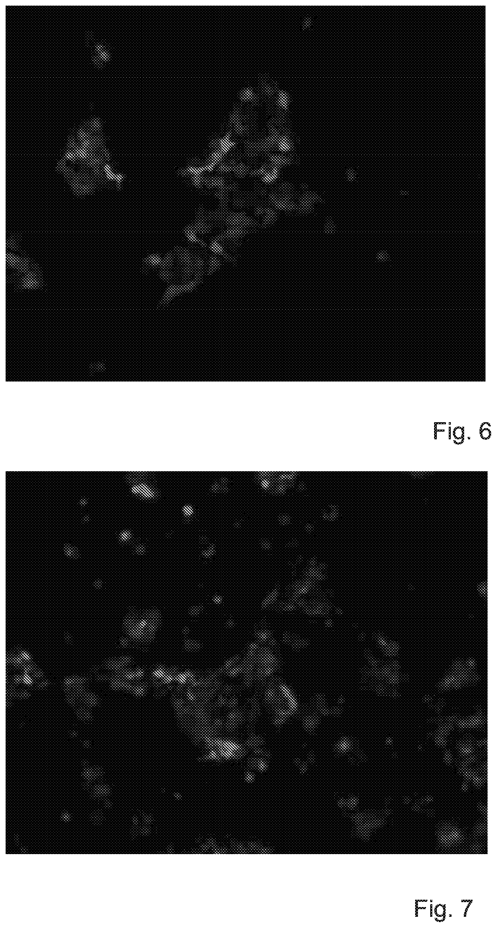

More specifically, HEK cells were transfected either with GFP or RFP vectors as described in in Experimental procedures. The fluorescence of both GFP and RFP transfected cells was examined following excitation. **Figure 6** (GFP) and **Figure 7** (RFP) clearly demonstrate that both GFP and RFP are expressed by the transfected cells and fluorescent signals can be observed.

HEK transfected cells were then injected into fresh eggs as described in Experimental procedures and the fluorescence intensity of eggs was assayed.

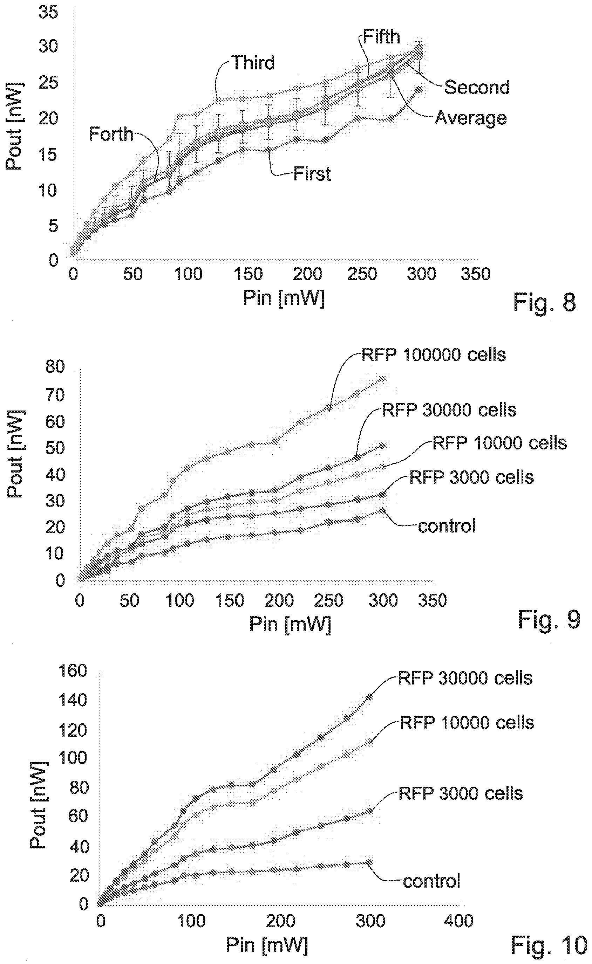

First, the fluorescence intensity of eggs with or without the Red Fluorescent Protein RFP-expressing cells was characterized by excitation of the eggs with the green laser (532nm) and a Red filter of +650nm. **Figure 8** shows that the fluorescence intensity of egg without RFP measured with five **different positions of the egg** is quite similar. **Figure 9** shows the measured fluorescence intensity using different concentrations of RFP-expressing cells when the egg was excited at the center. The correlation between the RFP concentration and the intensity on the detector was clearly observed.

Several tests were performed in order to check if in the presence of RFP expressing cells, the position of the egg, i.e. the exact place of excitation provided by the laser, was influencing the fluorescence intensity. It appears that the intensity on the detector greatly changed with the position of the egg during excitation. This was expected since, following injection of RFP-expressing cells into the egg, RFP is not uniformly distributed. **Figure 10** presents the maximum intensity observed at a specific position of the egg during excitation, which corresponds to the position where the concentration of RFP-expressing cells is the highest. This figure demonstrate that a minimal amount of about 3000 cells can be detected.

In embryo, it is known in the art that gender cells, PGCs are positioned in the center of the epiblast of freshly laid egg at developmental stage X of the egg. It should be noted that the embryo always facing the upper side of the egg yolk sack.

The results exposed above suggest that upon excitation of the region situated few millimeters behind the egg shall and nearby the yolk, maximal RFP emission may be detected.

The experiments were repeated one week following injection of the eggs with RFP expressing cells. The results were similar demonstrating that the RFP dye did not diffuse inside the egg.

The influence of PBS and glycerol on the fluorescence intensity of eggs using the green laser (532 nm) and the red filter of +650 nm was examined with or without the presence of RFP-expressing cells. It appears that neither glycerol nor PBS affect fluorescence intensity measurements of RFP expressing cells into eggs as illustrated in **Figure 11A and 11B.**

Following, the influence of PBS and glycerol on the fluorescence intensity of eggs using the blue laser (483 nm) and the filter of +500 nm was examined with or without the presence of GFP-expressing cells. It appears that GFP-expressing cells could not be detected in any given concentration (1000, 3000 10,000 and 30,000 cells), as illustrated by **Figure 12** for example with 30,000 GFP expressing cells. The same results were obtained using three different filters: +500 nm, 530-550 nm and 540-580 nm.

To further evaluate the surprising advantage of using RFP as a reporter gene, the fluorescence of GFP and RFP-expressing cells were finally compared as illustrated in **Figure 13**. The parameter R was defined to indicate the ratio between the fluorescence protein (FP) intensity ($I_{FP}$) and the autofluorescence intensity ($I_{autofluorescence}$):

$$R = \frac{I_{FP}}{I_{autofluorescence}}$$

The R parameter was calculated for different concentrations of both GFP and RFP expressing cells. As indicated by the Figure, it appears that GFP-expressing cells are not detectable in any of the given concentration (1000 and 30,000 cells as shown in **Figure 13**).

On the other hand, RFP-expressing cells provided significant R parameter values even at low concentration (1000 RFP-expressing cells). At high concentration of RFP-expressing cells (30000 RFP-expressing cells), the R value reached 3, and thus clearly demonstrating that the RFP reporter gene can be easily detected in excited eggs.

The main findings are summarized below:

    1. There is a strong autofluorescence at the blue while there is no autofluorescence in the green and red.

    2. The main autofluorescence originates from the eggshell.

    3. The system can detect green and red fluorescence dyes with relatively low dyes concentration and with low excitation power.

    4. Blue fluorescence is hardly detected (only in mM dyes concentration) due to the autofluorescence.

    5. RFP is much easily and significantly detectable inside the eggs.

    6. GFP type is not detectable in this system.

    7. The exact location of the RFP-expressing cells inside the egg is significant to define the region of excitation.

    8. The RFP dye did not diffuse into the eggs during 14 days from injection.

9. Nor PBS or Glycerol affects RFP detection.

These results clearly demonstrate that an embryo expressing a RFP gene integrated in a gender chromosome, can be detected in early developmental stage, where about 1000 cells express RFP.

## EXAMPLE 3

### *Design of guide RNAs vector*

In order to incorporate the RFP reporter gene into the gender chromosomes W or Z, the CRISPR/Cas9 mediated HDR method is selected. Relevant gRNA sites are then sought from both gender chromosomes.

Female Z chromosome integration

The regions 9156874-9161874, as denoted by SEQ ID NO:15, 27764943-27769943, as denoted by SEQ ID NO: 16, 42172748-42177748, as denoted by SEQ ID NO: 17, 63363656-63368656, as denoted by SEQ ID NO:18 and 78777477-78782477, as denoted by SEQ ID NO: 19 of Chromosome Z of female chicken are analyzed for guide RNA design. More specifically, for directing the integration of the RFP into Z chromosome, a gRNA designated gRNA7 of Z chromosome locus chrZ_42174515_-1, comprising the nucleic acid sequence GTAATACAGAGCTAAACCAG, as also denoted by SEQ ID NO:26, was next prepared.

The procedure was performed according to the "PrecisionV Cas9 SmartNuclease Vector System" user manual. More specifically, two primers were designed for the cloning: ChZgRNA_Forward: TGTATGAGACCACGTAATACAGAGCTAAACCAG, as denoted by SEQ ID NO. 53, ChZgRNA_Reverse: AAACCTGGTTTAGCTCTGTATTACGTGGTCTCA, as denoted by SEQ ID NO. 54. The primers were annealed to generate a duplex in a reaction mix that contains 5μM of each primer. The mixture was incubated at 95°C for 5 minutes and the removed to cool down to RT.

Later the duplex was cloned into the provided linearized Cas9 vector by ligation reaction that was set as follows: 1 μl of the linearized vector, 3 μl of the annealed oligo mix, 1 μl of ligation buffer and 0.25 μl of the Fast ligase. The mixture was incubated for 5-7 minutes at 25°C.

The mixture was transformed into DH5α competent cells and the cells were cultured on an LB plate containing 50 μg/ml Kanamycin and incubated overnight at 37°C.

The next day, two colonies were picked randomly and were grown in LB/Kanamycin medium overnight at 37°C with shaking.

The next day, plasmid DNAs were prepared using Qiagen mini-prep kit and were sequenced using the provided sequencing primer.

In yet some further embodiments, four additional guide RNAs are selected, synthesized and cloned separately into the Cas9 SmartNuclease vector containing the wild type Cas9 nuclease (Horizon) by Restriction free cloning protocol: gRNA3: ACAGACCTATGATATGT, as denoted by SEQ ID NO. 11; gRNA4: CGATTATCACTCACAAG, as denoted by SEQ ID NO. 12; gRNA5: CTGGTTAGCATGGGGAC, as denoted by SEQ ID NO. 13; gRNA6: GTAAAGAGTCAGATACA, as denoted by SEQ ID NO. 14.

Further non-limiting examples for gRNA sequences suitable for integration into specific loci within the Z chromosome, may include but are not limited to gRNA8 of Z chromosome locus chrZ_9157091_1, comprising the nucleic acid sequence ACAGACCTATGATATGTGAG, as also denoted by SEQ ID NO:27, gRNA9 of Z chromosome locus chrZ_27767602_-1, comprising the nucleic acid sequence GAGCTTGTGAGTGATAATCG, as also denoted by SEQ ID NO:28, gRNA10 of Z chromosome locus chrZ_78779927_1, comprising the nucleic acid sequence GTAAAGAGTCAGATACACAG, as also denoted by SEQ ID NO: 29, and gRNA11 of Z chromosome locus chrZ_63364946_-1, comprising the nucleic acid sequence CAGTGGGTACTGAAGCTGTG as also denoted by SEQ ID NO: 30.

These gRNAs have few predicted off-target sites, none of which were in known coding sequences.

Female W chromosome integration

The region 1022859-1024215 of Chromosome W of female chicken, comprising the nucleic acid sequence as denoted by SEQ ID NO. 3, is analyzed for guide RNA design.

Two guide RNAs are selected, synthesized and cloned separately into the Cas9 SmartNuclease vector containing the wild type Cas9 nuclease (Horizon) by Restriction free cloning protocol: gRNA1: GCACTAGGAACCAGCAGCAG, as denoted by SEQ ID NO. 1 and gRNA2: GTAGCCCCAAGAGGGCTAGG, as denoted by SEQ ID NO. 2. The predicted parameters of these two gRNAs are presented in Table 7:

**Table 7**

| gRNA parameters | gRNA1 | gRNA2 |
|---|---|---|
| sgRNA designer | 0.506 | 0.63 |
| sscore | 0.8677 | 0.5323 |
| sgRNA scorer | 94.8 | 99.9 |

**EXAMPLE 4**

*Design of RFP targeting vector*

Flanking sequences homological of the appropriate flanking sequences indicated above of female W chromosome or of the female Z chromosome loci, are introduced into the RFP-expressing vector, specifically, the pDsRed1-N1 plasmid.

For, integrating the reporter gene of the invention into the specific locus within the Z chromosome, left arm comprising the nucleic acid sequence as denoted by SEQ ID NO. 31, and right arm comprising the nucleic acid sequence as denoted by SEQ ID NO. 32, may be used to integrate the reporter gene of the invention to the specific loci directed by gRNA7 of SEQ ID NO:26.

More specifically, to integrate the reporter gene of the invention to the specific loci directed by gRNA7, specifically, GTAATACAGAGCTAAACCAG, as denoted by SEQ ID NO:26, cloning of the 'Left arm' was performed upstream the CMV enhancer in pDsRed1-N1 plasmid, using the RF methodology.

The following primers were used:

Forward primer:
ACGGGGTCATTAGTTCATAGCCCATATATGGTGAACGGATAGGCAGCAAGC, as denoted by SEQ ID NO. 55 (contains the 5' Left arm sequence and 30 nucleotides of the site of integration in the plasmid).
Reverse primer:
GGCGGGCCATTTACCGTAAGTTATGTAACGTGAGGAAGGGTCTGTTACTGG A, as denoted by SEQ ID NO. 56.

The Right arm was cloned downstream the Neo resistance gene, using the following primers.

Forward primer:
TTCTATCGCCTTCTTGACGAGTTCTTCTGAGTCATGTCGGTGGAGGAGAAA, as denoted by SEQ ID NO. 57 (3' Neo resistance sequence marked in green)
Reverse primer:

GTGATGGCAGGTTGGGCGTCGCTTGGTCGGGCATGGGATGTTAAAGAGAAG CT,

as denoted by SEQ ID NO. 58 (In orange vector sequences 3' to the Neo gene).

Cloning was verified by sequencing to ensure proper integration.

Note that there are 193 nucleotides deletion from the CMV enhancer due to repetition of 16 nucleotides (CGGTAAATGGCCCGCC, as denoted by SEQ ID NO. 59) in the enhancer region flanking the site of integration.

In yet some further embodiments, the RFP reporter gene may be cloned for using either the gRNA3, as denoted by SEQ ID NO. 11, gRNA4 : as denoted by SEQ ID NO. 12, gRNA5, as denoted by SEQ ID NO. 13, gRNA6, as denoted by SEQ ID NO. 14.

For cloning using the gRNA3, "Left arm" comprising the nucleic acid sequence as denoted by SEQ ID NO. 41, and the "Right arm" comprising the nucleic acid sequence as denoted by SEQ ID NO. 42 are provided. For cloning using the gRNA4, "Left arm" comprising the nucleic acid sequence as denoted by SEQ ID NO. 43, and the "Right arm" comprising the nucleic acid sequence as denoted by SEQ ID NO. 44 are provided. For cloning using the gRNA5, "Left arm" comprising the nucleic acid sequence as denoted by SEQ ID NO. 45, and the "Right arm" comprising the nucleic acid sequence as denoted by SEQ ID NO. 46 are provided. For cloning using the gRNA6, "Left arm" comprising the nucleic acid sequence as denoted by SEQ ID NO. 47, and the "Right arm" comprising the nucleic acid sequence as denoted by SEQ ID NO. 48 are provided.

In further embodiments, for integrating the reporter gene of the invention into the specific locus within the Z chromosome, left arm comprising the nucleic acid sequence as denoted by SEQ ID NO. 33, and right arm comprising the nucleic acid sequence as denoted by SEQ ID NO. 34, may be used to integrate the reporter gene of the invention to the specific loci directed by gRNA8 of SEQ ID NO:27. In still further embodiments, for integrating the reporter gene of the invention into the specific locus within the Z chromosome, left arm comprising the nucleic acid sequence as denoted by SEQ ID NO. 35, and right arm comprising the nucleic acid sequence as denoted by SEQ ID NO. 36, may be used to integrate the reporter gene of the invention to the specific loci directed by gRNA9 of SEQ ID NO:28. In further embodiments, for integrating the reporter gene of the invention into the specific locus within the Z chromosome, left arm comprising the nucleic acid sequence as denoted by SEQ ID NO. 37, and right arm comprising the nucleic acid sequence as denoted by SEQ ID NO. 38, may be used to integrate the reporter gene of the invention to the specific loci directed by gRNA10 of SEQ ID NO:29. In yet a further embodiment, for integrating the reporter gene of the invention into the specific locus within the Z chromosome, left arm comprising the nucleic acid sequence as denoted by SEQ ID NO. 39, and right arm comprising the nucleic acid sequence as denoted by SEQ ID NO. 40, may be used to integrate the reporter gene of the invention to the specific loci directed by gRNA11 of SEQ ID NO:30.

For the female W chromosome, the reporter gene, specifically RFP may be cloned for using either the Guide 1 (gRNAI), as denoted by SEQ ID NO. 1 or Guide 2 (gRNA2): as denoted by SEQ ID NO. 2. For cloning using the gRNA1, "Left arm" comprising the nucleic acid sequence as denoted by SEQ ID NO. 4, and the "Right arm" comprising the nucleic acid sequence as denoted by SEQ ID NO. 5 are provided. For cloning using the gRNA2, "Left arm" comprising the nucleic acid sequence as denoted by SEQ ID NO. 6, and the "Right arm" comprising the nucleic acid sequence as denoted by SEQ ID NO. 7 are provided.

Still further, a "left arm" for the region upstream to the CMV-promoter comprises the nucleic acid sequence as denoted by SEQ ID NO. 8, and a "right arm" for the region downstream the Neomycin-resistance, may comprise the nucleic acid sequence as denoted by SEQ ID NO. 9, or SEQ ID NO. 10 for the region downstream the polyA site.

**EXAMPLE 5**

*Integration of the RFP reporter gene into the Z chromosome of female chicken cell line*

Female chicken cell line was co-transfected with two plasmids i.e. pDsRed containing the chicken Chromosome Z Left and Right arms and the plasmid containing the CMV-hspCas9-H1-gRNA as detailed in the Experimental procedure section.

The employed gRNA sequence was the gRNA 7 as denoted by **SEQ ID NO: 26. Table 8** provides more details on the used guide RNA.

**Table 8. Characteristics of the used gRNA 7**

| proto spacer gRNA | strand | cut_site | chromosome | guide_id |
|---|---|---|---|---|
| GTAATACAGAGCT AAACCAG, SEQ ID NO. 26 | -1 | 4217 4515 | chrZ | ZNF367_HABP4_ENSGALG00000012629_E NSGALG00000012628_chrZ_42174515_-1 |

Transfected cells were then exited with red fluorescent. As can be seen in **Figure 14** the transfection was positive. The illuminate cells were collected, the genomic DNA was extracted and the sliding PCR assay (as described in the Experimental procedures) was performed. PCR fragments were then send for SANGER sequencing and the integrated sequences as illustrated in **Figure 15** were obtained, which demonstrate successful integration of the RFP gene into the Z chromosome.

In conclusion, these results demonstrate that:

- successful growing procedure of female chicken cell line was established
- successful transfection of the RFP reporter gene and CRISPR Cas9 system into female chicken cell line was established
- a strong fluorescence signal was generated in red excitation
- successful point integration of the RFP reporter gene into the Z chromosome was established

**EXAMPLE 6**

***Germline transmission of CRISPR-treated cells***

The use of primordial germ cells (PGCs) to produce transgenic chickens has many advantages in animal biotechnology and biomodeling. Because chicken embryos are oviparous, PGCs at an early stage are readily accessible and can be manipulated in vitro for practical applications, including the restoration of genetic material, genome editing, and transgenic research. Significant efforts have been made to establish culture systems for chicken PGCs from different embryonic origins, and it has been demonstrated that only chicken PGCs can be maintained indefinitely in vitro without losing their properties.

More specifically, in chickens, PGCs first separate in the epiblast in Eyal-Giladi and Kochav (EGK) stage X embryos and then move down the hypoblast of the area pellucida, and subsequently to the germinal crescent and enter the blood stream. After migration through the circulatory system, they arrive at the genital ridges.

These unique characteristics in germline development make it possible to use them to produce transgenic chickens via the injection of genetically manipulated PGCs into the blood vessels of recipient eggs.

The two above described vectors, specifically, the gRNA/Cas9 and the reporter-gene vectors are co-transfected to PGCs as detailed in experimental procedures. After stable clones are identified, the cells are expanded and confirmed for the RFP integration by PCR. Confirmed clones are injected into recipient chicken embryos at Stage 14-16 (H&H). The injected embryos are transferred to surrogate shells and incubated until hatch at 37°C. The sex of the chicks is determined after hatch by PCR for the W- or Z chromosomes.

Female and Male chimeras are grown to sexual maturity and bred to wild type male and female chickens. Hatched chicks are evaluated for the expression of RFP, and the germline progeny are confirmed by PCR to carry targeted RFP.

**Claims**

1.  A method of gender determination of an avian embryo in an unhatched egg comprising the embryo within a structurally integral shell, the method comprising the step of:

    (a) providing at least one transgenic avian subject comprising at least one exogenous reporter gene integrated into at least one position or location in at least one of gender chromosomes Z and W, wherein said reporter gene is a Red Fluorescent Protein (RFP); and
    (b) in an embryo comprised within a structurally integral shell in an unhatched egg obtained from said transgenic avian determining within said structurally integral shell of said unhatched egg if at least one detectable signal is detected, wherein detection of said at least one detectable signal indicates the expression of said at least one reporter gene in said embryo within said structurally integral shell of said unhatched egg, thereby the presence of said Z chromosome or W chromosome in said embryo,
    thereby determining the gender of an avian embryo in an unhatched egg comprising the embryo within a structurally integral shell .

2.  The method according to claim 1, wherein said reporter gene encodes an RFP protein having an excitation wavelength of 500-650 nm and an emission wavelength of 550-650 nm.

3.  The method according to claim 1 or 2, wherein said determining if a detectable signal is detected comprises the step of subjecting said unhatched egg comprising the embryo within a structurally integral shell to a light source.

4.  The method according to any one of claims 1 to 3, wherein said at least one transgenic avian subject is a female avian subject, and wherein said at least one reporter gene is integrated into at least one position of: (a) female chromosome Z, thereby detection of a detectable signal indicates that said embryo in said unhatched egg is a mate; or (b) female chromosome W, thereby detection of a detectable signal indicates that said embryo in said unhatched egg comprising the embryo within a structurally integral shell is a female.

5.  The method according to any one of claims 1 to 4, wherein said at least one reporter gene is integrated into said gender chromosome of said transgenic avian animal using at least one programmable engineered nuclease (PEN), wherein said PEN is a clustered regularly interspaced short palindromic repeat (CRISPR) type II system.

6.  The method according to any one of claims 1 to 5, wherein said at least one reporter gene is integrated into a gender chromosome of said transgenic avian subject by contacting or co-transfecting at least one cell of said avian subject or at least one cell introduced into said avian animal, with:

> a. at least one Cas9 protein or at least one first nucleic acid sequence comprising at least one nucleic acid sequence encoding said at least one Cas9 protein; and guide RNA (gRNA) that targets at least one protospacer within at least one gender chromosome Z and/or W, or at least one nucleic acid sequence encoding said at least one gRNA; and
> b. at least one second nucleic acid sequence comprising at least one said reporter gene.

7. The method according to any one of claims 1 to 6, wherein said at least one reporter gene is integrated into at least one site at: gender Z chromosome locus 42172748-42177748 or gender W chromosome locus 1022859-1024215.

8. The method according to claim 7, wherein said at least one reporter gene is integrated into at least one site at: (a) gender Z chromosome locus 42172748-42177748, wherein said at least one gRNA comprises the nucleic acid sequence as denoted by SEQ ID NO. 26 and said at least one reporter gene comprised within said second nucleic acid is flanked at 5' and 3' thereof by homologous arms comprising the nucleic acid sequence as denoted by SEQ ID NO. 31 and 32, respectively; or (b) gender W chromosome locus 1022859-1024215, wherein at least one of:

> (i) said gRNA comprises the nucleic acid sequence as denoted by SEQ ID NO. 1, and said at least one reporter gene comprised within said second nucleic acid sequence is flanked at 5' and 3' thereof by homologous arms comprising the amino acid sequence as denoted by SEQ ID NO. 4 and 5, respectively; and
> (ii) said gRNA comprises the nucleic acid sequence as denoted by SEQ ID NO. 2, and said at least one reporter gene comprised within said second nucleic acid sequence is flanked at 5' and 3' thereof by homologous arms comprising the amino acid sequence as denoted by SEQ ID NO. 6 and 7, respectively.

9. An avian transgenic subject comprising at least one exogenous reporter gene integrated into at least one locus in at least one of gender chromosome Z and W, wherein said reporter gene is a Red Fluorescent Protein (RFP), and wherein the expression of said at least one reporter is suitable for identification of a gender of an avian embryo in an unhatched egg comprising the embryo within a structurally integral shell.

10. The avian transgenic subject according to claim 9, wherein said reporter gene encodes an RFP protein having an excitation wavelength of 500-650 nm and an emission wavelength of 550-650 nm.

11. The avian transgenic subject according to claim 9 or 10, wherein said at least one transgenic avian subject is female, and wherein said at least one reporter gene is integrated into at least one position of: female chromosome Z or female chromosome W.

12. The avian transgenic subject according to any one of claims 9 to 11, wherein said at least one reporter gene is integrated into at least one site at gender Z chromosome locus 42172748-42177748, wherein said at least one gRNA comprises the nucleic acid sequence as denoted by SEQ ID NO. 26 and said at least one reporter gene comprised within said second nucleic acid is flanked at 5' and 3' thereof by homologous arms comprising the nucleic acid sequence as denoted by SEQ ID NO. 31 and 32, respectively.

13. The avian transgenic subject according to any one of claims 9 to 11, wherein said at least reporter gene is integrated into at least one site at gender W chromosome locus 1022859-1024215, wherein at least one of:

> (a) said gRNA comprises the nucleic acid sequence as denoted by SEQ ID NO. 1, and said at least one reporter gene comprised within said second nucleic acid sequence is flanked at 5' and 3' thereof by homologous arms comprising the amino acid sequence as denoted by SEQ ID NO. 4 and 5, respectively; and
> (b) said gRNA comprises the nucleic acid sequence as denoted by SEQ ID NO. 2, and said at least one reporter gene comprised within said second nucleic acid sequence is flanked at 5' and 3' thereof by homologous arms comprising the amino acid sequence as denoted by SEQ ID NO. 6 and 7, respectively.

14. A kit comprising:

> a. at least one Cas9 protein or at least one first nucleic acid sequence comprising at least one nucleic acid sequence encoding said at least one Cas9 protein; and at least one gRNA that targets at least one protospacer within at least one gender chromosome Z and/or W, or nucleic acid sequence encoding said at least one gRNA;

and

b. at least one second nucleic acid sequence comprising at least one reporter gene, wherein said reporter gene is a Red Fluorescent Protein (RFP); wherein the expression of said at least one reporter is suitable for identification of a gender of an avian embryo in an unhatched egg comprising the embryo within a structurally integral shell,

wherein said at least one reporter gene is integrated into at least one site at: (a) gender Z chromosome locus 42172748-42177748, wherein said at least one gRNA comprises the nucleic acid sequence as denoted by SEQ ID NO. 26 and said at least one reporter gene comprised within said second nucleic acid is flanked at 5' and 3' thereof by homologous arms comprising the nucleic acid sequence as denoted by SEQ ID NO. 31 and 32, respectively; or (b) gender W chromosome locus 1022859-1024215, wherein at least one of: (i) said gRNA comprises the nucleic acid sequence as denoted by SEQ ID NO. 1, and said at least one reporter gene comprised within said second nucleic acid sequence is flanked at 5' and 3' thereof by homologous arms comprising the amino acid sequence as denoted by SEQ ID NO. 4 and 5, respectively; and (ii) said gRNA comprises the nucleic acid sequence as denoted by SEQ ID NO. 2, and said at least one reporter gene comprised within said second nucleic acid sequence is flanked at 5' and 3' thereof by homologous arms comprising the amino acid sequence as denoted by SEQ ID NO. 6 and 7, respectively.

15. The kit according to claim 14, wherein said at least one reporter gene encodes an RFP protein having an excitation wavelength of 500-650 nm and an emission wavelength of 550-650 nm.

**Patentansprüche**

1. Verfahren zur Geschlechtsbestimmung eines Vogelembryos in einem ungeschlüpften Ei, das den Embryo innerhalb einer strukturell integralen Schale umfasst, wobei das Verfahren den Schritt umfasst:

(a) Bereitstellen mindestens eines transgenen Vogelsubjekts, umfassend mindestens ein exogenes Reportergen, das in mindestens eine Position oder Stelle in mindestens einem der Geschlechtschromosomen Z und W integriert ist, wobei das Reportergen ein rot fluoreszierendes Protein (RFP) ist;

(b) Bestimmen in einem Embryo, der innerhalb einer strukturell integralen Schale in einem ungeschlüpften Ei umfasst ist, das von dem transgenen Vogel erhalten wird, ob mindestens ein nachweisbares Signal innerhalb der strukturell integralen Schale des ungeschlüpften Eies nachgewiesen wird, wobei ein Nachweis des mindestens einen nachweisbaren Signals die Expression des mindestens einen Reportergens in dem Embryo innerhalb der strukturell integralen Schale des ungeschlüpften Eies anzeigt, wodurch das Vorhandensein des Z-Chromosoms oder W-Chromosoms in dem Embryo angezeigt wird, wodurch das Geschlecht eines Vogelembryos in einem ungeschlüpften Ei, das den Embryo innerhalb einer strukturell integralen Schale umfasst, bestimmt wird.

2. Verfahren nach Anspruch 1, wobei das Reportergen für ein RFP-Protein mit einer Anregungswellenlänge von 500-650 nm und einer Emissionswellenlänge von 550-650 nm kodiert.

3. Verfahren nach Anspruch 1 oder 2, wobei das Bestimmen, ob ein nachweisbares Signal nachgewiesen wird, den Schritt umfasst, das ungeschlüpfte Ei, das den Embryo innerhalb einer strukturell integralen Schale umfasst, einer Lichtquelle auszusetzen.

4. Verfahren nach einem der Ansprüche 1 bis 3, wobei das mindestens eine transgene Vogelsubjekt ein weibliches Vogelsubjekt ist, und wobei das mindestens eine Reportergen in mindestens eine Position der Folgenden integriert wird:

(a) weibliches Chromosom Z, wodurch ein Nachweis eines nachweisbaren Signals anzeigt, dass der Embryo in dem ungeschlüpften Ei männlich ist; oder

(b) weibliches Chromosom W, wodurch ein Nachweis eines nachweisbaren Signals anzeigt, dass der Embryo in dem ungeschlüpften Ei, das den Embryo innerhalb einer strukturell integralen Schale umfasst, weiblich ist.

5. Verfahren nach einem der Ansprüche 1 bis 4, wobei das mindestens eine Reportergen in das Geschlechtschromosom des transgenen Vogeltieres unter Verwendung mindestens einer programmierbar entwickelten Nuklease (PEN) integriert wird, wobei die PEN ein gehäuft auftretendes regelmäßig unterbrochenes kurzes palindromisches Wiederholungs-(CRISPR)-Typ II-System ist.

**6.** Verfahren nach einem der Ansprüche 1 bis 5, wobei das mindestens eine Reportergen in ein Geschlechtschromosom des transgenen Vogelsubjekts integriert wird, indem mindestens eine Zelle des Vogelsubjekts oder mindestens eine in das Vogeltier eingeführte Zelle in Kontakt gebracht oder co-transfiziert wird mit:

a. mindestens einem Cas9-Protein oder mindestens einer ersten Nukleinsäuresequenz, die mindestens eine Nukleinsäuresequenz umfasst, die für das mindestens eine Cas9-Protein kodiert; und guide RNA (gRNA), die auf mindestens einen Protospacer innerhalb mindestens eines Geschlechtschromosoms Z und/oder W abzielt, oder mindestens einer Nukleinsäuresequenz, die für die mindestens eine gRNA kodiert; und
b. mindestens einer zweiten Nukleinsäuresequenz, die mindestens ein solches Reportergen umfasst.

**7.** Verfahren nach einem der Ansprüche 1 bis 6, wobei das mindestens eine Reportergen in mindestens eine Stelle der Folgenden integriert wird:

Geschlechtschromosom-Z-Locus 42172748-42177748 oder
Geschlechtschromosom-W-Locus 1022859-1024215.

**8.** Verfahren nach Anspruch 7, wobei das mindestens eine Reportergen in mindestens eine Stelle der Folgenden integriert wird:

(a) Geschlechtschromosom-Z-Locus 42172748-42177748, wobei die mindestens eine gRNA die Nukleinsäuresequenz wie angegeben in SEQ ID NO. 26 umfasst und das mindestens eine Reportergen, das innerhalb der zweiten Nukleinsäure umfasst ist, an 5' und 3' davon von homologen Armen flankiert wird, die die Nukleinsäuresequenz wie angegeben in SEQ ID NO. 31 bzw. 32 umfassen; oder
(b) Geschlechtschromosom-W-Locus 1022859-1024215, wobei mindestens eines der Folgenden vorliegt:

(i) die gRNA die Nukleinsäuresequenz wie angegeben in SEQ ID NO. 1 umfasst, und das mindestens eine Reportergen, das innerhalb der zweiten Nukleinsäuresequenz umfasst ist, an 5' und 3' davon von homologen Armen flankiert wird, die die Aminosäuresequenz wie angegeben in SEQ ID NO. 4 bzw. 5 umfassen; und
(ii) die gRNA die Nukleinsäuresequenz wie angegeben in SEQ ID NO. 2 umfasst, und das mindestens eine Reportergen, das innerhalb der zweiten Nukleinsäuresequenz umfasst ist, an 5' und 3' davon von homologen Armen flankiert wird, die die Aminosäuresequenz wie angegeben in SEQ ID NO. 6 bzw. 7 umfassen.

**9.** Transgenes Vogelsubjekt, umfassend mindestens ein exogenes Reportergen, das in mindestens einen Locus in mindestens einem der Geschlechtschromosomen Z und W integriert ist, wobei das Reportergen ein rot fluoreszierendes Protein (RFP) ist, und wobei die Expression des mindestens einen Reporters geeignet ist, um ein Geschlecht eines Vogelembryos in einem ungeschlüpften Ei, das den Embryo innerhalb einer strukturell integralen Schale umfasst, zu identifizieren.

**10.** Transgenes Vogelsubjekt nach Anspruch 9, wobei das Reportergen für ein RFP-Protein mit einer Anregungswellenlänge von 500-650 nm und einer Emissionswellenlänge von 550-650 nm kodiert.

**11.** Transgenes Vogelsubjekt nach Anspruch 9 oder 10, wobei das mindestens eine transgene Vogelsubjekt weiblich ist, und wobei das mindestens eine Reportergen in mindestens eine Position des weiblichen Chromosoms Z oder des weiblichen Chromosoms W integriert ist.

**12.** Transgenes Vogelsubjekt nach einem der Ansprüche 9 bis 11, wobei das mindestens eine Reportergen in mindestens eine Stelle am Geschlechtschromosom-Z-Locus 42172748-42177748 integriert ist, wobei die mindestens eine gRNA die Nukleinsäuresequenz wie angegeben in SEQ ID NO. 26 umfasst und das mindestens eine Reportergen, das innerhalb der zweiten Nukleinsäure umfasst ist, an 5' und 3' davon von homologen Armen flankiert ist, die die Nukleinsäuresequenz wie angegeben in SEQ ID NO. 31 bzw. 32 umfassen.

**13.** Transgenes Vogelsubjekt nach einem der Ansprüche 9 bis 11, wobei das mindestens eine Reportergen in mindestens eine Stelle am Geschlechtschromosom-W-Locus 1022859-1024215 integriert ist, wobei mindestens eines der Folgenden vorliegt:

(a) die gRNA die Nukleinsäuresequenz wie angegeben in SEQ ID NO. 1 umfasst, und das mindestens eine Reportergen, das innerhalb der zweiten Nukleinsäuresequenz umfasst ist, an 5' und 3' davon von homologen Armen flankiert ist, die die Aminosäuresequenz wie angegeben in SEQ ID NO. 4 bzw. 5 umfassen; und

(b) die gRNA die Nukleinsäuresequenz wie angegeben in SEQ ID NO. 2 umfasst, und das mindestens eine Reportergen, das innerhalb der zweiten Nukleinsäuresequenz umfasst ist, an 5' und 3' davon von homologen Armen flankiert ist, die die Aminosäuresequenz wie angegeben in SEQ ID NO. 6 bzw. 7 umfassen.

**14.** Kit umfassend:

a. mindestens ein Cas9-Protein oder mindestens eine erste Nukleinsäuresequenz, umfassend mindestens eine Nukleinsäuresequenz, die für das mindestens eine Cas9-Protein kodiert; und mindestens eine gRNA, die auf mindestens einen Protospacer innerhalb mindestens eines Geschlechtschromosoms Z und/oder W abzielt, oder eine Nukleinsäuresequenz, die für die mindestens eine gRNA kodiert; und

b. mindestens eine zweite Nukleinsäuresequenz, umfassend mindestens ein Reportergen, wobei das Reportergen ein Rot-fluoreszierendes-Protein (RFP) ist; wobei die Expression des mindestens einen Reporters geeignet ist, um ein Geschlecht eines Vogelembryos in einem ungeschlüpften Ei, das den Embryo innerhalb einer strukturell integralen Schale umfasst, zu identifizieren,

wobei das mindestens eine Reportergen in mindestens eine Stelle der Folgenden integriert ist:

(a) Geschlechtschromosom-Z-Locus 42172748-42177748, wobei die mindestens eine gRNA die Nukleinsäuresequenz wie angegeben in SEQ ID NO. 26 umfasst, und das mindestens eine Reportergen, das innerhalb der zweiten Nukleinsäure umfasst ist, an 5' und 3' davon von homologen Armen flankiert ist, die die Nukleinsäuresequenz wie angegeben in SEQ ID NO. 31 bzw. 32 umfassen; oder

(b) Geschlechtschromosom-W-Locus 1022859-1024215, wobei mindestens eines der Folgenden vorliegt:

(i) die gRNA die Nukleinsäuresequenz wie angegeben in SEQ ID NO. 1 umfasst, und das mindestens eine Reportergen, das innerhalb der zweiten Nukleinsäuresequenz umfasst ist, an 5' und 3' davon von homologen Armen flankiert ist, die die Aminosäuresequenz wie angegeben in SEQ ID NO. 4 bzw. 5 umfassen; und

(ii) die gRNA die Nukleinsäuresequenz wie angegeben in SEQ ID NO. 2 umfasst, und das mindestens eine Reportergen, das innerhalb der zweiten Nukleinsäuresequenz umfasst ist, an 5' und 3' davon von homologen Armen flankiert ist, die die Aminosäuresequenz wie angegeben in SEQ ID NO. 6 bzw. 7 umfassen.

**15.** Kit nach Anspruch 14, wobei das mindestens eine Reportergen für ein RFP-Protein mit einer Anregungswellenlänge von 500-650 nm und einer Emissionswellenlänge von 550-650 nm kodiert.

**Revendications**

**1.** Procédé de détermination du genre d'un embryon aviaire dans un oeuf non-éclos contenant l'embryon dans une coque structurellement intégré, le procédé comprenant l'étape de :

(a) fourniture d'au moins un sujet aviaire transgénique comprenant au moins un gène rapporteur exogène intégré dans au moins une position ou locus dans au moins l'un des chromosomes de genre Z et W, dans lequel ledit gène rapporteur est la Protéine Fluorescente Rouge (PFR) ; et

(b) dans un embryon contenu dans la coque structurellement intégrée dans un oeuf non-éclos obtenu à partir dudit aviaire transgénique, détermination au sein de ladite coque structurellement intégrée dudit oeuf non-éclos si au moins un signal détectable est détecté, dans lequel la détection dudit au moins signal détectable indique l'expression dudit au moins un gène rapporteur dans ledit embryon au sein de ladite coque structurellement intégrée dudit oeuf non-éclos, et ainsi la présence dudit chromosome Z ou chromosome W dans ledit embryon, déterminant ainsi le genre d'un embryon aviaire dans un oeuf non-éclos contenant l'embryon au sein d'une coque structurellement intégrée.

**2.** Procédé selon la revendication 1, dans lequel ledit gène rapporteur code une protéine RFP présentant une longueur d'onde d'excitation de 500-650 nm et une longueur d'onde d'émission de 550-650 nm.

**3.** Procédé selon la revendication 1 ou 2, dans lequel ladite détermination si un signal détectable est détecté comprend l'étape consistant à soumettre ledit oeuf non-éclos contenant l'embryon dans une coque structurellement intégrée à une source lumineuse.

**4.** Procédé selon l'une quelconque des revendications 1 à 3, dans lequel ledit au moins un sujet aviaire transgénique est un sujet aviaire femelle, et dans lequel ledit au moins un gène rapporteur est intégré dans au moins un locus : (a) du chromosome femelle Z, ce qui permet la détection d'un signal détectable indiquant que ledit embryon dans ledit oeuf non-éclos est un mâle ; ou (b) du chromosome femelle W, ce qui permet la détection d'un signal détectable indiquant que ledit embryon dans ledit oeuf non-éclos est une femelle .

**5.** Procédé selon l'une quelconque des revendications 1 à 4, dans lequel ledit au moins un gène rapporteur est intégré dans ledit chromosome de genre dudit animal aviaire transgénique en utilisant au moins une nucléase modifiée génétiquement (PEN), dans lequel ledit PEN est un système à courtes répétitions palindromiques groupées et régulièrement espacées de type II (CRISPR).

**6.** Procédé selon l'une quelconque des revendications 1 à 5, dans lequel ledit au moins un gène rapporteur est intégré dans un chromosome de genre dudit sujet aviaire transgénique en mettant en contact ou en co-transfectant au moins une cellule dudit sujet aviaire ou au moins une cellule introduite dans ledit animal aviaire, avec:

   a. au moins une protéine Cas9 ou au moins une première séquence d'acides nucléiques comprenant au moins une séquence d'acides nucléiques codant pour ladite au moins une protéine Cas9 ; et un ARN guide (ARNg) qui cible au moins un proto-espaceur dans au moins un chromosome de genre Z et/ou W, ou au moins une séquence d'acides nucléiques codant pour ledit au moins un ARNg ; et
   b. au moins une deuxième séquence d'acides nucléiques comprenant au moins un dit gène rapporteur.

**7.** Procédé selon l'une quelconque des revendications 1 à 6, dans lequel ledit au moins un gène rapporteur est intégré dans au moins un site au : locus du chromosome de genre Z 42172748-42177748 ou locus du chromosome de genre W 1022859-1024215.

**8.** Procédé selon la revendication 7, dans lequel ledit au moins un gène rapporteur est intégré dans au moins un site au : (a) locus du chromosome de genre Z 42172748-42177748, dans lequel ledit au moins un ARNg comprend la séquence d'acides nucléiques telle qu'indiquée par SEQ ID NO. 26 et ledit au moins un gène rapporteur compris dans ladite deuxième séquence d'acides nucléiques est flanquée en 5' et 3' de celle-ci par des bras homologues comprenant la séquence d'acides nucléiques telle qu'indiquée par SEQ ID NO. 31 et 32, respectivement ; ou (b) au locus du chromosome de genre W 1022859-1024215, dans lequel au moins l'un parmi :

   (i) ledit ARNg comprend la séquence d'acides nucléiques telle qu'indiquée par SEQ ID NO. 1, et ledit au moins un gène rapporteur compris dans ladite deuxième séquence d'acides nucléiques est flanquée en 5' et 3' de celle-ci par des bras homologues comprenant la séquence d'acides aminés telle qu'indiquée par SEQ ID NO. 4 et S, respectivement ; et
   (ii) ledit ARNg comprend la séquence d'acides nucléique telle qu'indiquée par SEQ ID NO. 2, et ledit au moins un gène rapporteur compris dans ladite deuxième séquence d'acides nucléiques est flanquée en 5' et 3' de celle-ci par des bras homologues comprenant la séquence d'acides aminés telle qu'indiquée par SEQ ID NO. 6 et 7, respectivement.

**9.** Sujet transgénique aviaire comprenant au moins un gène rapporteur exogène intégré dans au moins un locus dans au moins l'un des chromosomes de genre Z et W, et dans lequel ledit gène rapporteur est la Protéine Fluorescente Rouge (PFR), et dans lequel l'expression dudit au moins un rapporteur est adaptée pour l'identification d'un genre d'un embryon aviaire dans un oeuf non-éclos comprenant l'embryon au sein d'une coque structurellement intégrée.

**10.** Sujet transgénique aviaire selon la revendication 9, dans lequel ledit gène rapporteur code une protéine RFP présentant une longueur d'onde d'excitation de 500-650 nm et une longueur d'onde d'émission de 550-650 nm.

**11.** Sujet transgénique aviaire selon la revendication 9 et 10, dans lequel ledit au moins un sujet transgénique aviaire est une femelle, et dans lequel ledit au moins un gène rapporteur est intégré dans au moins un locus dans : un chromosome femelle Z ou un chromosome femelle W.

**12.** Sujet transgénique aviaire selon l'une quelconque des revendications 9 à 11, dans lequel ledit au moins un gène rapporteur est intégré dans au moins un site au locus du chromosome de genre Z 42172748-42177748, dans lequel ledit au moins un ARNg comprend la séquence d'acides nucléiques telle qu'indiquée par SEQ ID NO. 26 et ledit au moins un gène rapporteur compris dans ladite deuxième séquence d'acides nucléiques est flanquée en 5' et 3' de celle-ci par des bras homologues comprenant la séquence d'acides nucléiques telle qu'indiquée par SEQ ID NO. 31 et 32, respectivement.

13. Sujet transgénique aviaire selon l'une quelconque des revendications 9 à 11, dans lequel ledit au moins un gène rapporteur est intégré dans au moins un site au locus du chromosome de genre W 1022859-1024215, dans lequel au moins l'un parmi :

(a) ledit ARNg comprend la séquence d'acides nucléiques telle qu'indiquée par SEQ ID NO. 1, et ledit au moins un gène rapporteur compris dans ladite deuxième séquence d'acides nucléiques est flanquée en 5' et 3' de celle-ci par des bras homologues comprenant la séquence d'acides aminés telle qu'indiquée par SEQ ID NO. 4 et S, respectivement ; et

(b) ledit ARNg comprend la séquence d'acides nucléique telle qu'indiquée par SEQ ID NO. 2, et ledit au moins un gène rapporteur compris dans ladite deuxième séquence d'acides nucléiques est flanquée en 5' et 3' de celle-ci par des bras homologues comprenant la séquence d'acides aminés telle qu'indiquée par SEQ ID NO. 6 et 7, respectivement.

14. Kit comprenant :

a. au moins une protéine Cas9 ou au moins une première séquence d'acides nucléiques comprenant au moins une séquence d'acides nucléiques codant pour ladite au moins une protéine Cas9 ; et au moins un ARNg qui cible au moins un proto-espaceur dans au moins un chromosome de genre Z et/ou W, ou une séquence d'acides nucléiques codant pour ledit au moins un ARNg ; et

b. au moins une deuxième séquence d'acides nucléiques comprenant au moins un gène rapporteur, dans lequel ledit gène rapporteur est la Protéine Fluorescente Rouge (PFR) ; dans lequel l'expression dudit au moins un rapporteur est adaptée pour l'identification d'un genre d'un embryon aviaire dans un oeuf non-éclos comprenant l'embryon au sein d'une coque structurellement intégrée,

dans lequel ledit au moins un gène rapporteur est intégré dans au moins un site au : (a) locus du chromosome de genre Z 42172748-42177748, dans lequel ledit au moins un ARNg comprend la séquence d'acides nucléiques telle qu'indiquée par SEQ ID NO. 26 et ledit au moins un gène rapporteur compris dans ladite deuxième séquence d'acides nucléiques est flanquée en 5' et 3' de celle-ci par des bras homologues comprenant la séquence d'acides nucléiques telle qu'indiquée par SEQ ID NO. 31 et 32, respectivement ; ou (b) au locus du chromosome de genre W 1022859-1024215, dans lequel au moins l'un parmi :

(i) ledit ARNg comprend la séquence d'acides nucléiques telle qu'indiquée par SEQ ID NO. 1, et ledit au moins un gène rapporteur compris dans ladite deuxième séquence d'acides nucléiques est flanquée en 5' et 3' de celle-ci par des bras homologues comprenant la séquence d'acides aminés telle qu'indiquée par SEQ ID NO. 4 et S, respectivement ; et (ii) ledit ARNg comprend la séquence d'acides nucléique telle qu'indiquée par SEQ ID NO. 2, et ledit au moins un gène rapporteur compris dans ladite deuxième séquence d'acides nucléiques est flanquée en 5' et 3' de celle-ci par des bras homologues comprenant la séquence d'acides aminés telle qu'indiquée par SEQ ID NO. 6 et 7, respectivement.

15. Kit selon la revendication 14, dans lequel ledit au moins un gène rapporteur code une protéine RFP présentant une longueur d'onde d'excitation de 500-650 nm et une longueur d'onde d'émission de 550-650 nm.

Fig. 1A

Fig. 1B

Fig. 2

Fig. 3

Fig. 4

Fig. 5

Fig. 6

Fig. 7

Fig. 8

Fig. 9

Fig. 10

Fig. 11A

Fig. 11B

Fig. 12

Fig. 13

Fig. 14

TGGAGATCCCTTATTGGTGAAGCTTTCCCCTGCAAACCTAGAATCTTCTGATGCAAGTGAGCAATCTTCTTCCCTTCCCTAGCAGCTCTCTGTTGCACC
AGTCCTTCCATCCTTGCACCTTTGTTGTAATGCTTTTCCCATGGCATTGATCTGTCCAGTTGCTACAGTGTAGAAACAAGAAAGTGTTTTCTCAAAACA
TTATACATGCTTACTATCAGATCTTACTTAACATTGATAAGAAGTACACCTTTCATTACAACA*GTGAACGGATAGGCAGCAAGCATTCAGAACAATGAA*
*AAAGATCTGCGTTCTATTGTAAAGCAAACAAAGGTGATGACGGAACGTTTATTTGAGATACAGGCACCATAGTACAAGATATCAAAGAGGAATTAATTC*
*AACCCTCCCAACAACAGTTGGGAAAAAAAAGGATAAGCTGAGGAATGCAGCCTTCTCCGATTTTTACTCTGGTGCCGGAAGGAGGAGGAGGAGCGGGG*
*GAAAACCCAGCGGACTCAGACTGCATACGCACTCTCGGAAGTACCTCTGAGGCGGGGTGAGAGGAAGGGTGGATCTAGGCTCCCCCGCTCCACACT*
*CACGTGCTCGTGTACTGGTTCAGGCTGTGACTTGGCAACTCCTCTACATCTATCCATCTAGTTAGACTCAACAAGACCATCGTGATTCAGGAGTCTGAA*
*GATGCTTAACTCTCTCAGAACGTCTAGTTCTCCTGGCCTGGTAAATGCTATGTTTCTCATACTGCCTCTCTGAAGAATGCTTCGAATGCTTCTAGTGAT*
*GCTCTAAAGTTCTAAACAGAAAAATCTGGAGACAGTTCTGGTCTTTAGATAGAAAAAATGCCAACATGCCAAAGGATGGTTACATCCTTCAAGCAACCT*
*TGTTGCATGCTGTACAATAGACTCATGTAATAACTTAGCCGTAGTCATCGTATCTCTTATTTACCTGTTCGTTATTACATTTTCCTGGTACTGCTTTAT*
*ATTTAGTCAGTTGTCCTTTTAAGACAAATTTTTTGGTGTGTGCTAATAGGCAGTTACCAAATGTTCTAGAGGGAGGGAATATAATCAGTGAGTATGTAG*
*ATGTATATAGATGTATAACCAGTAAGTATACAGAGAAGTTAGGGTGGTCTTTTGAGAGCATATAGCTGGAAAGATTATCACATGGGAAAGGTAATCAG*
*AAATAAATGGAAAAATGCTCACCAGTGTCATAGGCTCACAAGAAAACTCCCCAAGGAGCAATCTCCAGTAACAGACCCTTCCTCAGT*<u>TGGAGTTCCG</u>
<u>CGTTACATAACTTACGGTAAATGGCCCGCCTGGCTGACCGCCCAACGACCCCCGCCCATTGACGTCAATAATGACGTATGTTCCCAT</u>
<u>AGTAACGCCAATAGGGACTTTCCATTGACGTCAATGGGTGGAGTATTTACGGTAAACTGCCCACTTGGCAGTACATCAAGTGTATCA</u>
<u>TATGCCAAGTACGCCCCCTATTGACGTCAATGACGGTAAATGGCCCGCCTGGCATTATGCCCAGTACATGACCTTATGGGACTTTCC</u>
<u>TACTTGGCAGTACATCTACGTATTAGTCATCGCTATTACCATGGTGATGCGGTTTTGGCAGTACATCAATGGGCGTGGATAGCGGTTT</u>
<u>GACTCACGGGGATTTCCAAGTCTCCACCCCATTGACGTCAATGGGAGTTTGTTTTGGCACCAAAATCAACGGGACTTTCCAAAATGT</u>
<u>CGTAACAACTCCGCCCCATTGACGCAAATGGGCGGTAGGCGTGTACGGTGGGAGGTCTATATAAGCAGAGCTGGTTTAGTGAACCG</u>
<u>TCAGATCCGCTAGCGCTACCGGACTCAGATCTCGAGCTCAAGCTTCGAATTCTGCAGTCGACGGTACCGCGGGCCCGGGATCCACCG</u>
<u>GTCGCCACC</u>*ATGGCCTCCTCCGAGAACGTCATCACCGAGTTCATGCGCTTCAAGGTGCGCATGGAGGGCACCGTGAACGGCCACGAGT*
*TCGAGATCGAGGGCGAGGGCGAGGGCCGCCCCTACGAGGGCCACAACACCGTGAAGCTGAAGGTGACCAAGGGCGGCCCCCTGCCCT*
*TCGCCTGGGACATCCTGTCCCCCCAGTTCCAGTACGGCTCCAAGGTGTACGTGAAGCACCCCGCCGACATCCCCGACTACAAGAAGCT*
*GTCCTTCCCCGAGGGCTTCAAGTGGGAGCGCGTGATGAACTTCGAGGACGGCGGCGTGGCGACCGTGACCCAGGACTCCTCCCTGCA*
*GGACGGCTGCTTCATCTACAAGGTGAAGTTCATCGGCGTGAACTTCCCCTCCGACGGCCCCGTGATGCAGAAGAAGACCATGGGCTGG*
*GAGGCCTCCACCGAGCGCCTGTACCCCCGCGACGGCGTGCTGAAGGGCGAGACCCACAAGGCCCTGAAGCTGAAGGACGGCGGCCAC*
*TACCTGGTGGAGTTCAAGTCCATCTACATGGCCAAGAAGCCCGTGCAGCTGCCCGGCTACTACTACGTGGACGCCAAGCTGGACATCA*
*CCTCCCACAACGAGGACTACACCATCGTGGAGCAGTACGAGCGCACCGAGGGCCGCCACCACCTGTTCCTGTAGCGGCCGCGACTCTA*
*GATCATAATCAGCCATACCACAT*

Fig. 15A

[SV40 poly(A) signal- f1 ori-AmpR promoter-sv40 promoter- NeoR/KanR-HSVTKpoly(A) signal-ori]

*GAGTCATGTCGGTGGAGGAGAAAGTGGCTGTAGAAAAGGTAACGGACAAGAAATCCCTCACTGGCTTAGCTGTAAAGGTCAGAGGAAAATCATCTTCTG*
*TTCTCCATACACATCTCCTGTAATTAGGCACCTGTGCCCTTTAAAAAAGTAAAGTAATCAAAGAGTCATTTGGTGAAAATAGAAGCCAAACAGTTACAA*
*AATTTAGTTAATATTCAAAGACAAAACAACTCTGGTTCCAAATATAAAACCCTTTGTGTAAACACTGCGGGAAGGTGCCAAAGAGCCACCTACACAAAG*
*AGATGCCACAGAGTAATTTGCGTACCCCAACAATAGGATCATATATGGGCAAACCAAAAGACCAACAAAGACCCCTTGCAATAATCCCTTGATTGGCAG*
*AAGCAAGTGCAGCGTTAGATTTAGTATGTGTGACAATTCTGAGAATAGGAAGGAATTTCACTGAAACTGTGTGAAGATTTGTATGTTAAATACATATAA*
*TGAGGGTTATTTAGCTCTGGGGTGTGCATGCTATGTGGAGAGATCCCCATGTGCCCAGCACTGCAATAGACTAATGTCAGCTTTTTAAACCATCATTTG*
*GTTTGAAGAGTTTATTATGATTTTCAGAAACAGAATTATGTATTCAGTGTCTGTACATTCTTACAAGCCTGCTGTTCTGTACACATGAAAAGCTACTT*
*ATGGTGCAAATCAGTATAGAGAAGCAGTTTTGACATACAGTGGTCATGACTGGGTGATCTGCTGTCAGAAGGTAAGACACTGGTATACAGAATTGCAAA*
*CGTGAGATGAAGACCTCAAGTGCAACTCTGTCATCATACCACTTCTCTTGCTATTATCTATTCAGTGTTTGTTCTAAATATTCAGGCAGAAGGCTTGGT*
*TTCACATCAGGTGTTACATTTAAGTATTCTTTGCCCATTTTTTGCTGTTTGTATGTGTAACTTCAGATTCTCACATTGACTGTGTAGTGTAAATTTGCA*
*AATAGATGTAACAGCTTCTCTTTAACATCCCATGCA*CAAACTTTTAATCACTTTCAATCAATGTTGGAAATCTGCAGTACATAAATTTTATTTTGATTC
ATTATTCAGAGAATCAGAGAAATAAGGACTTTTTTAGAGGTTTATGTTTATTTTAGAAAATATAAATAAATCTTGTATACATAGATTTCCAACTAGTAA
AATTTTTAGCTATCGCTGTAGCATTTCTATGAGGAGTTTTACTTTTTACTGTCTCTTATGAAACTTAGTGGAAACAAAGCTAG

Fig. 15B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010103111 A
- WO 20140296707 A
- US 6244214 B
- US 06124456 A2
- US 2014069336 A
- WO 16005539 A
- WO 9639505 A
- WO 9749806 A
- WO 06124456 A2
- CA 2264450

### Non-patent literature cited in the description

- **QUANSAH, E. ; LONG, J.A. ; DONOVAN, D.M. ; BECKER, S.C. ; TELUGU, B. ; FOSTER FREY, J.A. ; URWIN, N.** Sperm-mediated transgenesis in chicken using a PiggyBac transposon system. *Poultry Science Association Meeting Abstract,* 2014
- **JINEK, M. ; CHYLINSKI, K. ; FONFARA, I. ; HAUER, M. ; DOUDNA, J. A. ; CHARPENTIER, E.** A programmable dual-RNA-guided DNA endonuclease in adaptive bacterial immunity. *Science,* 2012, vol. 337 (6096), 816-821
- **CONG, L. ; ZHANG, F.** Genome engineering using CRISPR-Cas9 system. *Chromosomal Mutagenesis,* 2015, 197-217
- **VÉRON N. ; QU Z. ; KIPEN PA. ; HIRST CE. ; MARCELLE C.** CRISPR mediated somatic cell genome engineering in the chicken. *Dev. Biol.,* 13 August 2015, vol. 407 (1), 68-74
- **NIU, Y. ; B. SHEN ; Y. CUI ; Y. CHEN ; J. WANG et al.** Generation of genemodified cynomolgus monkey via cas9/rna-mediated gene targeting in one-cell embryos. *Cell,* 2014, vol. 156 (4), 836-843
- **HWANG, W. Y. ; Y. FU ; D. REYON ; M. L. MAEDER ; S. Q. TSAI et al.** Efficient genome editing in zebrafish using a CRISPR-Cas system. *Nat. Biotechnol.,* 2013, vol. 31 (3), 227-229
- **NADÈGE, V. ; Q. ZHENGDONG ; P. A. S. KIPEN ; C. E. HIRST ; M. CHRISTOPHE et al.** CRISPR mediated somatic cell genome engineering in the chicken. *Dev. Biol.,* 2015, vol. 407 (1), 68-74
- **BAI, Y. ; L. HE ; P. LI ; K. XU ; S. SHAO et al.** Efficient genome editing in chicken DF-1 cells using the CRISPR/Cas9 system. *G3 (Bethesda),* 2016
- **DORAN T. et al.** Sex selection in layer chickens. *ASAP Animal Production,* 2016
- **TIZARD M. ; DORAN T.** Precision genome engineering in the chicken: Th gap between science and market place. *A presentation at IWRAB - II,* 2014
- **MATZ, M. V. et al.** *Nature Biotech.,* 1999, vol. 17, 969-973
- **GOEDDEL. et al.** Gene Expression Technology: Methods in Enzymology. Academic Press, 1990, vol. 185
- **POUWELS et al.** Cloning Vectors: a Laboratory Manual. Elsevier, 1985
- Vectors: a Survey of Molecular Cloning Vectors and their Uses. Buttersworth, 1988
- **SAMBROOK et al.** Molecular cloning: A laboratory manual. Cold Springs Harbor Laboratory, 19890000
- **AUSUBEL et al.** Current Protocols in Molecular Biology. John Wiley and Sons, 1988
- **PELEG Y et al.** *J. Struct. Biol.,* 2010, vol. 172 (1), 34-44